# EUROPEAN PATENT SPECIFICATION

(11) **EP 3 004 036 B1**
(45) Date of publication and mention of the grant of the patent: **22.11.2017**
(21) Application number: 14728884.9
(22) Date of filing: 28.05.2014
(51) Int. Cl.: C07C 67/08, C07C 45/68, C07F 7/18, C07C 45/65, C07C 47/21, C07C 49/727, C07C 45/72, C07C 49/743, C07C 17/02, C07C 49/693, C07C 49/627, C07C 45/64, C07C 29/42, C07C 45/40, C07C 35/28, C07C 45/30, C07D 317/70, C07C 29/00

(54) **METHODS OF SYNTHESIS OF INGENOL AND INTERMEDIATES THEREOF**
VERFAHREN ZUR SYNTHESE VON INGENOL UND ZWISCHENPRODUKTEN DAVON
PROCÉDÉS DE SYNTHÈSE D'INGÉNOL ET DE SES INTERMÉDIAIRES

(30) Priority: 31.05.2013 US 201361829861 P; 18.02.2014 US 201461941321 P
(43) Date of publication of application: 13.04.2016
(73) Proprietor: Leo Laboratories Limited, Dublin 12 (IE)
(72) Inventor: BARAN, Phillipe S., La Jolla, California 97037 (US); JØRGENSEN, Lars, DK-2750 Ballerup (DK); KUTTRUFF, Christian A., 88397 Biberach an der Riss (DE); MCKERRALL, Steven J., La Jolla, California 97037 (US); YEH, Chien-Hung, La Jolla, California 97037 (US)
(74) Representative: Leo Pharma A/S
(86) International application number: PCT/EP2014/061053
(87) International publication number: WO 2014/191457

(56) References cited:
- ISAO KUWAJIMA ET AL: "Total Synthesis of Ingenol", CHEMICAL REVIEWS, vol. 105, no. 12, 11 September 2005 (2005-09-11), pages 4661-4670, XP055134231, ISSN: 0009-2665, DOI: 10.1021/cr040636z cited in the application

## Description

### FIELD OF THE INVENTION

The field relates generally to methods of synthesis of diterpene heterocylic compounds. More particularly, the field relates to efficient methods of synthesis of ingenol (CAS 30220-46-3, **21**) from a compound **1,** (+)-3-carene. Ingenol is a highly oxygenated tetracyclic diterpene. Also provided are various advantageous intermediates along the synthetic route of ingenol. Synthesis of ingenol is useful for efficient synthesis of compounds such as ingenol-3-angelate (**29**), a compound found in *Euphorbia peplus,* which is the active ingredient in an FDA-approved topical treatment for actinic keratosis. Ingenol has the structure shown below, (with carbon atoms numbered):

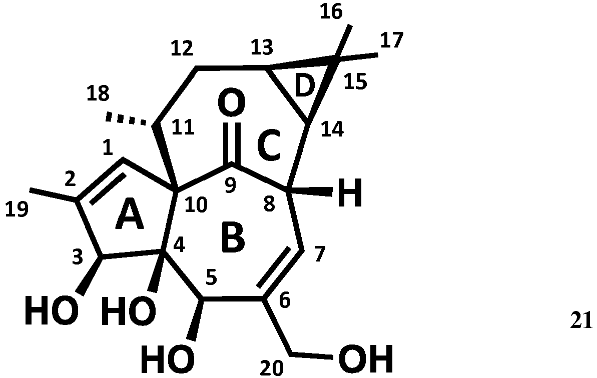

### BACKGROUND OF THE INVENTION

Ingenol is a tetracyclic diterpene natural product produced by the spurge family of plants (*Euphorbiacea*), belonging to a family of molecules referred to as ingenanes. The ingenane family of molecules possesses a common core structure including an "inside-outside" bridged BC ring system, but differs in the appearance of various functional groups decorating the rings of the core structure. Ingenol is the most widely distributed diterpene nucleus of the genus *Euphorbia.* (*See,* Abo et al., Phytochemistry, 1982, 21:725). The first reported isolation of ingenol was made by Hecker in 1968, who was investigating the co-carcinogenic properties of seed oil from *Croton tiglium* and other *Euphorbiacea* (Hecker, E., Cancer Res., 1968, 28:2338-2348). Ingenol was originally isolated from *Euphorbia ingens,* but is also easily isolated from the seeds of *Euphorbia lathyris.* (*See,* Appendino, G. et. al., J. Nat Prod., 1999, 62:76-79). Furthermore, ingenol is commercially available, for example from LC Laboratories, 165 New Boston Street, Woburn, MA 01801, USA.

It was known that such seed oil was toxic to amphibia and fish and is a "drastic cathartic." (*Id.*) Today, ingenol is known to possess promising bioactivity, including tumor-promotion, anti-leukemic, and anti-human immunodeficiency virus (HIV) activity. Ingenol is the core structure upon which ingenol mebutate (also called ingenol-3-angelate, trade name PICATO®, compound **29**) is based. Ingenol-3-angelate is also produced by *Euphorbia* plants, for instance, *Euphorbia peplus* and is a known inducer of cell death. PICATO® has been approved by the U.S. Food and Drug Administration for the topical treatment of actinic keratosis. Due to the close relationship between the structure of phorbol and ingenol and the known biochemical properties of phorbol, a diterpene of the tigliane family found in croton oil, the esters of which possess tumor promotion activity conducted through activation of protein kinase C (PKC), Hasler et al. investigated the ability of ingenol to specifically binding to PKC and reported a *K*ᵢ of 30 µM. (*See,* "Specific binding to protein kinase C by ingenol and its induction of biological responses," Hasler et al., Cancer Res., 1992, 52:202-208).

Ingenol is among the most extremely challenging tetracyclic terpenoid compounds to synthesize. (*See adso,* Chen et al., "Total synthesis of eudesmane terpenes by site-selective C-H oxidations," Nature, 459:824-828, 2009; and Shi et al., "Scalable Synthesis of Cortistatin A and Related Structures," J. Am. Chem. Soc., 133:8014-8027, 2011). Previous reports of the total synthesis of ingenol required 37 or more independent steps (chemical reactions) to attain the tetracyclic diterpene (D.F. Taber, "Total Synthesis of Ingenol," Org. Chem. Highlights, March 1, 2004; Winkler et al., "The First Total Synthesis of (±)-Ingenol," J. Am. Chem. Soc., 2002, 124:9726-9728; Nickel et al., "Total Synthesis of Ingenol," J. Am. Chem. Soc., 2004, 126:16300-16301; Tanino et al., J. Am. Chem. Soc., 2003, 125:1498-1500; Watanabe et al., J. Org. Chem., 2004, 69:7802-7808). Nickel et al. pursued the ingenol core structure *via* ring closure using a ruthenium catalyst. Other investigators have reported various attempts to synthesize the vaunted *"in,out" trans-*[4.4.1]bicyclododecane core structure. (*See,* Funk et al., J. Org. Chem., "Stereoselective construction of the complete ingenane ring system," 1993, 58(22):5873-5875; Tang et al., Org. Lett., 2001, 3:1563-1566; Rigby et al., Org. Lett., 2002, 4:799-801). Ingenol (**21**) is comprised of an unusual, highly strained, and difficult to synthesize *trans-*[4.4.1]bicyclododecane ring system which possesses *in,out* stereochemistry, as shown in below: 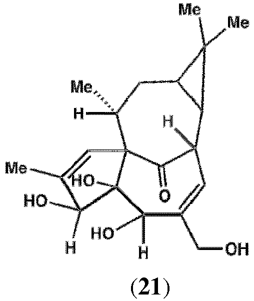 (*See,* Winkler et al., "Inside-outside stereoisomerism. VII. Methodology for the Synthesis of 3-Oxygenated Ingenanes. The First Ingenol Analogs with High Affinity for Protein Kinase C," J. Org. Chem., 60:1381, 1995; and Paquette et al., J. Am. Chem. Soc., 1984, 106:1446). In 2004, Oleg et al. reported the ability to synthesize the *"in,out"* tetracyclic core of ingenol **21** using a pinacol-type rearrangement of a TBS-protected epoxy alcohol. (*See,* Oleg et al., "Rapid Access to the 'in, out'-Tetracyclic Core of Ingenol," Angew. Chem. Int. Ed., 2004, 44(1):121-123).

Rigby reported a compendium of recent advances in attempts to synthesize various tumor-promoting diterpenes in 1993. (*See,* Rigby, J.H., "Recent Advances in the Synthesis of Tumor-Promoting Diterpenes," Atta-ur-Rahman (Ed.) Studies in Natural Products Chemistry, Vol. 12, pages 233-274, 1993, Elsevier). Kim et al. describe an approach to synthesis of ingenol using an intramolecular dioxenone photocycloaddition in "Approaches to the synthesis of ingenol," Chem. Soc. Rev., 1997, 26:387. Somewhat similar to the present approach beginning with 3-carene (**1**), Funk et al. reported an approach using this molecule as a starting point whereby the "crucial *trans* relationship of C-8 and C-10 (ingenol numbering) was set early in the sequence by stereoselective alkylation of a chiral homocarene enone-ester obtained from (+)-3-carene." (*Id.,* and Funk et al., J. Am. Chem. Soc., 1988, 110(10):3298-3300). More recent approaches have been reported, but still required large numbers of steps and overcoming various difficult hurdles. (Tanino et al., J. Am. Chem. Soc., 2003, 125:1498-1500; and Kuwajima et al., "Total Synthesis of Ingenol," Chem. Rev., 2005, 105:4661-4670).

Later, in 2006, Cha et al. published a review in Tetrahedron summarizing various approaches which have been attempted, and indicating newer and more promising approaches still being investigated. (Cha et al., "Synthetic approaches to ingenol," Tetrahedron, 2006, 62:1329-1343). Cha et al. also disclose a synthetic approach beginning with (+)-3-carene. (*Id.* at page 1335). More recently, in 2012, Munro et al. proposed a synthetic approach to ingenol involving a stereospecific and chemoselective 1,5-alkylidene carbene C-H insertion reaction. (K.R. Munro et al., "Selective alkylidene carbene insertion reactions: Studies towards the synthesis of ingenol," presentation given August 20, 2012, 244th Am. Chem. Soc. National Meeting & Exposition, Philadelphia, PA).

Ingenol is a protein kinase C (PKC) activator. Winkler et al. reported synthesis of ingenol derivatives in 1993 that possessed biological activity, *i.e.* a measurable affinity for protein kinase C. (*See,* Winkler et al., "Synthesis of ingenol analogs with affinity for protein kinase C," Bioorg. Med. Chem. Lett., 1993, 3(4):577-580). Ingenol is known to induce apoptosis and possesses anticancer activity. Ingenol derivatives have received constant and multidisciplinary attention due to their reported pleiotropic pattern of biological activity, such as activation of protein kinase C (PKC), tumor-promotion, anticancer activity, anti-HIV properties. For instance, certain ingenol esters show powerful anticancer activity, and a structure-activity relationship model to discriminate between their apoptotic and non-apoptotic properties has been developed. (*See,* Hasler et al., Cancer Res., 1992, 52:202-208; Armstrong et al., Cardiovasc. Res., 1994, 28:1700-1706; Kuwajima et al., Chem. Rev., 2005, 105:4661-4670).

Various derivatives of ingenol and their biological activities are reported. For instance, ingenol 3,20-dibenzoate (CAS 59086-90-7, C₃₄H₃₆O₇) is a PKC activator which induces apoptosis and has anticancer activity and antileukemic activity. (See, "Antileukemic principles isolated from euphorbiaceae plants," Kupchan et al., Science, 1976, 191:571; "Zur Chemie des Ingenols, II. Ester des Ingenols, und des A'-'-Isoingenols," Sorg et al., Z. Naturforsch., 1982, 37b:748; "Induction of thymocyte apoptosis by Ca2+-independent protein kinase C (nPKC) activation and its regulation by calcineurin activation," Asada et al., J. Biol. Chem., 1998, 273:28392; "Ingenol esters induce apoptosis in Jurkat cells through an AP-1 and NF-kappaB independent pathway," Blanco-Molina et al., Chem. Biol. Interact., 8:767, 2001).

Vigone et al. also report on the study of the biological activity of various ingenol derivatives, including fluoro-ingenol, ingenol-20-deoxy-20-phtalimido, ingenol-3-benzoate-20-deoxy-20-benzamide, ingenol-3-benzoate, ingenol-3,5-dibenzoate, ingenol-3,20-dibenzoate, 20-deoxy-20-benylureidoingenol-3-benzoate, ingenol-20-deoxy-20-fluoro-3-benzoate, ingenol-20-deoxy-20-fluoro-3,5-dibenzoate, ingenol-20-phenylcarbamate, ingenol-20-benzoate, ingenol-3-benzoate-20-phenylcarbamate. (*See,* Vigone et al., Eur. J. Gynaecol. Oncol., 2005, 26(5):526-530). The tests of Vigone et al. of ingenol derivatives on breast cancer cell lines T47D and MDA-MB-231 revealed that ingenol-20-benzoate exhibited antitumour activity characterized by inhibition of cell growth and apoptotic cell death involving a p53-mediated pathway. (*Id*.). Further, the 3-hexadecanoyl natural product derivative of ingenol is known to be a most potent Epstein-Barr virus inducer in lymphoblastoid cells. (*See,* Keller et al., Exp. Cell. Biol., 1982, 50:121). Of course, ingenol is not the only terpenoid compound with known biological activity. In fact many, if not most, of the terpene compounds possess interesting and specific biological activity. (*See,* for instance, Dixon et al., "Scalable, Divergent Synthesis of Meroterenoids via 'Borono-sclareolide'," J. Am. Chem. Soc., 134:8432-8435, 2012; Foo et al., "Scalable, Enantioselective Synthesis of Germacrenes and Related Sesquiterpenes Inspired by Terpene Cyclase Phase Logic," Angew. Chem. Int. Ed., 51:11491-11495, 2012; and Renata et al., "Strategic Redox Relay Enables A Scalable Synthesis of Ouabagenin, A Bioactive Cardenolide," Science, 339:59-63, 2013).

Ingenol-3-angelate (**29**) can also be isolated from various *Euphorbia* species, and particularly from *Euphorbia peplus* and *Euphorbia drummondii* by extraction followed by chromatography as described in U.S. Patent No. 7,449,492. (*See adso,* Sayed et.al., Experienta, 1980, 36:1206-1207; and Hohmann et al., Planta Med., 2000, 66:291-294). As previously reported in WO 2012/010172, extraction of 17 kg of fresh *Euphorbia peplus* affords 7 g of a crude oil, which subsequently must be purified by HPLC to generate pure ingenol-3-angelate. The purification method presents difficulties for larger scale production, due to the difficult removal of co-migrating chlorophyll, substantially limiting the yield of ingenol-3-angelate by plant extraction. (*See,* "The skin cancer chemotherapeutic agent ingenol-3-angelate (PEP005) is a substrate for the epidermal multidrug transporter (ABCB1) and targets tumor vasculature," Li et al., Cancer Res., 2010, 70(11):4509-4519). Ingenol-3-angelate may also be synthesized semi-synthetically using an enzymatic procedure, as disclosed in PCT/EP2013/051431.

Difficulties remain in synthesizing sufficient quantities of ingenol for the many uses of this compound. Furthermore, ingenol derivatives are known to degrade in the presence of acid (Appendino et. al., "Synthesis of Modified Ingenol Esters," Eur. J. Org. Chem., 1999, 12:3413-3420). The goal of the present application is to provide new and very efficient approaches to the synthesis of the highly challenging structure of ingenol, as well as various interesting and advantageous intermediates along the disclosed synthetic pathway.

### SUMMARY OF THE INVENTION

Presently disclosed are scalable processes for the synthesis of ingenol (**21**) starting from a compound (**1**):

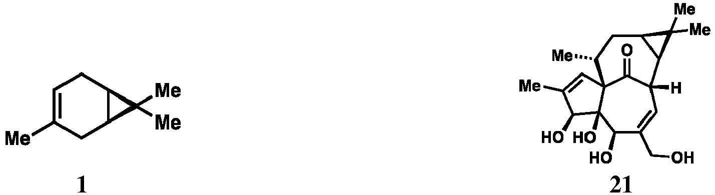

The methods disclosed provide unique synthetic routes to ingenol and intermediates along the pathway to synthesis of ingenol. These synthetic routes may also be used to further convert ingenol to ingenol-3-angelate (alternatively referred to throughout the present application as ingenol mebutate, PEP005, **29**).

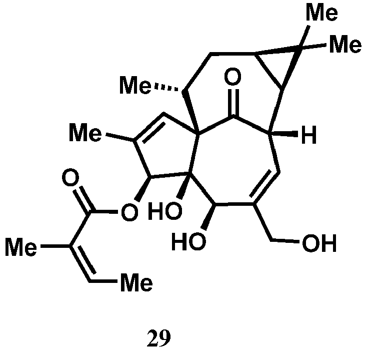

These synthetic routes may also be used to further convert ingenol to ingenol 3-(N-methyl-N-phenyl-carbamate), ingenol 3-(N-(3-fluoro-phenyl)-N-methyl-carbamate), ingenol 3-(3-ethyl-5-methyl-isoxazole-4-carboxylate), ingenol 3-(2,4-dimethylfuran-3-carboxylate), ingenol 3-(3,5-diethylisoxazole-4-carboxylate), ingenol 3-(2,4,5-trimethylfuran-3-carboxylate), ingenol 3-(2-methyl-4-phenyl-pyrazole-3-carboxylate), ingenol 3-(3-methylthiophene-2-carboxylate), ingenol 3-(indoline-1-carboxylate), ingenol 3-(5-methyl-3-phenyl-isoxazole-4-carboxylate) or ingenol 3-(pyrrolidine-1-carboxylate), as described in WO2012/083953.

In one aspect of the disclosed methods is the ability to synthesize ingenol (**21**) from starting material **23.** Starting material of the formula **23** may exist either as an 2R epimer or 2S epimer (**30**) as depicted below:

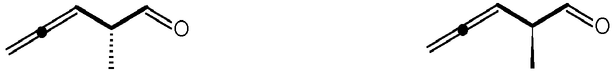

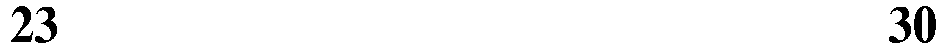

The synthetic procedures described herein may begin with either of these two epimers, **23** or **30,** in Step 4, described in further detail below.

In the present methods, an intermediate in the synthesis of ingenol may be any one or more of the intermediates selected from the group consisting of: a compound of formula **4,** a compound of formula **33,** a compound of formula **34,** a compound of formula **35,** a compound of formula **37,** and a compound of formula **38,** wherein P₁ and P₂ are each individually a hydroxyl protecting group, and R is a diol protecting group:

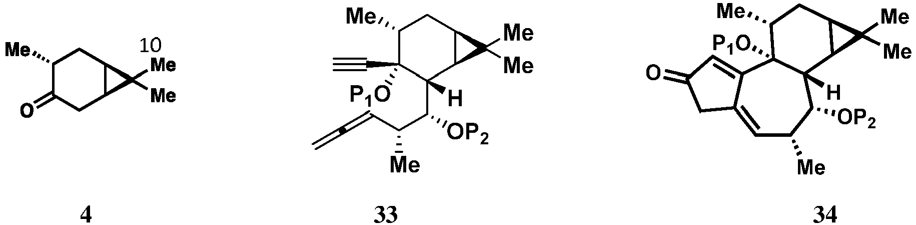

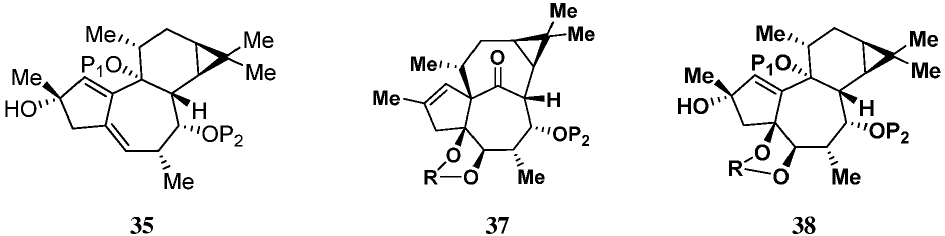

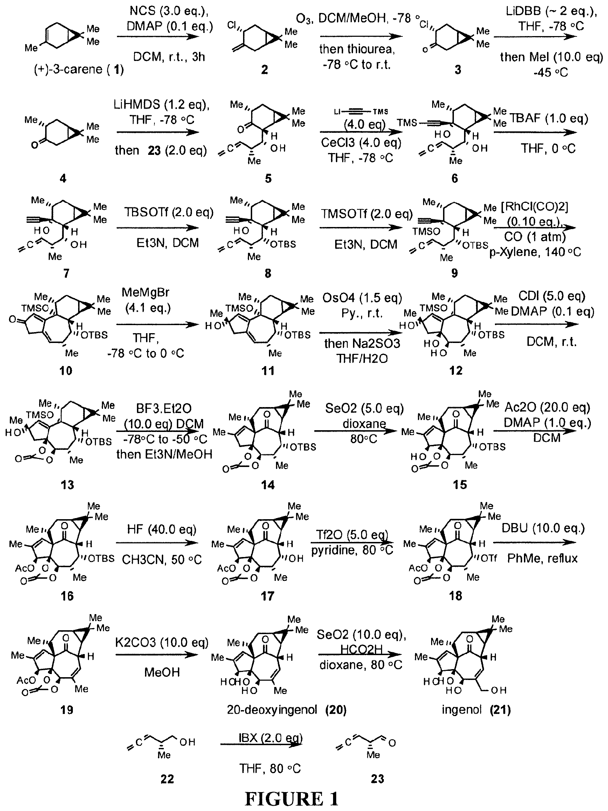

In one embodiment of the present methods, conversion of compound **33** to compound **34** is catalyzed by a rhodium (I) catalyst which may be, for instance, chlorodicarbonylrhodium(I) dimer ([RhCl(CO)₂]₂), [RhCl(COD)]₂, [RhCl(dppp)]₂, or [Rh(dppp)₂]Cl. In this embodiment, the compound of formula **33** is incubated with the rhodium (I) catalyst at a temperature of, or greater than, 140 °C, in a high boiling point solvent. The high boiling point solvent may optionally be an aromatic solvent, such as, for example, xylenes, toluene, mesitylene, dichlorobenzene or other solvents such as dibutyl ether (see Figure 1).

In another embodiment of the present methods, the compound of formula **37** is converted to the compound of formula **38** by pinacol rearrangement at a temperature of -78 °C or lower. In this embodiment, bicarbonate, or other similar neutralizing agent(s), may be added to quench the reaction at -78 °C. The compound of formula **37** may be dissolved in a solvent such as dichloromethane. The compound of formula **38,** the product of pinacol rearrangement of compound **37,** is warmed to room temperature in a neutral solution to avoid exposure to acid. In this embodiment, the reagents boron trifluoride diethyl etherate can be added to compound **37** to form the product compound **38.** The reagent boron trifluoride diethyl etherate may be added to compound **37** in an amount of, or approximately equivalent to, 10.0 equivalents.

In another embodiment, the method of synthesis of ingenol may proceed as depicted in Scheme 1 (Figure 1). In this method, compound 1 is converted to compound **4:**

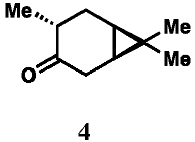

In this embodiment, conversion of compound **1** to compound **4** may proceed through any one or more of intermediates **2** and/or **3:**

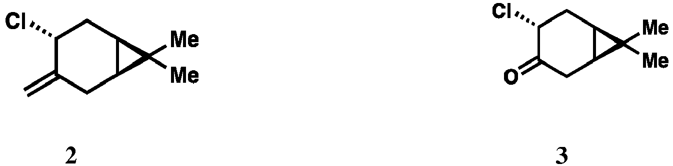

In this embodiment, compound **1** is converted to compound **2** by chlorination of compound **1,** then compound **2** is converted to compound **3** by ozonolysis or other methods for oxidative cleavage (like Lemieux-Johnson reaction) of compound **2.** Finally, compound **3** is converted to compound **4** by reductive alkylation of compound **3.** In one embodiment, enantiomerically pure **4** may be optionally produced by beginning the conversion of **1** to **4** with enantiomerically pure **1.** Enantiomerically pure **1** is obtained commercially. The compound **3** may be reduced at a temperature of -78 °C or lower, followed by alkylating at a temperature of - 45 °C or lower, to produce compound **4.** The reduction step may involve incubation of compound **3** in a lithium-naphthalenide or lithium di-tert-butylbiphenyl solution, for instance, to form a reduced compound, followed by alkylation with methyl iodide, although other reducing agents and alkylating agents known to one of skill may be substituted.

Generally, the synthesis of ingenol may proceed by way of Schemes 1 and/or 2 (Figures 1 and 2, respectively), or variations thereof, such as provided in Schemes 4 and 5 (Figures 4 and 5, respectively). For instance, in Scheme 2, several steps of Scheme 1 have been condensed into single pot reactions using telescoping reagents, *i.e.* one set of reagents is first added and the reaction allowed to continue to completion, followed by addition of a second set of reagents into the same pot, without further purification or otherwise manipulating the reaction between addition of the first and second set of reagents. All, some, or none of the combined steps of Scheme 2 may be employed, depending on the embodiment desired.

In one embodiment, ingenol synthesized by the methods disclosed herein may be further modified to produce ingenol-3-angelate (*see,* for instance, WO 2012/010172).

Further embodiments include specific compounds which correspond to advantageous intermediates along the present synthetic routes, as described in Schemes 1 and 2. For instance, intermediates **4, 34, 35,** and **38.**

Also contemplated as embodiments of the present synthetic methods and intermediates are equivalents which may be known by one of skill. For instance, in many reactions, specific sets of reagents and conditions are indicated or suggested, but one of skill will know that other equivalent reagents may be substituted to achieve similar results, perhaps with different yields.

### BRIEF DESCRIPTION OF THE DRAWINGS

The accompanying drawings, which are incorporated in and form a part of this specification, illustrate embodiments of the invention and, together with the description, serves to explain the invention:
Figure 1 depicts *Scheme 1,* an embodiment of the total synthesis of ingenol. This protocol is disclosed in further detail in Examples 1-21 of the Experimental section, below.

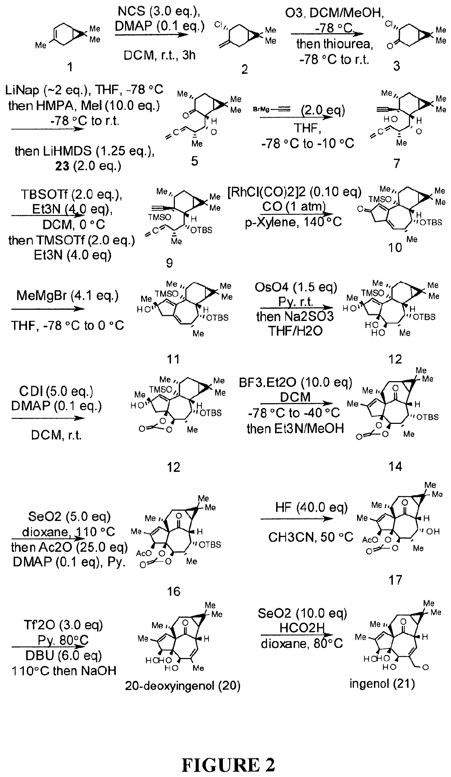

Figure 2 depicts *Scheme 2*, an embodiment of the total synthesis of ingenol which is shorter than that depicted in *Scheme 1.* This route is further characterized in Examples 22-28, in the Experimental section.

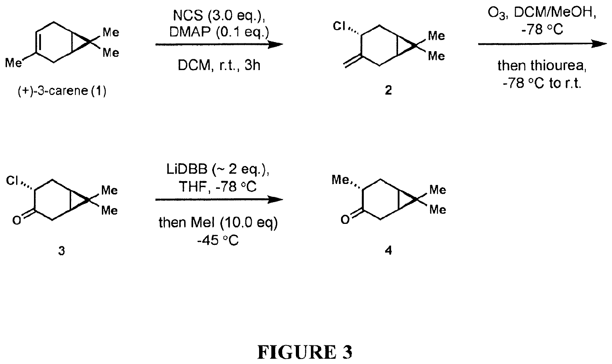

Figure 3 depicts *Scheme 3,* synthesis of **4** from starting material **1.** This route is further characterized in Examples 1-3, in the Experimental section.

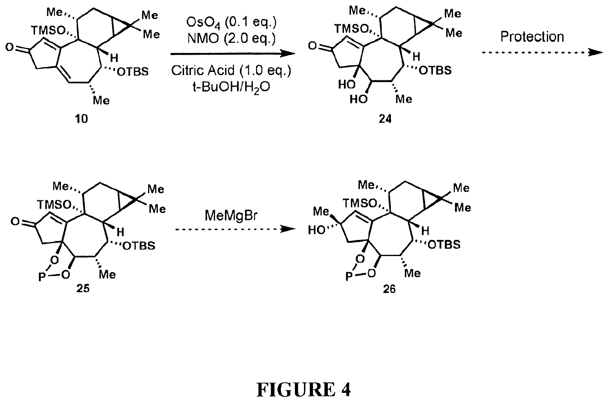

Figure 4 depicts *Scheme 4,* an alternative route of synthesis of **26** from **10.** This route is further characterized in Examples 29-30, in the Experimental section.

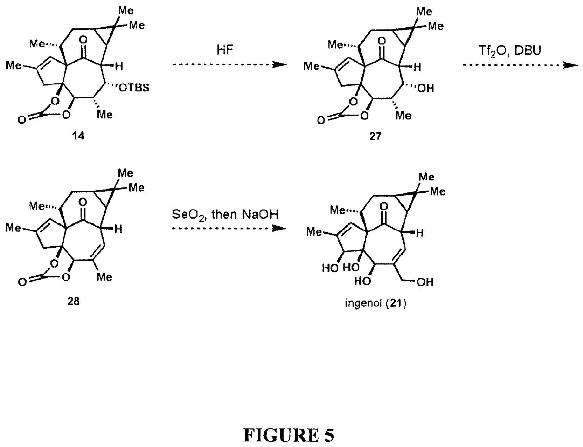

Figure 5 depicts *Scheme 5,* an alternative route of synthesis of **21** from **14.** This route is further characterized in Examples 31-32, in the Experimental section

### DETAILED DESCRIPTION OF THE INVENTION

Although reports of total synthesis of ingenol are provided in the literature, the present invention provides a heretofor unavailable efficient synthetic route that saves time and resources, is scalable, and provides other advantages, as provided in further detail below. The synthetic strategy begins with the simple starting materials of the cyclic monoterpene **1** (commonly known as carene, delta-3-carene, isodiprene, delta-carene, (+)-3-carene, car-3-ene, 3-carvene, 3,7,7-trimethylbicyclo[4.1.0]hept-3-en, CAS 13466-78-9), which is relatively inexpensive and commercially available in enantiomerically pure form (SIGMA ALDRICH, St. Louis, MO, US).

One presently disclosed synthetic route to ingenol includes as many as twenty steps, as depicted in *Scheme 1* (Figure 1). However, this synthetic route may alternatively be reduced to as few as 20, 19, 18, 17, 16, 15, 14, 13, 12, 11 or even 10 steps or fewer. An embodiment of a shorter synthetic route is depicted in *Scheme 2* (Figure 2), where the synthetic route is shortened to fourteen steps, or chemical reactions. That is, *Scheme 2* discloses a shorter synthetic route to ingenol than *Scheme 1* by way of combining certain steps to achieve efficiencies in the synthetic process. However, *Scheme 2* is not intended to depict the shortest route possible to synthesis of **21.** One of skill in the art may be able to further condense some reactions depicted in *Scheme 2* by use of alternative reagents and/or protecting groups and the like. These efficiencies may be beneficial when larger scale production is employed or contemplated. Large scale synthesis may require further modifications, unique conditions and other unforeseen and unpredictable adaptations to successfully achieve larger scale production of ingenol by the presently disclosed methods and by way of the presently disclosed intermediates.

*Scheme 2* provides a shorter synthetic route to ingenol from starting materials **1** and **23,** by way of performance of various combinations of steps in a single pot. That is, one set of reagents may first be added to the intermediate and the reaction allowed to proceed to completion to the next intermediate, followed by addition of a second set of reagents into the same pot, without further purification or otherwise manipulating the reaction or intermediates between addition of the first and second set of reagents. All, some, or none of the combined steps of *Scheme 2*, *Scheme 4,* and/or *Scheme 5* may be employed, depending on the desired result. Further, additional shortening of the synthetic route may be possible, as noted below.

Other efficiencies may be achieved in the synthetic route as may be known to one of skill in the art. Throughout the description, various reagents are provided for each step in the synthetic pathway. However, as is known to one of skill in the art of synthetic organic chemistry, alternative commercially available reagents are often known and substitutable generally throughout the protocol, and more specifically in the steps explicitly described. The presently disclosed protocol discloses certain process steps and conditions for success. However, further optimization using known equivalent reagents and/or methodologies in various steps throughout the pathway are intended to be encompassed by the present description and are generally known to one of skill.

Steps 1 through 4 of the synthesis involve conversion of **1** to structure **4** (see Figure 3, *Scheme 3*). Enantiomerically pure **4** may be achieved by starting with enantiomerically pure **1.** Compound **1** corresponds to carene, a bicyclic monoterpene. Carene is also known as δ³-carene or 3,7,7-trimethylbicyclo[4.1.0]hept-3-ene, and is available as 99% pure (1*S*, 6*R*) enantiomer, (+)-3-carene (CAS 498-15-7), which may be isolated from natural sources. Additionally, 90% chemically pure (+)-3-carene-which is still enantiomerically pure-can be utilized in place of the more expensive 99% chemically pure material. The present application additionally discloses the convenient and efficient synthesis of **4** in isomerically pure form. This compound is highly desired in the field and may be useful for other purposes, *i.e.* as a starting material for other synthetic procedures useful in accessing synthesis of other large molecules.

### Definitions

All terms are intended to be understood as they would be understood by a person skilled in the art.

The term "hydroxyl protective group" or "protective group" or "protecting group" (denoted as "P₁", "P₂", and/or "P₃" in some instances herein) is intended to include any group which forms a derivative of the hydroxyl group that is stable to the projected reactions wherein said hydroxyl protective group subsequently optionally can be selectively removed. Said hydroxyl derivative can be obtained by selective reaction of a hydroxyl protecting agent with a hydroxyl group.

The term "hydroxyl protecting group" is intended to have the same meaning as the term "hydroxyl protective group." Likewise, the term "protecting group" is intended to have the same meaning as the term "protective group."

Ether derivatives, such as allyl ether, prenyl ether, p-methoxybenzyl ether, triphenylmethyl ether, 2-trimethylsilylethyl ether, tert-butyl ether, cinnamyl ether, propargyl ether, *p*-methoxyphenyl ether, benzyl ether, 3,4-dimethoxybenzyl ether, 2,6-dimethoxybenzyl ether, o-nitrobenzyl ether, *p*-nitrobenzyl ether, 4-(trimethylsilylmethyl)-benzyl ether, 2-naphthylmethyl ether, diphenylmethyl ether, (4-methoxyphenyl)-phenylmethyl ether, (4-phenyl-phenyl)-phenylmethyl ether, *ρ,ρ*'-dinitrobenzhydryl ether, 5-dibenzosuberyl ether, tris(4-tert-butylphenyl)methyl ether, (*α*-naphthyl)-diphenylmethyl ether, *ρ*-methoxyphenyldiphenylmethyl ether, di(*ρ*-methoxyphenyl)phenylmethyl ether, tri(*ρ*-methoxyphenyl)methyl ether or 9-(9-phenyl)xanthenyl ether are non-limiting examples of hydroxyl protecting groups.

Ether derived hydroxyl protective groups also include, but are not limited to, alkoxyalkylethers (acetals and ketals) such as 1-ethoxyethyl ether, 1-methyl-1-methoxyethyl ether, [(3,4- dimethoxybenzyl)oxy]methyl ether, guaiacolmethyl ether, 2-methoxyethoxymethyl ether, 2-(trimethylsilyl)ethoxymethyl ether, tetrahydropyranyl ether, tetrahydrofuranyl ether, methoxymethyl ether benzyloxymethyl ether, *ρ*-methoxybenzyloxymethyl ether, *ρ-*nitrobenzyloxymethyl ether, *o*-nitrobenzyloxymethyl ether, (4-methoxyphenoxy)methyl ether, tert-butoxymethyl ether, 4-pentenyloxymethyl ether, siloxymethyl ether, 1-methoxycyclohexyl ether, 4-methoxytetrahydropyranyl ether, 1-[(2-chloro-4-methyl)phenyl]-4-methoxypiperidin-4-yl ether, 1-(2-fluorophenyl)-4-methoxypiperidin-4-yl ether, 1-(4-chlorophenyl)-4-methoxypiperidin-4-yl ether or 1-methyl-1-benzyloxyethyl ether.

Ether derived hydroxyl protective groups also include, but are not limited to, thioacetals and thio ketals such as tetrahydrothiopyranyl ether, 4-methoxytetrahydrothiopyranyl ether, tetrahydrothiofuranyl ether or 1,3-benzodithiolan-2-yl ether.

Hydroxyl protective groups also include, but are not limited to, silyl ether derivatives, such as trimethylsilyl ether, triethylsilyl ether, triisopropylsilyl ether, tert-butyldimethylsilyl ether, dimethylisopropylsilyl ether, diethylisopropylsilyl ether, diphenylmethylsilyl ether, triphenylsilyl ether, dimethylthexylsilyl ether, 2- norbornyldimethylsilyl ether, tert-butyldiphenylsilyl ether, (2- hydroxystyryl)dimethylsilyl ether, (2-hydroxystyryl)diisopropylsilyl ether, tert- butylmethoxyphenylsilyl ether or tert-butoxydiphenylsilyl ether.

Hydroxyl protective groups also include, but are not limited to, esters of hydroxyl groups such as acetate ester, chloroacetate ester, trifluoroacetate ester, phenoxyacetate ester, formate ester, benzoylformate ester, dichloroacetate ester, trichloroacetate ester, methoxyacetate ester, *ρ-*chlorophenoxyacetate ester, phenylacetate ester, 3-phenylpropionate ester, 4-pentenoate ester, 4-oxopentanoate ester, pivaloate ester, crotonate ester, 4-methoxycrotonate ester, angelate ester, benzoate ester or *ρ*-phenylbenzoate ester.

Hydroxyl protective groups also include, but are not limited to, carbonates of hydroxyl groups such as methoxymethyl carbonate, 9-fluorenyl methyl carbonate, methyl carbonate, ethyl carbonate, 2,2,2-trichloroethyl carbonate, 2-(trimethylsilyl)ethyl carbonate, vinyl carbonate, allyl carbonate or *ρ*-nitrophenyl carbonate.

Hydroxyl protective groups also include sulfenates of hydroxyl groups such as 2,4-dinitrophenylsulfenate.

A dihydroxyl protective group, sometimes herein indicated as variable group "R," is any group which forms a derivative of a diol which is stable to the projected reactions wherein said dihydroxyl protective group subsequently optionally can be selectively removed. Said dihydroxyl derivative can be obtained by selective reaction of a dihydroxyl protecting agent with a diol. Ketal derivatives, such as isopropylidene ketal (acetonide), cyclopentylidene ketal, cyclohexylidene ketal, cycloheptylidene ketal, benzophenone ketal, 1-tert-butylethylidene ketal or 1-phenylethylidene ketal, 3-pentylidene ketal, 2,4-dimethyl-3-pentylidene ketal, 2,6-dimethyl-4-heptylidene ketal, 3,3-dimethyl-2- butylidene ketal; and acetal derivatives such as benzylidene acetal, 2,4-dimethoxybenzylidene acetal, 4-nitrobenzylidene acetal, 2,4,6-trimethylbenzylidene acetal, 2,2-dimethyl-1-propylidene acetal, methylene acetal, ethylidene acetal, *ρ-*methoxybenzylidene acetal, tert-butylmethylidene acetal, 3-(benzyloxy)propylidene acetal, acrolein acetal, 2-nitrobenzylidene acetal, mesitylene acetal or 2-naphthaldehyde acetal, are non-limiting examples of dihydroxyl protective groups.

Other dihydroxyl protective groups include, but are not limited to, cyclic ortho esters or ortho esters, such as methoxymethylene acetal, ethoxymethylene acetal, 2-oxacyclopentylidene ortho ester or isopropoxymethylene acetal.

Other dihydroxyl protective groups include, but are not limited to, bisacetal derivatives such as butane 2,3-bisacetal or cyclohexane-1,2-diacetal, or dispiroketals such as octahydro-[2,2']-bipyranyl ketal.

Other dihydroxyl protective groups include, but are not limited to, silyl derivatives such as di-tert-butylsilylene, dialkylsilylene, 1,3-(1,1,3,3-tetraisopropyldisiloxanylidene), 1,1,3,3-tetra-tert-butoxydisiloxanylidene, methylene-bis-(diisopropylsilanoxanylidene, or 1,1,4,4-tetraphenyl-1,4-disilanylidene derivatives.

Dihydroxyl protective groups also include, but are not limited to, cyclic carbonates. Other dihydroxyl protective groups include, but are not limited to, cyclic boronates such as phenyl boronate, methyl boronate or ethyl boronate.

Hydroxyl protective groups and dihydroxyl protective groups also include, but are not limited to, solid phase supported protective groups. Solid phase supported reagents for the introduction of solid phase supported protective groups may include, for example, polymer-bound 2-Chlorotrityl chloride for the introduction of a solid phase supported trityl protective group, or Acetylpolystyrene resin or 4-(4-Hydroxyphenyl)butan-2-one-based resins for the preparation of solid phase supported ketal-protective groups.

The term "alkyne protecting group" or "alkyne protective group" (denoted as "Q" in some instances herein) is intended to include any group which forms a derivative of the alkyne group that is stable to the projected reactions wherein said alkyne protective group subsequently optionally can be selectively removed. Said alkyne derivative can be obtained by selective reaction of an alkyne protecting agent with an alkyne group. Examples of such alkyne protecting groups include, but are not limited to, trialkylsilyl groups such as trimethylsilyl (TMS), triethylsilyl (TES), triisopropylsilyl (TIPS) and t-butyldimethylsilyl (TBDMS).

Non-limiting examples of hydroxyl protective groups, dihydroxyl protective groups, and alkyne protecting groups included in the scope of this invention, can be found, for example, in "Protective Groups in Organic Synthesis," 4th ed. P.G.M. Wuts; T.W. Greene, John Wiley, 2007, page 16-366, and in P.J. Kocienski, "Protecting Groups," 3rd ed. G. Thieme, 2003, which are hereby incorporated by reference in their entirety for all purposes.

Reagents for the introduction of protecting groups are typically commercially available from standard suppliers of fine chemicals, such as FLUKA, SIGMA-ALDRICH and, for instance, BOEHRINGER-INGELHEIM, MERCK and BASF.

An "hydroxyl activating group," (sometimes indicated herein as variable group "L") means a labile chemical moiety which is known in the art to increase the reactivity of the hydroxyl moiety, and that activates a hydroxyl group so that it will depart during synthetic procedures such as in a substitution or an elimination reaction. Many hydroxyl protecting groups are also hydroxyl activating groups. Examples of hydroxyl activating group include, but are not limited to, for instance, mesylates (methanesulfonyl groups), tosylates (p-toluenesulfonyl groups), triflates (trifluoromethanesulfonyl groups, Tf), nonflyls (nonafluorobutanesulfonyl groups), p-nitrobenzoates (such as 3-nitrobenzenesulfonyl groups), phosphonates and the like. Hydroxyl activating groups may also include, but are not limited to, triphenylphosphine and alkyl or aryl sulfonates. Reagents for the introduction of hydroxyl activating groups include, but are not limited to, methane sulfonic anhydride, methane sulfonic chloride, toluene sulfonic chloride and trifluoroacetic chloride.

The term "activated hydroxyl", as used herein, refers to a hydroxy group activated with a hydroxyl activating group, as defined above, including mesylates, tosylates, triflates, p-nitrobenzoates, and phosphonate groups, for example.

As used herein the term "single pot" process denotes that in a sequence of synthesis reactions there is no need for the isolation and purification (even partial purification) of intermediates obtained by each chemical reaction step which occurs in the reaction vessel, until the synthesis of the product at the end of the sequence. That is, "single pot" means the reaction(s) are performed in a single reaction vessel without need to transfer the intermediate to a second reaction vessel or otherwise purify or isolate the reactants and/or products. "One pot" reactions improve the efficiency of chemical synthesis because a reactant is subject to successive chemical reactions in a single pot, or reaction vessel, by addition of telescoping reagents. Nonetheless, it should be understood that if desired, each intermediate product in the sequence of synthesis reactions may optionally be isolated and purified and thus used for other purposes. The term "single pot" indicates that it may be conducted in a vessel through multiple steps, but does not indicate that the preferred method is in a single vessel as a semi-batch process.

The term "telescoping reactions," when used herein indicates a methodology or process often alternatively referred to elsewhere as telescoping synthesis, whereby one set of reagents is first added to a reaction vessel and the reaction allowed to continue to completion, or nearly to completion, followed by addition of a second set of reagents into the same reaction vessel (or "pot"), without further purifying the intermediate product or otherwise manipulating the reaction between addition of the first and second set of reagents. For instance, step 1 reagents may be added to one reaction vessel and the reaction allowed to proceed to completion, or nearly to completion. Instead of working up this intermediate product, the next set of reagents of step 2 are then added and the reaction again allowed to proceed to completion. Such telescoping synthesis, or telescoping reactions, may also be more commonly referred to as a "single pot" or "one pot" reactions. Such reactions are traditionally most favored by chemists because they efficiently avoid the need for possibly lengthy purification processes which may need to be employed to isolate intermediates between each reaction step.

The compound ingenol-3-angelate may be alternatively referred to throughout the present application, and may be known in various literature publications in the field, as ingenol mebutate, PEP005, PICATO®, **29,** and CAS 75567-37-2.

The compound ingenol may be alternatively referred to throughout the present application, and may be known in various literature publications in the field, as CAS 30220-46-3, C₂₀H₂₈O₅, **21,** (1aR,2S,5R,5aR,6S,8aS,9R,10aR)-1a,2,5,5a,6,9,10,10a-Octahydro-5,5a,6-trihydroxy-4-(hydroxymethyl)-1,1,7,9-tetramethyl-1H-2,8a-methanocyclopenta[a]cyclopropa-[e]cyclodecen-11-one.

The term "aromatic" used in the present application means an aromatic group which has at least one ring having a conjugated pi electron system, *i.e.,* aromatic carbon molecules having 4n+2 delocalized electrons, according to Hückel's rule, and includes both carbocyclic aryl, *e.g.,* phenyl, and heterocyclic aryl groups, *e*.*g*., pyridine. The term includes monocyclic or fused-ring polycyclic, *i.e.,* rings which share adjacent pairs of carbon atoms, groups.

The term "aromatic" when used in the context of "aromatic solvent" as used in the present disclosure means any of the known and/or commercially available aromatic solvents, such as, but not limited to, toluene, benzene, xylenes, any of the Kesols, and/or GaroSOLs, and derivatives and mixtures thereof.

The term "alkyl," by itself or as part of another substituent means, unless otherwise stated, a straight or branched chain, or cyclic hydrocarbon radical, or combination thereof, which may be fully saturated, mono- or polyunsaturated and can include di- and multivalent radicals, having the number of carbon atoms designated, *i.e.* C₁-C₁₀ means one to ten carbon atoms in a chain. Non-limiting examples of saturated hydrocarbon radicals include groups such as methyl, ethyl, n-propyl, isopropyl, n-butyl, t-butyl, isobutyl, sec-butyl, cyclohexyl, (cyclohexyl)methyl, cyclopropylmethyl, homologs and isomers of, for example, n-pentyl, n-hexyl, n-heptyl, n-octyl, and the like. An unsaturated alkyl group is one having one or more double bonds or triple bonds. Examples of unsaturated alkyl groups include, but are not limited to, vinyl, 2-propenyl, crotyl, 2-isopentenyl, 2-(butadienyl), 2,4-pentadienyl, 3-(1,4-pentadienyl), ethynyl, 1- and 3-propynyl, 3-butynyl, and the higher homologs and isomers. The term "alkyl," unless otherwise noted, is also meant to include those derivatives of alkyl defined in more detail below, such as "heteroalkyl."

The term "alkylene" by itself or as part of another substituent means a divalent radical derived from an alkane, as exemplified, but not limited, by --CH₂CH₂CH₂CH₂--, and further includes those groups described below as "heteroalkylene." Typically, an alkyl (or alkylene) group will have from 1 to 24 carbon atoms, with those groups having 10 or fewer carbon atoms being preferred in the present invention. A "lower alkyl" or "lower alkylene" is a shorter chain alkyl or alkylene group, generally having eight or fewer carbon atoms.
The term "alkynyl" is intended to indicate a hydrocarbon radical comprising 1-3 triple C-C bonds and 2-10 carbon atoms, typically comprising 2-6 carbon atoms, in particular 2-4 carbon atoms, such as 2-3 carbon atoms, e.g. ethynyl, propynyl, butynyl, pentynyl or hexynyl.

The terms "alkoxy," "alkylamino" and "alkylthio" (or thioalkoxy) are used in their conventional sense, and refer to those alkyl groups attached to the remainder of the molecule *via* an oxygen atom, an amino group, or a sulfur atom, respectively.

The term "heteroalkyl," by itself or in combination with another term, means, unless otherwise stated, a stable straight or branched chain, or cyclic hydrocarbon radical, or combinations thereof, consisting of the stated number of carbon atoms and at least one heteroatom selected from the group consisting of O, N, Si and S, and wherein the nitrogen and sulfur atoms may optionally be oxidized and the nitrogen heteroatom may optionally be quaternized. The heteroatom(s) O, N and S and Si may be placed at any interior position of the heteroalkyl group or at the position at which the alkyl group is attached to the remainder of the molecule. Examples include, but are not limited to, -CH₂-CH₂-O-CH₃, -CH₂-CH₂-NH-CH₃,-CH₂-CH₂-N(CH₃)-CH₃, -CH₂-S-CH₂-CH₃, -CH₂-CH₂, -S(O)-CH₂, -CH₂-CH₂-S(O)₂-CH₃,-CHCH-O-CH₃, -Si(CH₃)₃, -CH₂-CHN-OCH₃, and -CHCH-N(CH₃)-CH₃. Up to two heteroatoms may be consecutive, such as, for example, -CH₂-NH-OCH₃ and -CH₂-O-Si(CH₃)₃. Similarly, the term "heteroalkylene" by itself or as part of another substituent means a divalent radical derived from heteroalkyl, as exemplified, but not limited by, -CH₂-CH₂-S-CH₂-CH₂- and -CH₂-S-CH₂-CH₂-NH-CH₂-. For heteroalkylene groups, heteroatoms can also occupy either or both of the chain termini, *e*.*g*., alkyleneoxy, alkylenedioxy, alkyleneamino, alkylenediamino, and the like. Still further, for alkylene and heteroalkylene linking groups, no orientation of the linking group is implied by the direction in which the formula of the linking group is written. For example, the formula -C(O)₂R'- represents both -C(O)₂R'- and -R'C(O)₂-.

The terms "cycloalkyl" and "heterocycloalkyl," by themselves or in combination with other terms, represent, unless otherwise stated, cyclic versions of "alkyl" and "heteroalkyl," respectively. Additionally, for heterocycloalkyl, a heteroatom can occupy the position at which the heterocycle is attached to the remainder of the molecule. Examples of cycloalkyl include, but are not limited to, cyclopentyl, cyclohexyl, 1-cyclohexenyl, 3-cyclohexenyl, cycloheptyl, and the like. Examples of heterocycloalkyl include, but are not limited to, 1-(1,2,5,6-tetrahydropyridyl), 1-piperidinyl, 2-piperidinyl, 3-piperidinyl, 4-morpholinyl, 3-morpholinyl, tetrahydrofuran-2-yl, tetrahydrofuran-3-yl, tetrahydrothien-2-yl, tetrahydrothien-3-yl, 1-piperazinyl, 2-piperazinyl, and the like.

The terms "halo" or "halogen," by themselves or as part of another substituent, mean, unless otherwise stated, a fluorine, chlorine, bromine, or iodine atom. Additionally, terms such as "haloalkyl," are meant to include monohaloalkyl and polyhaloalkyl. For example, the term "halo(C₁-C₄)alkyl" is mean to include, but not be limited to, trifluoromethyl, 2,2,2-trifluoroethyl, 4-chlorobutyl, 3-bromopropyl, and the like.

The term "aryl" means, unless otherwise stated, a polyunsaturated, aromatic, substituent that can be a single ring or multiple rings (preferably from 1 to 5 rings), which are fused together or linked covalently. The term "heteroaryl" refers to aryl groups (or rings) that contain from one to four heteroatoms selected from N, O, and S, wherein the nitrogen and sulfur atoms are optionally oxidized, and the nitrogen atom(s) are optionally quaternized. A heteroaryl group can be attached to the remainder of the molecule through a heteroatom. Non-limiting examples of aryl and heteroaryl groups include phenyl, 1-naphthyl, 2-naphthyl, 4-biphenyl, 1-pyrrolyl, 2-pyrrolyl, 3-pyrrolyl, 3-pyrazolyl, 2-imidazolyl, 4-imidazolyl, pyrazinyl, 2-oxazolyl, 4-oxazolyl, 2-phenyl-4-oxazolyl, 5-oxazolyl, 3-isoxazolyl, 4-isoxazolyl, 5-isoxazolyl, 2-thiazolyl, 4-thiazolyl, 5-thiazolyl, 2-furyl, 3-furyl, 2-thienyl, 3-thienyl, 2-pyridyl, 3-pyridyl, 4-pyridyl, 2-pyrimidyl, 4-pyrimidyl, 5-benzothiazolyl, purinyl, 2-benzimidazolyl, 5-indolyl, 1-isoquinolyl, 5-isoquinolyl, 2-quinoxalinyl, 5-quinoxalinyl, 3-quinolyl, tetrazolyl, benzo[b]furanyl, benzo[b]thienyl, 2,3-dihydrobenzo[1,4]dioxin-6-yl, benzo[1,3]dioxol-5-yl and 6-quinolyl. Substituents for each of the above noted aryl and heteroaryl ring systems are selected from the group of acceptable substituents described below.

For brevity, the term "aryl" when used in combination with other terms, *e*.*g*., aryloxy, arylthioxy, arylalkyl, includes both aryl and heteroaryl rings as defined above. Thus, the term "arylalkyl" is meant to include those radicals in which an aryl group is attached to an alkyl group, *e.g.,* benzyl, phenethyl, pyridylmethyl and the like, including those alkyl groups in which a carbon atom, *e.g.,* a methylene group, has been replaced by, for example, an oxygen atom, *e.g.,* phenoxymethyl, 2-pyridyloxymethyl, 3-(1-naphthyloxy)propyl, and the like.

Each of the above terms, *e*.*g*., "alkyl," "heteroalkyl," "aryl" and "heteroaryl," is meant to include both substituted and unsubstituted forms of the indicated radical. Preferred substituents for each type of radical are provided below.

Substituents for the alkyl and heteroalkyl radicals, including those groups often referred to as alkylene, alkenyl, heteroalkylene, heteroalkenyl, alkynyl, cycloalkyl, heterocycloalkyl, cycloalkenyl, and heterocycloalkenyl, are generically referred to as "alkyl group substituents," and they can be one or more of a variety of groups selected from, but not limited to: --OR', =O, =NR', =N--OR', --NR'R", --SR', -halogen, --SiR'R"R"', --OC(O)R', --C(O)R', --CO₂R',-CONR'R", --OC(O)NR'R", --NR"C(O)R', --NR'--C(O)NR"R"', --NR"C(O)₂R', --NR-C(NR'R"R"')=NR"", --NR--C(NR'R")=NR"', --S(O)R', --S(O)₂R', --S(O)₂NR'R", --NRSO₂R',-CN and --NO₂ in a number ranging from zero to (2M'+1), where M' is the total number of carbon atoms in such radical. R', R", R'" and R"" each preferably independently refer to hydrogen, substituted or unsubstituted heteroalkyl, substituted or unsubstituted aryl, *e.g.,* aryl substituted with 1-3 halogens, substituted or unsubstituted alkyl, alkoxy or thioalkoxy groups, or arylalkyl groups. When a compound of the invention includes more than one R group, for example, each of the R groups is independently selected as are each R', R", R'" and R"" groups when more than one of these groups is present. When R' and R" are attached to the same nitrogen atom, they can be combined with the nitrogen atom to form a 5-, 6-, or 7-membered ring. For example, --NR'R" is meant to include, but not be limited to, 1-pyrrolidinyl and 4-morpholinyl. From the above discussion of substituents, one of skill in the art will understand that the term "alkyl" is meant to include groups including carbon atoms bound to groups other than hydrogen groups, such as haloalkyl, *e.g.,* --CF₃ and --CH₂CF₃) and acyl, *e.g.,* --C(O)CH₃, --C(O)CF₃, --C(O)CH₂OCH₃, and the like).

Similar to the substituents described for the alkyl radical, substituents for the aryl and heteroaryl groups are generically referred to as "aryl group substituents." The substituents are selected from, for example: halogen, --OR', =O, =NR', =N--OR', --NR'R", --SR', -halogen,-SiR'R"R"', --OC(O)R', --C(O)R', --CO₂R', --CONR'R", --OC(O)NR'R", --NR"C(O)R', --NR'-C(O)NR"R"', --NR"C(O)₂R', --NR--C(NR'R"R"')=NR"', --NR--C(NR'R")=NR"', --S(O)R', - S(O)₂R', --S(O)₂NR'R", --NRSO₂R', --CN and --NO₂, --R', --N₃, --CH(Ph)₂, fluoro(C₁-C₄)alkoxy, and fluoro(C₁-C₄)alkyl, in a number ranging from zero to the total number of open valences on the aromatic ring system; and where R', R", R'" and R"" are preferably independently selected from hydrogen, substituted or unsubstituted alkyl, substituted or unsubstituted heteroalkyl, substituted or unsubstituted aryl and substituted or unsubstituted heteroaryl. When a compound of the invention includes more than one R group, for example, each of the R groups is independently selected as are each R', R", R'" and R"" groups when more than one of these groups is present. In the schemes that follow, the symbol X represents "R" as described above.
The term 'catalytic amount' is intended to indicate an amount of catalyst which is smaller than the stoichiometric amount relative to the reactant to be transformed. A catalytic amount of osmium tetraoxide in the dihydroxylation of an alkene is thus intended to indicate an amount of osmium tetraoxide expressed in moles which is less than the amount of alkene, expressed in moles, to be dihydroxylated. Catalytic amounts of osmium tetraoxide is for example 0.5-20, 1-10, 2-7, 3-5 or 5 mole percent relative to alkene-derivative to be dihydroxylated.
The term 'effective buffer range' is intended to indicate a pH range where a buffer effectively neutralizes added acids and bases, while maintaining a relatively constant pH.

Applicants are aware that there are many conventions and systems by which organic compounds may be named and otherwise described, including common names as well as systems, such as the IUPAC system. Not wishing to be bound by such systems or names, Applicants offer hereinbelow one possible set of chemical names for each of the intermediates disclosed herein. These names are not meant to be in any way limiting and in all instances where doubt or contradiction occurs between the names provided below and the structures depicted herein, the structure takes priority and is at all times intended to convey Applicant's meaning.
**1:** (1*S*,6*R*)-3,7,7-trimethylbicyclo[4.1.0]hept-3-ene
**2:** (1*R*,3*R*,6*S*)-3-chloro-7,7-dimethyl-4-methylenebicyclo[4.1.0]heptane
**3:** (1*S*,4*R*,6*R*)-4-chloro-7,7-dimethylbicyclo[4.1.0]heptan-3-one
**4:** (1*S*,4*R*,6*R*)-4,7,7-trimethylbicyclo[4.1.0]heptan-3-one
**5:** (1*R*,2*R*,4*R*,6*R*)-2-((1*R*,2*R*)-1-hydroxy-2-methyl-penta-3,4-dien-1-yl)-4,7,7-trimethylbicyclo[4.1.0]heptan-3-one
**6:** (1*R*,2*R*,3*R*,4*R*,6*R*)-2-((1*R*,2*R*)-1-hydroxy-2-methyl-penta-3,4-dien-1-yl)-4,7,7-trimethyl-3-((trimethylsilyl)ethynyl)bicyclo[4.1.0]heptan-3-0l
**7**: (1*R*,2*R*,3*R*,4*R*,6*R*)-3-ethyny1-2-((1*R*,2*R*)-1-hydroxy-2-methyl-penta-3,4-dien-1-yl)-4,7,7-trimethylbicyclo[4.1.0]heptan-3-ol
**8:** (1*R*,2*R*,3*R*,4*R*,6*R*)-2-((1*R*,2*R*)-1-((*tert*-butyldimethylsilyl)oxy)-2-methyl-penta-3,4-dien-1-yl)-3-ethynyl-4,7,7-trimethylbicyclo[4.1.0]heptan-3-ol
**9**: *tert*-butyl(((1*R,*2*R*)-1-((1*R,*2*R,*3*R,*4*R,*6*R)*-3-ethynyl-4,7,7-trimethyl-3-((trimethylsilyl)oxy) bicyclo[4.1.0]heptan-2-yl)-2-methyl-penta-3,4-dien-1-yl)oxy)dimethylsilane
**10:** (1a*R*,1b*R*,2*R*,3*R*,7b*R*,8*R*,9a*R*)-2-((*tert*-butyldimethylsilyl)oxy)-1,1,3,8-tetramethyl-7b-((trimthylsilyl)oxy)-1,1a,1b,2,3,5,7b,8,9,9a-decahydro-6*H*-cyclopropa[3,4]benzo[1,2-*e*]azulen-6-one
**11:** (1a*R*,1b*R*,2*R*,3*R*,6*S*,7b*R*,8*R*,9a*R*)-2-((*tert*-butyldimethylsilyl)oxy)-1,1,3,6,8-pentamethyl-7b-((trimethylsilyl)oxy)-1a,1b,2,3,5,6,7b,8,9,9a-decahydro-1H-cyclopropa[3,4]benzo[1,2-*e*] azulen-6-ol
**12:** (1a*R*,1b*R*,2*R*,3*R*,4*R*,4a*S*,6*S*,7b*R*,8*R*,9a*R*)-2-((*tert*-butyldimethylsilyl)oxy)-1,1,3,6,8-pentamethyl-7b-((trimethylsilyl)oxy)-1,1a,1b,2,3,4,5,6,7b,8,9,9a-dodecahydro-4a*H*-cyclopropa[3,4] benzo[1,2-*e*]azulene-4,4a,6-triol
**13:** (2*S*,3a*S*,6a*R*,7*R*,8*R*,8a*R*,8b*R*,9a*R*,11*R*,11a*R*)-8-((*tert*-butyldimethylsilyl)oxy)-2-hydroxy-2,7,9,9,11-pentamethyl-11a-((trimethylsilyl)oxy)-2,3,6a,7,8,8a,8b,9,9a,10,11,11a-dodecahydro-cyclopropa[5',6']benzo[1',2':7,8]azuleno[3a,4-*d*][1,3]dioxol-5-one
**14:** (3a*S*,6a*R*,7*R*,8*R*,9*R*,9a*R*,10a*R*,12*R*,12a*S*)-8-((*tert*-butyldimethylsilyl)oxy)-2,7,10,10,12-pentamethyl-6a,7,9,9a,10,10a,11,12-octahydro-3*H*,8*H*-9,12a-methanocyclopenta[1,10]cyclo-propa[6,7]cyclodeca[1,2-*d*][1,3]dioxole-5,13-dione
**15:** (3*S*,3a*R*,6a*R*,7*R*,8*R*,9*R*,9a*R*,10a*R*,12*R*,12a*S*)-8-((*tert*-butyldimethylsilyl)oxy)-3-hydroxy-2,7,10,10,12-pentamethyl-6a,7,9,9a,10,10a,11,12-octahydro-3*H*,8*H*-9,12a-methanocyclopenta[1,10]cyclopropa[6,7]cyclodeca[1,2-*d*][1,3]dioxole-5,13-dione
**16:** (3*S*,3a*S*,6a*R*,7*R*,8*R*,9*R*,9a*R*,10a*R*,12*R*,12a*S*)-8-((*tert*-butyldimethylsilyl)oxy)-2,7,10,10,12-pentamethyl-5,13-dioxo-6a,7,9,9a,10,10a,11,12-octahydro-3*H*,8*H*-9,12a-methanocyclopenta [1,10]cyclopropa[6,7]cyclodeca[1,2-*d*][1,3]dioxol-3-yl acetate
**17:** (3*S*,3a*S*,6a*R*,7*S*,8*R*,9*R*,9a*R*,10a*R*,12*R*,12a*S*)-8-hydroxy-2,7,10,10,12-pentamethyl-5,13-dioxo-6a,7,9,9a,10,10a,11,12-octahydro-3*H*,8*H*-9,12a-methanocyclopenta[1,10]cyclopropa[6,7] cyclodeca[1,2-*d*][1,3]dioxol-3-yl acetate
**18:** (3*S*,3a*S*,6a*R*,7*R*,8*R*,9*R*,9a*R*,10a*R*,12*R*,12a*S*)-2,7,10,10,12-pentamethyl-5,13-dioxo-8-(((trifluoromethyl)sulfonyl)oxy)-6a,7,9,9a,10,10a,11,12-octahydro-3*H*,8*H*-9,12a-methanocyclopenta[1,10]cyclopropa[6,7]cyclodeca[1,2-*d*][1,3]dioxol-3-yl acetate
**19:** (3*S*,3a*R*,6a*R*,9*S*,9a*R*, 10a*R*,12*R*,12a*S*)-2,7,10,10,12-pentamethyl-5,13-dioxo-6a,9,9a, 10a, 11,12-hexahydro-3*H*,10*H*-9,12a-methanocyclopenta[1,10]cyclopropa[6,7]cyclodeca[1,2-*d*][1,3]dioxol-3-yl acetate
**20:** (1a*R*,2*S*,5*R*,5a*S*,6*S*,8a*S*,9*R*,10a*R*)-5,5a,6-trihydroxy-1,1,4,7,9-pentamethyl-1a,2,5,5a,6,9,10,10a-octahydro-1*H*-2,8a-methanocyclopenta[*a*]cyclopropa[*e*][10]annulen-11-one
**21:** (1a*R*,2*S*,5*R*,5a*R*,6*S*,8a*S*,9*R*,10a*R*)-5,5a,6-trihydroxy-4-(hydroxymethyl)-1,1,7,9-tetra-methyl-1a,2,5,5a,6,9,10,10a-octahydro-1*H*-2,8a-methanocyclopenta[*a*]cyclopropa[*e*][10] annulen-11-one
**22:** (*R*)-2-methylpenta-3,4-dien-1-ol
**23:** (*R*)-2-methylpenta-3,4-dien-1-al
**24:** (1a*R*,1b*R*,2*R*,3*R*,4*R*,4a*S*,7b*R*,8*R*,9a*R*)-2-((*tert*-butyldimethylsilyl)oxy)-4,4a-dihydroxy-1,1,3,8-tetramethyl-7b-((trimethylsilyl)oxy)-1,1a,1b,2,3,4,4a,5,7b,8,9,9a-dodecahydro-6*H-*cyclopropa[3,4]benzo[1,2-*e*]azulen-6-one
**27:** (3a*S*,6a*R*,7*S*,8*R*,9*R*,9a*R*,10a*R*,12*R*,12a*S*)-8-hydroxy-2,7,10,10,12-pentamethyl-6a,7,9,9a,10,10a,11,12-octahydro-3*H*,8*H*-9,12a-methanocyclopenta[1,10]cyclopropa[6,7]cyclodeca[1,2-*d*][1,3]dioxole-5,13-dione
**28:** (3a*S*,6a*R*,9*S*,9a*R*,10a*R*,12*R*,12a*S*)-2,7,10,10,12-pentamethyl-6a,9,9a,10a,11,12-hexahydro-3*H*,10*H*-9,12a-methanocyclopenta[1,10]cyclopropa[6,7]cyclodeca[1,2-*d*][1,3]dioxole-5,13-dione
**29:** (1a*R*,2*S*,5*R*,5a*S*,6*S*,8a*S*,9*R*,10a*R*)-5,5a-dihydroxy-4-(hydroxymethyl)-1,1,7,9-tetramethyl-11-oxo-1a,2,5,5a,6,9,10,10a-octahydro-1*H*-2,8a-methanocyclopenta[*a*]cyclopropa[*e*][10]annulen-6-yl (Z)-2-methylbut-2-enoate

### Abbreviations

Abbreviations used throughout the present application have the meanings provided below. The meanings provided below are not meant to be limiting, but are meant to also encompass any equivalent common or systematic names understood by one of skill in the art. The meaning commonly understood by one of skill in the art should be ascribed to any other abbreviated names not listed below.
Ac: acyl
Ac₂O: acetic anhydride, ethanoic anhydride
BF₃: boron trifluoride
CDI: carbonyldiimidazole
CeCl₃: cerium (III) chloride, cerous chloride, cerium trichloride
CH₃CN: acetonitrile
CO: carbon monoxide
DABCO: 1,4-diazabicyclo[2.2.2]octane
DBB: di-tert-butyl-biphenyl
DBU: 1,8-Diazabicyclo[5.4.0]undec-7-ene, diazabicycloundecene
DCM: dichloromethane, CH₂Cl₂
DMAP: dimethylaminopyridine
DMS: dimethylsulfide
Et₂O: diethyl ethyer, ethyl ether, ether
Et₃N: triethylamine
EtOAc: ethyl acetate
HF: hydrogen fluoride
HMPA: hexamethylphosphoramide
IBX: 2-iodoxybenzoic acid, *O*-iodobenzoic acid
K₂CO₃: potassium carbonate
KHMDS: potassium hexamethylphosphoramide
LDA: lithium diisopropylamide, [(CH₃)₂CH]₂NLi
LiDBB: lithium di-tert-butyl-biphenyl
LiHMDS: lithium bis(trimethylsilyl)amide
LN: lithium-naphthalenide
MeI: methyliodide
MeLi: methyl lithium
MeOH: methanol
NH₄Cl: ammonium chloride
NaHCO₃: sodium bicarbonate
NaHMDS: sodium hexamethylphosphoramide ((CH₃)₃Si)₂NNa
Na₂SO₃: sodium sulfite
Na₂SO₄: sodium sulfate
n-BuLi: n-butyl lithium
NCS: N-chlorosuccinimide
NMO: *N*-methylmorpholine-N-oxide
O₃: Ozone
OsO₄: osmium tetraoxide
PhMe: toluene, phenylmethyl
Py: pyridine
[RhCl(CO)₂]₂: chlorodicarbonylrhodium(I) dimer
[RhCl(COD)]₂: cyclooctadiene rhodium chloride dimer[RhCl(Dppp)₂]Cl: bis[1,3-bis(diphenyl phosphine)propane] rhodium chloride
SeO₂: selenium dioxide
SiO₂: silicon dioxide
TBAF: tetra-*n*-butylammonium fluoride, (CH₃CH₂CH₂CH₂)₄N⁺F⁻
TBHP: Tert-butyl hydroperoxide
TBS: *tert*-butyldimethylsilyl ether, TBDMS
TBSOTf: trifluoromethanesulfonic acid *tert*-butyldimethylsilylester, TBDMS triflate
TES: triethylsilyl ether
Tf: trifluoromethanesulfonate, triflate
Tf₂O: trifluoromethanesulfonic anhydride
THF: tetrahydrofuran
TIPS: triisopropyl silyl ether
TMANO: Trimethylamine-*N*-oxide
TMS: trimethylsilylacetylene
TMSOTf: trifluoromethanesulfonic acid trimethylsilylester, TMS triflate

Additionally, numbered compounds are referred to generally as "formulas" which correspond to the number of the compound, *i.e.* numbered compound **1** is identical to, and will be referred to interchangeably as, formula **1,** compound **1,** or just **1,** for example.

### Synthetic Methods

Generally, the methods provided below are reflected in the longer synthetic route of *Scheme 1* (Figure 1) and the shorter synthetic route of *Scheme 2* (Figure 2). However, slight modifications of these two routes, presenting other synthetic route options, are also provided in *Scheme 4* and *Scheme 5. Scheme 1* presents a specific embodiment of the procedures of the present application, but as one of skill in the art will know, various alternative sets of reagents may be utilized in various steps, as are indicated below as optional. It is also contemplated that the end product, ingenol, may be used in further synthetic steps to create other derivatives, as reported in the literature and summarized, above, including, for instance, ingenol-3-angelate (**29**).

The size and scale of the synthetic methods will vary depending on the desired amount of end product. Contemplated quantities of end product include, but are not limited to, one or more micrograms, one or milligrams, one or more grams and/or one or more kilograms of ingenol.

Important intermediates in the present methods are highlighted below. But it is to be understood that other intermediates, other than those highlighted, will also have advantageous uses in other synthetic chemistry protocols for their ability to shorten various synthetic routes to other end products. Thus, all intermediates disclosed herein are believed to be unique, advantageous and useful for various purposes.

Furthermore, although one may begin the synthesis of ingenol at step 1, below, and proceed to the end product of ingenol, one may also desire simply to achieve synthesis of one or more key intermediates, such as **34, 35,** or **38,** for instance. In these instances, it is contemplated that the process will be followed only until the desired intermediate is achieved. Thus, the synthetic methods disclosed include synthesis of all intermediates disclosed herein as if each intermediate were considered to be the desired end product. That is, the methods contemplate the disclosure of the efficient synthesis of each and every intermediate disclosed herein as if each is a desired, useful and advantageous end product unto itself. Therefore, the methods contemplated herein include methods of synthesis involving all of the disclosed steps, some of the disclosed steps, one or more of the disclosed steps, or any combination thereof. Disclosed methods include, for instance, the synthesis of **34,** involving only steps 1-9, described below, or the synthesis of **40,** involving only steps 1-15, of *Scheme 1,* etc.

In all instances, where a drying agent is used, contemplated drying agents include all those reported in the literature and known to one of skill, such as, but not limited to, magnesium sulfate, sodium sulfate, calcium sulfate, calcium chloride, potassium chloride, potassium hydroxide, sulfuric acid, quicklime, phosphorous pentoxide, potassium carbonate, sodium, silica gel, aluminum oxide, calcium hydride, lithium aluminum hydride (LAH), potassium hydroxide, and the like. (*See,* Burfield et al., "Dessicant Efficiency in Solvent Drying. A Reappraisal by Application of a Novel Method for Solvent Water Assay," J. Org. Chem., 42(18):3060-3065, 1977). The amount of drying agent to add in each work up may be optimized by one of skill in the art and is not particularly limited. Further, although general guidance is provided for work-up of the intermediates in each step, it is generally understood by one of skill that other optional solvents and reagents may be equally substituted during the work-up steps. However, in some exceptional instances, it was found the very specific work-up conditions are required to maintain an unstable intermediate. Those instances are indicated below in the steps in which they occur.

Many of the steps below indicate various work-ups following termination of the reaction. A work-up involves generally quenching of a reaction to terminate any remaining catalytic activity and starting reagents. This is generally followed by addition of an organic solvent and separation of the aqueous layer from the organic layer. The product is typically obtained from the organic layer and unused reactants and other spurious side products and unwanted chemicals are generally trapped in the aqueous layer and discarded. The work-up in standard organic synthetic procedures found throughout the literature is generally followed by drying the product by exposure to a drying agent to remove any excess water or aqueous byproducts remaining partially dissolved in the organic layer and concentration of the remaining organic layer. Concentration of product dissolved in solvent may be achieved by any known means, such as evaporation under pressure, evaporation under increased temperature and pressure, and the like. Such concentrating may be achieved by use of standard laboratory equipment such as rotary-evaporator distillation, and the like. This is optionally followed by one or more purification steps which may include, but is not limited to, flash column chromatography, filtration through various media and/or other preparative methods known in the art and/or crystallization/recrystallization. (*See,* for instance, Addison Ault, "Techniques and Experiments for Organic Chemistry," 6th Ed., University Science Books, Sausalito, CA, 1998, Ann B. McGuire, Ed., pp. 45-59). Though certain organic co-solvents and quenching agents may be indicated in the steps described below, other equivalent organic solvents and quenching agents known to one of skill may be employed equally as well and are fully contemplated herein. Further, most of the work-ups in most steps may be further altered according to preference and desired end use or end product. Drying and evaporation, routine steps at the organic synthetic chemist bench, need not be employed and may be considered in all steps to be optional. The number of extractions with organic solvent may be as many as one, two, three, four, five, or ten or more, depending on the desired result and scale of reaction. Except where specifically noted, the volume, amount of quenching agent, and volume of organic solvents used in the work-up may be varied depending on specific reaction conditions and optimized to yield the best results.

Additionally, where inert gas or noble gas is indicated, any inert gas commonly used in the art may be substituted for the indicated inert gas, such as argon, nitrogen, helium, neon, etc.

### Step 1: Chlorination of 1 to produce 2

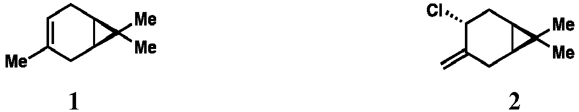

Compound of formula **1** (SIGMA-ALDRICH, Inc., St. Louis, MO) is dissolved in solvent. In one embodiment, the solvent is dichloromethane. To a solution of **1** in dichloromethane (CH₂Cl₂, DCM) is added a chlorinating reagent and a catalytic amount of dimethylaminopyridine (DMAP). Various other chlorinating/oxidizing reagents are known and can be substituted in this step to achieve similar results, such as *N*-chlorosuccinimide (NCS), bleach (NaOCl) and CeCl₃, *t*BuOCl, trichloroisocyanuric acid. Stirring may be for a period of about three hours or more, depending on the scale of the reaction, *i.e.,* the quantity of starting material. The reaction may be monitored and continued at room temperature (r.t.) with stirring until the desired quantity of product is achieved. Various quantities of starting material may produce variable yield and require a higher temperature to be used to speed the reaction and increase yield. Thus, adjustment of reaction time and reagents may be necessary to optimize the yield desired based on the quantity of starting materials. For more on formation of optically active 2- and 3-carene systems, see Paquette et al., "Regioselective Routes to Nucleophilic Optically Active 2- and 3-Carene Systems," J. Org. Chem., 55:1589-1598, 1990, incorporated herein by reference in its entirety for all purposes.

Upon termination of the reaction, pentane is added and stirring is continued until the solution turns into a suspension. The resulting suspension may be filtered through a pad of SiO₂. Alternatively, pentanes may be avoided and the reaction simply allowed to proceed to completion, after which aqueous solution, such as water, is added and the organic layer is separated, washed with saturated aqueous salt solution, preferably sodium chloride (NaCl), and dried over an appropriate drying agent, such as, but not limited to, sodium sulfate (Na₂SO₄₎, or any of the other drying agents previously disclosed herein. The product may then be filtered and concentrated under reduced pressure. The resultant residue may optionally be further purified to remove excess succinimide, for instance by flash column chromatography (preparative thin-layer chromatography (TLC), silica gel, pentane or hexane) to yield the product **2** as a colorless liquid.

### Step 2: Ozonolysis of 2 to yield 3

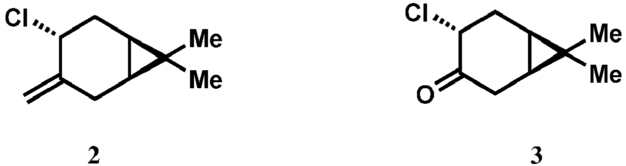

The intermediate **2** (crude or further purified), obtained from Step 1 may be dissolved in a solvent, such as a 2:1 mixture (or other ratios, such as, but not limited to, 3:1, 4:1, 5:1, etc.) of dichloromethane:methanol (CH₂Cl₂:MeOH) or any other appropriate solvent, such as chloroform, diethyl ether, or ethyl acetate. However, it is noted that use of methanol may be used as at least one solvent in this step. The solution is then cooled to at least -78 °C or lower. The solution is ozonized until it turns dark-blue. Ozone may be produced by any of the known means of causing ozonolysis, including, but not limited to, generation of ozone (O₃) by corona discharge, ultraviolet light, or electrolysis. Exposure to ozone is recommended until the solution turns dark blue in color. This may require incubation for 1 hr, 2 hr, 3 hr, 4 hr, or any time between, depending on the amount of starting material, selected solvent and other condition variables. Product formation may also be monitored in real time by known means. Excess ozone may be removed from the solution with air or O₂ until the solution returns to colorless. Thiourea and optionally dimethyl sulfide (DMS, (CH₃)₂S) (or other reducing agents) is added and the reaction mixture is warmed, preferably to room temperature, and stirring may be continued for a period of time, such as several hours, or as many as, for instance, 1 hour, 2 hours, 3 hours, or 4 hours or more.

As in Step 1, work up is by addition of aqueous media, such as water and brine, separation of the organic layer and extraction of the aqueous layer. Extraction may be accomplished with, for instance, dichloromethane, or other appropriate extracting agent, such as chloroform, diethyl ether or ethyl acetate. The extracted organic layers may then be combined and dried over an appropriate drying agent, such as Na₂SO₄, then filtered and concentrated *in vacuo.*

Optionally, the product **3** may be purified by flash column chromatography, using various known and appropriate developing solvent combinations that allow physical separation of the product **3** from starting material **2,** such as, for instance, silica gel packed in dichloromethane or similar solvent, then pentane/Et₂O = 20:1 → 10:1, to yield chloro-ketone 3 as a colorless liquid.

Note that conversion of **1** to **3** may proceed in a single pot. That is, steps 1 and 2 may be performed in one pot, as depicted in *Scheme 2* (Figure 2). This is achieved by what is commonly referred to as addition of "telescoping" reagents. For instance, step 1 reagents may be added to one reaction vessel and the reaction allowed to proceed to completion. Instead of working up this intermediate product, the next set of reagents of step 2 are then added and the reaction again allowed to proceed to completion. The final product **3** is then worked up as indicated and/or further purified as needed for step 3.

While it was found that many intermediates vary in stability, the compound of formula **3** was found to be rather stable so long as it is maintained at in cold temperature at around -20 °C or below.

### Step 3: Reductive alkylation of 3

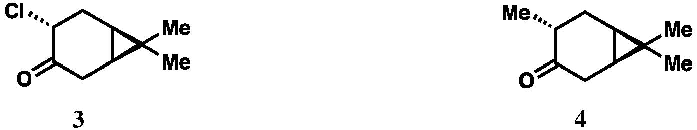

To a three necked glass flask is added a reducing agent, such as naphthalene or di-tert-butylbiphenyl (DBB) and lithium metal in dry THF. Preferably, the lithium metal is freshly cut. Other reducing agents may be used in this step, such as, for instance sodium, potassium, SmI₂. The suspension is then sonicated. Sonication may be for several hours, preferably for 1, 2, 3, 4 or as many hours as needed to yield a dark-green solution. Alternatively, the mixture is stirred for 3 hours or longer, depending on the scale of the reactants, at room temperature. Higher temperatures may be employed to quicken the reaction, if needed.

In a separate flask, a compound of formula **3** is dissolved in an appropriate solvent. Solvents which may be used in this step include, for instance, THF or other aprotic, polar solvents. The solution of **3** is cooled to -78 °C. The temperature of the solution of **3** should be lowered to about -78 °C. Preferably, the temperature is maintained at as close to -78 °C as possible throughout the reaction. Higher temperature may produce spurious side products under some conditions. Thus, even at -50 °C, unwanted products are produced, or no products at all. Thus, the temperature should not be higher or near -50 °C. The temperature should be maintained closer to -78 °C.

The lithium-naphthalenide or lithium di-tert-butylbiphenyl (LiDBB) solution is added to the solution of **3,** for instance by use of a cannula or other means allowing drop-wise addition, over a period of time. Preferably the reducing agent is added over a long enough time to ensure sufficient cooling and maintenance of the -78 °C temperature. Preferably the drop-wise addition may take as long as 20 minutes, 30 minutes, 45 minutes, 60 minutes, or several hours, depending on the amount of starting materials. The reaction is allowed to proceed at -78 °C until a green color persists in the solution.

To the cooled solution, methyl iodide is slowly added and stirring continued at least at a temperature as low as -45 °C (or lower). The methyl iodide is allowed to react for a sufficient amount of time, depending on the amount of starting material. Preferably the reaction proceeds for as long as 2 hours, but may be from 1 to 6 hours, such as 3 hrs, 4 hrs, 5 hrs, or 6 hrs or more. The use of HMPA can allow for shorter reaction times and tolerates higher temperatures (*vide infra*). The reaction in this stage is carefully maintained at as close to -45 °C as possible. Saturated aqueous ammonium chloride (NH₄Cl), aqueous HCl, saturated sodium bicarbonate, or water is added and the reaction mixture is gradually warmed to room temperature.

Work-up involves separation of the organic layer and the aqueous layer, and multiple extractions of the aqueous layer with Et₂O or other appropriate extraction reagent. The combined organic layers are dried, filtered and concentrated under reduced pressure. The crude product may be further optionally purified, for instance by flash column chromatography (silica gel, pentane/Et₂O = 100:1 → 40:1 → 20:1 → 10:1) to yield the methyl ketone product **4** as a light yellow oil.

### Step 4: Aldol Reaction of 4 to Make Intermediate 5

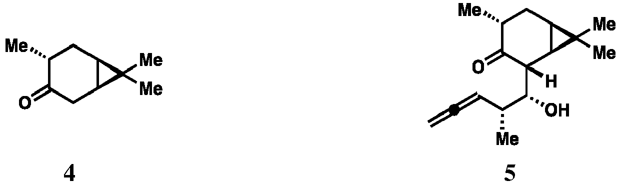

To a solution of **4** in an appropriate aprotic polar solvent, such as THF, under argon or other inert (noble) gas, at -78 °C was added LiHMDS also dissolved in an appropriate solvent, for example, THF. Preferably, the LiHMDS, or any other similar lithium base, used in this step is fresh to obtain the best yields. Other strong amine bases may also be used in this step, for instance LDA, NaHMDS, and KHMDS. The mixture is stirred at -78 °C a period of time sufficient to allow complete deprotonation of **4,** preferably for 1 hour or more, depending on the amount of starting material employed. Then, a solution of **23** in solvent, preferably THF, though other solvents may be used, is slowly added over several minutes. The mixture is then stirred at - 78 °C for sufficient time to allow the reaction to be completed, for instance for several hours or more, perhaps as many as 3 hours, or 4 hours, or even 5 hours, depending on the volume of the reaction and quantity of reactants.

The reaction is then quenched by addition of a sufficient amount of appropriate quencher, such as saturated aqueous NH₄Cl solution, more preferably NH₄Cl and EtOAc. The reaction mixture may be separated and the aqueous layer extracted, for instance with EtOAc. In the work-up, the combined organic fractions are dried, evaporated, and may optionally be further purified by column chromatography, for instance by use of a 5% EtOAc:Hex system (for instance, any suitable solvent system may be employed, such as hexanes/EtOAc = 20:1 → 15:1) to provide **5** as a colorless oil.

Preparation of **23** from a compound of formula **22** proceeds by modification of known procedures for oxidation of alcohols with *o*-iodoxybenzoic acid (*see,* More et. al., "A Simple and Advantageous Protocol for the oxidation of alcohols with o-Iodoxybenzoic Acid (IBX)," Org. Lett., 4(17):3001-3003, 2002), as follows:

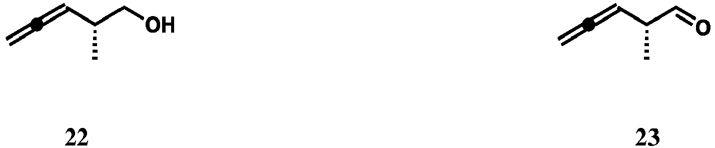

A flask is charged with a solution of **22** in solvent, such as THF. Compound **22** may be obtained by known methods. (*See,* for instance, Konegawa et al., "Enzyme-mediated optical resolution of 2-methyl-3,4-pentadien-1-ol, a chiral building block possessing terminal allenyl group," Synlett, 1997(11):1297-1299, 1997). The reagent 2-iodoxybenzoic acid (IBX) is added to the flask which is then tightly sealed and the resulting suspension heated. The flask should be sealed tightly to permit efficient conversion of the reagent to the product. Heating may continue to as high as 80 °C for as long as sufficient to allow the reaction to go to completion. The suspension is then cooled.

Work-up involves filtration. Filtration may be achieved by use of, for instance, cotton or similar filtration media. The residue is washed, for instance with THF or other aprotic polar solvent, and the filtrate containing **23** may be used in the above aldol reaction without further purification. The compound of formula **23** is preferably prepared fresh, immediately prior to use, to afford the best yield of **5.**

Conversion of **3** to **5** may proceed in a single pot, thereby combining Steps 3 and 4 into a single step. Much effort and experimentation was expended to achieve this efficiency. Surprisingly, it was discovered that the 4-methyl ketone was difficult to isolate, making an apparently simple reaction quite challenging to accomplish in a manner that provided adequate yields. Briefly, a flask charged with naphthalene, freshly distilled THF and freshly cut lithium may be sonicated, as described above. A separate flask may then be charged with **3** and freshly distilled THF and cooled to at least -78 °C. The lithium naphthalene solution may then be slowly added over time to the solution of **3** until a dark-green color persists. Then, a solution of hexamethylphosphoramide (HMPA) and methyl iodide may be added to the dark-green colored solution over time while maintaining the temperature at -78 °C. This reaction is then stirred for an hour or more to allow complete reaction. The reaction flask may then be transferred to a water bath at r.t. and excess methyl iodide removed by vacuum. Upon cooling back to -78 °C, LiHMDS is added drop-wise over time and allowed to stir until reduction is complete, again maintaining the temperature at -78 °C. A freshly prepared solution of aldehyde **23** may then be added with stirring at -78 °C. After several hours the reaction may be quenched. Quenching, as above, may include addition of saturated NH₄Cl. Extraction of the product and work up will yield **5** as above. Extraction may employ a convenient and appropriate solvent, such as EtOAc. Combined organic layers may be dried, filtered and concentrated as above.

### Step 5: Acetylide Addition to 5 to Yield 31

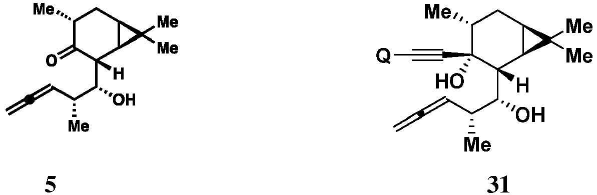

To a solution of trimethylsilylacetylene (TMS), for instance, in THF under argon gas (or other inert gas) at -78° C is added *n*-butyl lithium (*n*-BuLi) in hexanes, or other appropriate solvent needed to achieve anhydrous conditions. Other alkyne protecting groups (**Q**), other than TMS, may be employed in this step. Many alternative alkyne protecting groups are known in the art and may be substituted for TMS in this step, yielding similar or possibly identical results under the proper conditions. The solution is stirred at -78 °C until completion, then added to a suspension of CeCl₃ in THF under argon at -78 °C. THF may be substituted by other appropriate ether solvents, and CeCl₃ may be used as the Lewis acid for this Friedel-Crafts acylation technique. Other approaches may be used to achieve compound **7** as explained in further detail below, where Steps 5 and 6 are optionally combined. The resulting suspension is vigorously stirred at -78 °C. A solution of **5** dissolved in THF is added dropwise to the above solution. This suspension is then stirred at -78 °C until reaction is complete.

In work-up, the reaction is quenched by the addition of water and EtOAc. The layers are separated and the aqueous layer extracted several times with an appropriate extracting solvent, such as EtOAc. The combined extractions are dried and evaporated to yield **31** as a mixture of diastereomers, which may either be resolved or used without further purification.

Although TMS is indicated as one of the protecting groups that may be used in this step, it should be understood that other similar protecting groups such as TBS, TES, and TIPS may also be utilized with similar results.

### Step 6: Desilylation of 31 to Yield 7

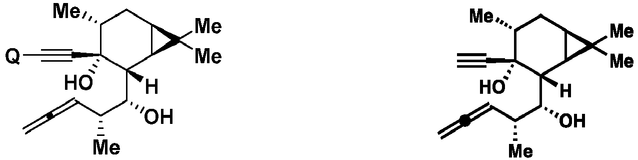

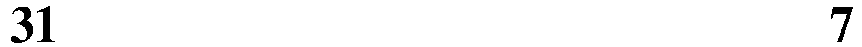

To a solution of **31** dissolved in THF or other appropriate solvent is added tetra-*n-*butylammonium fluoride (TBAF, commercially available) dissolved in THF at 0 °C under inert gas. The stoichiometry of TBAF to **31** is important to maintain adequate levels of fluoride needed to remove the silyl protecting group (**Q**). Preferably the ratio of **31** to TBAF is 1 molar equivalent to 1 molar equivalent. If too much fluoride is used in the reaction, the yield will suffer. One reagent that may be used in this step is TBAF. Other reagents likely will not work as well and it is therefore important to use TBAF in this step. However, other protective groups may be used, other than TMS, in the prior step. If an alternative protecting group is used in the prior step, one would employ the appropriate deprotecting agent known in the art to remove the protecting group used in the prior step. After incubation, a standard work up yields the product. For instance, a solution of EtOAc and H₂O may be added and the organic layer washed with water several times to extract reactants from the deprotected product. The organic layer may be dried and evaporated to provide **7** as a white solid. Removal of most of the amine salt, or as much as possible, may be helpful under some conditions.

Note that conversion of **5** to **7** may proceed in a single pot. That is, steps 5-7 may be performed in one pot, as depicted in *Scheme 2* (Figure 2). As above, this may be achieved by adding telescoping reagents in a single reaction vessel without need to further purify the intermediate **31.** A solution of ketone **5** in THF cooled to -78 °C may have added dropwise to it a Grignard reagent, such as ethynyl magnesium bromide, in THF. Other organolithium reagents may also be useful for this step under some conditions. However, one may use lithium acetylide and like reagents, though lithium acetylide is less desirable since it is not commercially available. The reaction mixture may then be warmed to -10 °C and stirring continued until completion. Saturated aqueous NH₄Cl (10 mL), or other aqueous base, is added as a quenching agent and the mixture extracted several times with organic solvent, such as EtOAc, Et₂O, or DCM. The combined organic layers are dried and optionally further purified, for instance by flash column chromatography (silica gel, hexanes/EtOAc = 10:1 → 5:1) to provide alcohol **7** as a colorless oil.

### Step 7: Protection of 7

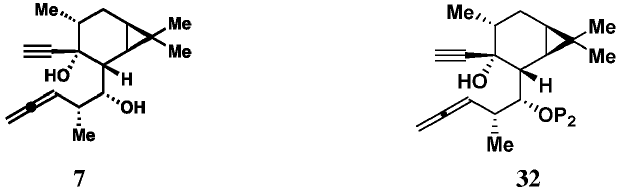

The point of this step is to protect the 7-hydroxyl group on what will eventually be the seven-membered ring of ingenol. Although TBS is one hydroxyl protecting group that may be utilized in this step, it should be understood that other similar protecting groups (**P₂**) such as TMS, TES, TBDPS, and TIPS may also be utilized with similar results.

To a solution of **7** and triethyl amine (Et₃N) in dichloromethane (DCM) under inert gas at 0 °C is added tert-butyldimethylsilyl trifluoromethanesulfonate (TBSOTf), or other protecting group reagent. If a different protecting group is employed, other known conditions will be needed to utilize the selected protecting group. Any other non-nucleophilic base may also be substituted in this reaction. After allowing the reaction to proceed to completion, the reaction may be quenched by the addition of saturated aqueous NaHCO₃ or other appropriate quenching agent. DCM is added to extract the impurities and the layers separated. The aqueous layer may be extracted multiple times with organic solvent, as needed. The combined organic fractions are dried and evaporated to yield **32** as a colorless oil.

### Step 8: Further Protection of 32 to Yield 33

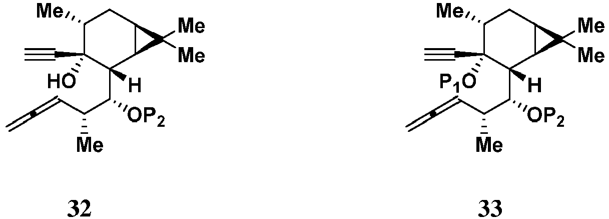

The point of this step is to protect the 10-hydroxyl group on what will eventually become the seven-membered ring of ingenol. Although TMS is one hydroxyl protecting group that may be utilized in this step, it should be understood that other similar protecting groups (**P₁**) such as TBS, TES, or Ac may also be utilized with similar results. For instance, P₁ may be TMS and P₂ may be TBS.

A solution containing **32** dissolved in an effective amount of Et₃N and DCM under inert gas at 0 °C was incubated with an hydroxyl protecting agent, such as TMSOTf. Other amine bases may be substituted in this step with equal, or similar, results, such as (*i*Pr)₂Net (Hünig's base), 2,6-lutidine, or pyridine, and the like. After the reaction is allowed to proceed to completion, the reaction is quenched with an aqueous solution, such as saturated aqueous NaHCO₃ and the product **9** worked up in a typical manner. For instance, an organic solvent, such as DCM may be added and the layers separated. The aqueous layer may be extracted multiple times with DCM and combined. The combined organic fractions are dried, evaporated, and the **9** product may be optionally further purified, for instance by column chromatography in solvent, such as hexanes, to give **33** as a colorless oil.

Note that conversion of **7** to **33** may proceed in a single pot. That is, steps 7 and 8 may performed in one pot, as depicted in *Scheme 2* (Figure 2). Again, as above, reagents may be added in a telescoping manner, so that steps 7 and 8 are performed in a single reaction vessel without the need to isolate intermediate **32,** between steps. For example, to a solution of **7** in CH₂Cl₂ can be added triethylamine then TBSOTf dropwise at 0 °C. Upon reaction end point, triethylamine, then TMSOTf are added dropwise. Endpoint may be monitored by sampling the reaction and visualizing the reactant and product by TLC or other analytical separation means. The reaction mixture is then stirred at 0 °C for a period of time before being quenched with saturated aqueous base, such as NaHCO₃. The mixture can then be extracted several times with standard organic solvents, such as EtOAc and the combined organic layers optionally dried, filtered and concentrated under reduced pressure. Optional purification of the crude product by flash column chromatography (silica gel, pentane) may also be employed.

### Step 9: Pauson-Khand Reaction to Yield 34

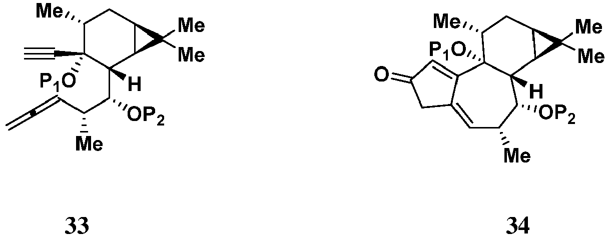

In this step, the core tetracyclic ring structure of ingenol is obtained in a surprising manner. The protecting groups may be as defined above. For instance, P₁ may be TMS and P₂ may be TBS. Many aspects of this conversion are surprising, from the basic fact that the reaction achieved significant yield of **34** to the discovery made after many iterations and attempts that dilute conditions substantially improved yield of **34.** It was surprisingly found that the more dilute the reactants were in the reaction, the better yield was observed at the scales tested, and as presented in the Examples, below. In fact, the yield improved by ∼30% once the reaction was diluted to about 0.005 M. It was further found that the yield was highly sensitive to the quality of the solvent used. Merely changing the solvent from regular xylenes or distilled xylenes to anhydrous para-xylene, a small change that one normally would not predict to substantially impact the reaction, actually drastically increased the yield of **10** at the scale tested. A reaction vessel is charged with **33** in anhydrous *p*-xylene and degassed, for instance using carbon monoxide, with sonication. Though other xylene-based solvents may be utilized, best yields were observed using anhydrous *p*-xylene. Catalytic amounts of a rhodium (I) complex, such as [RhCl(CO)₂]₂ are added to the reaction. The rhodium (I) catalyst useful for this step is a rhodium(I) complex, such as, for example, [RhCl(CO)₂]₂, [RhCl(COD)]₂, [RhCl(CO)(dppp)]₂, and [Rh(dppp)₂]Cl. (*See,* for instance, Jeong et al., "Pauson-Khand-type reaction mediated by Rh(I) catalysts," Pure Appl. Chem., 74(1):85-91, 2002; and Brummond , Kay M., "Rh(I)-catalyzed intramolecular [2 + 2 + 1] cycloaddition of allenenes: Construction of bicyclo[4.3.0]nonenones with an angular methyl group and tricyclo[6.4.0.01,5]dodecenone," Beilstein J. Org. Chem., 7:404-409, 2011, both of which are incorporated by reference in their entirety, especially all Rh(I) complexes disclosed therein, for all purposes). Other catalysts may also be used in this reaction including metals such as, but not limited to, Rhodium, Molybdenum, Zirconium, Iron, Cobalt and Iridium. These metal catalysts may be used with or without ligands. Some commonly employed metal ligands known in the art that may be used in the present reaction include, for instance, 1,3-bis(diphenylphosphino)propane (dppp), bidentate phosphine ligands and others, such as triphenylphosphine, *etc.* For instance, specific metal catalysts may include, but are not limited to, Mo(CO)₆, Fe(CO)₄(NMe₃), [Cp₂ZrCl]₂, IrCl(CO)(PPh₃)₂, [Ir(COD))Cl]₂, [Co₂(CO)₈], [Co₂(CO)₈(P(OPh)₃)], as well as the Rhodium catalysts already mentioned, above, and the like. (*See,* for instance, Alcaide et al., "The Allenic Pauson-Khand Reaction in Synthesis," Euro. J. Org. Chem., 2004(16):3377-3383, 2004, and the various catalysts disclosed therein). These catalysts are commercially available or easily prepared from commercially available sources.

The vessel is warmed to 140 °C, preferably by transfer to a bath, such as a preheated oil bath, under 1 atm CO for several hours or until the reaction is substantially complete. Other appropriate solvents may include, but are not limited to, those with high boiling points, such as aromatic and non-aromatic solvents, and dibutyl ether, toluene, mesitylene, naphthalene, and dichlorobenzene. The boiling point of the solvent is preferably at least 140 °C. The reaction should be heated to this temperature to maintain adequate yield under some conditions. The reaction is heated to 140 °C under CO, which can be at approximately 1 atm, for instance, until completion. The reaction can be cooled and additional rhodium (I) catalyst is added if needed. The reaction is heated a second time to 140 °C under a CO atmosphere if additional catalyst is needed. Upon completion, the reaction mixture is loaded directly onto a column and purified quickly by column chromatography to provide **34** as a white foam. It is recommended, though not necessary, that this intermediate be used in the next step, step 10, described below, and succeeding steps without substantial purification, until **20** is achieved, to avoid instability of intervening compounds.

### Step 10: Methyl Addition of 34 to Yield 35

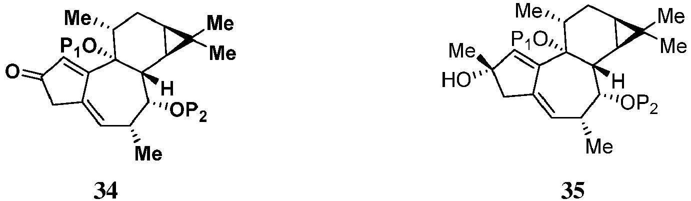

Solvent is used to suspend **34** under inert gas and cooled to -78 °C. The solvent may be, for instance, THF. However, other solvents may work equally as well in this step, such as, but not limited to, other etherial solvents. To this solution is added methyl magnesium bromide (MeMgBr). Other organometallic reagents, or Grignard reagents, may also be used with similar results. For instance, methyl lithium and CeCl₃ may also be used to achieve the same product **11.** However, some may prefer the use of MeMgBr because it is more convenient and CeCl₃ may require other protocols to maintain the reagent in a dry state. If not properly dried, CeCl₃ may decompose and negatively impact the reaction. After completion, the mixture is warmed to 0 °C for a short time, about 15 minutes; however, this incubation time will vary depending on the scale of reactants used in the step. The reaction is then cooled to -78 °C and carefully quenched by the addition of water. The mixture is then slowly warmed to room temperature.

Extraction solvents, such as EtOAc and water are added, the layers separated, and the aqueous layer extracted with additional solvent, such as EtOAc. The combined organic fractions are dried and evaporated to yield **35** as a colorless oil, which may be used in the next step without further purification. It is generally not recommended that this intermediate be further purified due to its inherent instability.

### Step 11: Dihydroxylation of 35 to Yield 36

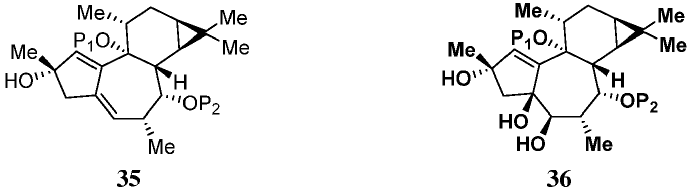

The solvent pyridine may be used to dissolve **36** under inert gas, such as argon, to which freshly prepared OsO₄, which also may be dissolved in pyridine, is added. (*See,* Lemieux-Johnson oxidation, as disclosed in, for instance, Pappo et al., "Osmium Tetroxide-Catalyzed Periodate Oxidation of Olefenic Bonds," J. Org. Chem., 21(4):478-479, 1956). Other co-solvents, such as DCM, THF, EtOH, EtOAc, Et₂O, and THF, with pyridine, may be employed and similar results achieved. This mixture is then stirred at room temperature for a period of time of about 12 hours to 24 hours or more. Alternatively, the reaction may be allowed to incubate for less than 16 hours so long as product formation is monitored. The reaction is then quenched by the addition of, for instance, saturated aqueous Na₂SO₃ and EtOAc, though other aqueous and organic neutralizing quench solvent systems may be used. The layers are then separated and the aqueous layer further extracted with the organic solvent, such as EtOAc. The combined organic fractions are evaporated and THF is added. Saturated aqueous reducing agent, such as Na₂SO₃, is then added and the resultant biphasic mixture vigorously stirred for approximately 24 hours to allow for sufficient mixing and extraction as well as layer resolution. The layers are separated and the aqueous layer further extracted several times with EtOAc. In this step, as in previous steps where EtOAc or THF are employed, other organic solvents may be substituted with equivalent yields and resolution, as known to one of skill. The combined organic fractions are dried and evaporated to yield diol **36** as a yellow solid, which may be used in the next step without further purification.

The formation of diol **36** may also be accomplished with catalytic amounts of OsO₄, such as 1-10, such as 2-8, such as 3-5, such as 5 molar percentage relative to alkene, and a co-oxidant such as for example TMANO, NMO or TBHP. The reaction may preferably be performed in the presence of a buffer, such as an aqueous buffer composition comprising acids such as citric acid, phophoric acid, and acetic acid and salts thereof, and mixtures thereof. Effective buffers can range from pH 1 to pH 6, such as pH 2-5.5, such as pH 3-5.

Under the standard conditions the compound **35** is dissolved in a mixture of acetone, acetonitrile, and an aqueous buffer of approximately pH 3, to which a catalytic quantity of OsO₄ relative to compound **35** and a stoichiometric quantity of DABCO and excess TMANO co-oxidant relative to compound **35** are added. The reaction is stirred at 50 °C for 20 to 50 hours or more. Other solvents such as THF, EtOH, *t*-BuOH, and H₂O may be employed in the reaction with similar results. The reaction can also be conducted at higher or lower temperatures ranging from 20 °C to 80 °C, with increased reaction times needed for lower temperatures. The aqueous buffer composition can include, but is not limited to acids such as citric acid, phosphoric acid, and acetic acid and salts thereof, and mixtures thereof. Effective buffers can range from pH 1 to pH 6. Alternative tertiary amine reagents to DABCO, such as triethylamine, diisopropylethylamine, or quinuclidine, may also be employed. The reaction can also be conducted without the addition of a tertiary amine with similar results. Other co-oxidants such as NMO and TBHP may also be employed with similar results. The reaction is quenched by the addition of a saturated aqueous reducing agent, such as Na₂SO₃. The layers are separated and the aqueous layer further extracted several times with EtOAc. In this step, as in previous steps where EtOAc or acetone are employed, other organic solvents may be substituted with equivalent yields and resolution, as known to one of skill. The combined organic fractions are dried and evaporated to yield diol **36** as a yellow solid, which is used in the next step without further purification.

It is noted that the compound of formula **36** is acid labile and should be stored under neutral conditions to maintain stability. Preferably **36** may be stored but is unstable to purification by silica and other similar means. Further purification is not recommended and may not be necessary since the crude product may be used directly in the next step.

### Step 12: Protection of 36 to Form 37

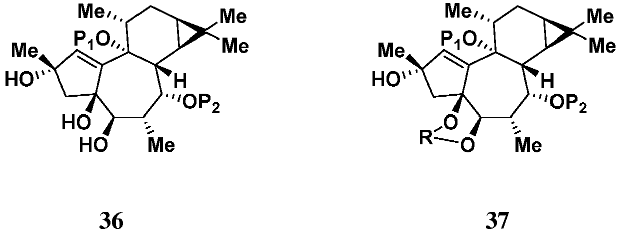

The solvent DCM may be used to dissolve **36** under inert gas. The reagents N,N-carbonyldiimidazole (CDI) and optionally DMAP are added and the solution stirred at room temperature until completion. DMAP can be used to cause the reaction to proceed faster, if desired, but it is not necessary. Although CDI is indicated for this step, other similar reagents such as phosgene (COCl₂) or triphosgene (bis(trichloromethyl) carbonate, C₃Cl₆O₃) may be equally employed to yield similar results. Reaction progress may be monitored by standard known procedures.

It is further noted that while a specific protecting group **(R)** is indicated in the above description of this step, other diol protecting groups are known in the art and are interchangeably useful in the present step for the same purpose, *i.e.* to protect the indicated diol of **36** during foregoing steps described below.

The reaction is then quenched by the addition of saturated aqueous CuSO₄, or other suitable neutralizing agent, and the layers separated with the aqueous layer being extracted several times with solvent, such as DCM. The combined organic fractions are dried and evaporated to produce **37** as a white solid, which may be either purified at this step as an end product, or used directly in the next step.

It is noted that the compound of formula **37** is acid labile and should be stored under neutral conditions to maintain stability.

Optionally, **13** may be formed from reaction of **36** with OsO₄, NMO, citric acid in t-BuOH and water to form **24,** as depicted in *Scheme 4* (Figure 4). Protection of the two hydroxyls on the 8-membered ring to form **25** may be achieved by employing any standard and appropriate protecting group, such as, but not limited to, TBS, TMS, TMSOTf, Ac, etc. Reaction with Grignard reagent MeMgBr, as above in methyl addition, Step 10, then affords the intermediate **26.** This intermediate may then be used in the remainder of the steps outlined in *Scheme 1* or *Scheme 2*, beginning at the pinacol rearrangement Step 13, below, where **37** is incubated with boron trifluoride diethyl ether complex, as explained in further detail in the next step. Substitution of **37** with **26** will then yield the corresponding protected intermediate **38,** depending on which protecting group was employed in this alternate pathway.

### Step 13: Pinacol rearrangement of 37 to Provide 38

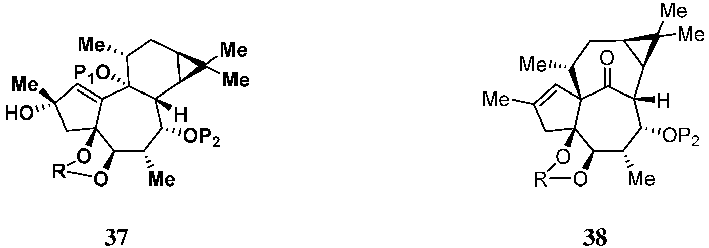

The compound of formula **37** is dissolved in DCM, or similar solvent, under inert gas and cooled to between approximately -50 °C and -78 °C or lower. The reaction can be temperature sensitive and deviation from the cool temperature indicated may substantially decrease yield under some conditions. A Lewis acid, such as, but not limited to, boron trifluoride, diethyl ether complex (BF₃•Et₂O) may be added dropwise with stirring to the dissolved **37.** *(See,* Lockner et al., "Practical Radical Cyclizations with Arylboronic Acids and Trifluoroborates," Org. Lett., 13(20):5628-5631, 2011). The reaction may then be stirred for a few minutes at this temperature, then warmed to -50 °C. After several minutes at the warmer temperature, for instance for about 30 min or more, depending on the amount of starting materials, a mixture of Et₃N/MeOH (3 mL) is added at -40 °C and the solution stirred for a few minutes. Saturated aqueous NaHCO₃ is then added. The reaction mixture is then warmed to r.t. and extracted several times with DCM. The combined organic fractions are dried and evaporated. The crude product may optionally be chromatographed using a solvent system comprised most preferably of 5% EtOAc:Hex to provide **38** as a clear oil. It is especially important in this step that the work-up described here is followed. It was empirically determined that most other work-up procedures employed yielded one or more spurious side products produced by elimination to the diene (likely the thermodynamic product).

It is noted that product **38** is relatively unstable as compared to other intermediates herein disclosed. Compound **38** is especially unstable in the context of acidic environments and especially when exposed to strong acid. This intermediate must be maintained in a neutral environment otherwise the reaction will reverse itself in acidic conditions. The intermediate **38** may optionally be purified flash column chromatography using a suitable solvent system such as, but not limited to, silica gel, column packed in DCM, then hexanes/EtOAc = 20:1 → 10:1 → 5:1, if desired. Purification of **14** will yield a white foam.

Furthermore, the steps required to make **38** from **34** require generally that the steps proceed one after the other in rapid succession without pause or break to avoid decomposition of the intervening intermediates. The intermediates along the presently disclosed synthetic pathway from **34** to **38** are relatively unstable and should not be stored for any long period of time. Thus, purification of these intermediates between steps is possible, but generally discouraged in order to maintain acceptable yield.

### Step 14: Allylic Oxidation of 38 to Yield 39

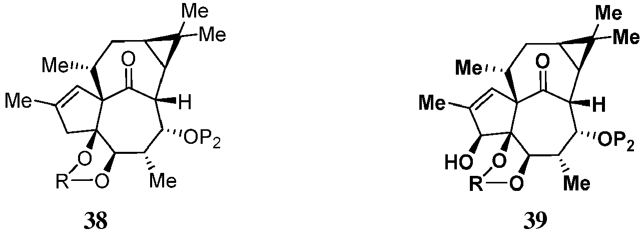

A desired amount of compound **38** is dissolved in dioxane (1,4-dioxacyclohexane, 1,4-dioxane, [6]-crown-2) or similar aprotic solvent, under inert gas, such as argon gas. To this solution is added SeO₂ and the mixture heated to 80 °C for several hours, for instance, as long as 10 to 14 hours. The duration of heating may vary depending on the quantity of starting material employed. However, it is essential that the mixture be heated to at least approximately 80 °C.

The resultant mixture is treated with solid NaHCO₃, dried and filtered. The filtering media may be any standard media, such as, but not limited to, celite. If celite is used, it is then washed with solvent, such as EtOAc and the combined organic fractions are concentrated to yield **39** as a colorless oil. Alternative media are known in the art for filtration purposes. Whenever filtration by mechanical means is mentioned, it is understood that substitutions may be made under appropriate conditions with other filtering media such as, but not limited to, celite, cotton, glass wool, alumina, Kieselguhr, silica, and the like. (*See,* for instance, "Handbook of Filter Media," D.B. Purchase and K. Sutherland, Eds., Elsevier Science & Technology Books, 2002; and "Filters and Filtration Handbook," K. Sutherland, 5th Ed., Elsevier, Butterworth-Heinemann, 2008, Burlington, MA, both of which are incorporated herein by reference in its entirety for all purposes).

### Step 15: Acylation of 39 to Produce 40

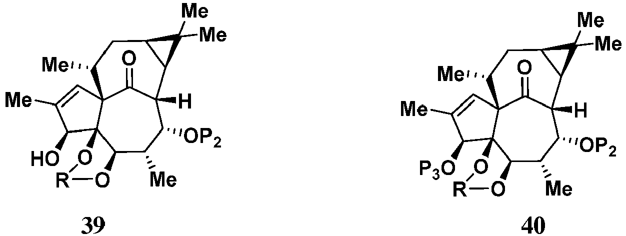

The compound of formula **39** may be used as a crude product without further purification in this next step. Briefly, **39** is dissolved in a suitable organic solvent, such as DCM, though other like solvents may be employed, under inert gas. The reagents Ac₂O and a catalytic (or stoichiometric) quantity of DMAP are then added and the mixture stirred until the reaction is complete, though other similar bases may be employed, such as, but not limited to, pyridine, lutidine, and/or triethylamine. Additionally, other acetylating reagents such as, but not limited to, acetyl chloride may be employed to yield protecting group **P₃.** It is noted that typically use of more Ac₂O than normally used in such a reaction may improve yields. Thus, as much as 2 equivalents, or 2.5 equivalents, 3 equivalents, or even 3.5 or 4 equivalents of Ac₂O may be employed to increase yields in this step.

Work up of the reaction involves quenching by addition of a suitable neutralizing reagent, such as saturated aqueous CuSO₄, followed by separation of the resultant layers, and washing of the aqueous layer with a suitable organic solvent, such as DCM, several times. The combined organic fractions are then dried and evaporated to yield **40** as a colorless oil.

It is noted that the conversion of **38** to **40,** encompassing steps 14 and 15, may be executed in a single pot reaction by successive addition of the reagents indicated separately for these steps in a telescoping manner, as described in the case of other combinable steps, above. The compound of formula **38** is dissolved in dioxane under inert gas, as above, and SeO₂ is added. The mixture is carefully sealed and heated to about 80 °C for several hours, for instance for as long as between 10 and 14 hours, or more. To this reaction is then cooled to r.t. and Ac₂O and DMAP are added. The reaction is stirred while incubating until completion, typically several minutes to an hour or more. Work up follows the same procedure described above, with filtration through media such as celite, or similar media, washing with a neutralizing reagent such as CuSO₄. The combined organic layers are then dried and concentrated. Product **40** may optionally be further purified, if so desired, by flash column chromatography, or other similar means, including but not limited to: silica gel, column packed in DCM, then hex/EtOAc = 20:1 → 10:1 → 5:1, for example. This affords **16** as an orange oil.

### Step 16: Deprotection of 40 to Provide 41

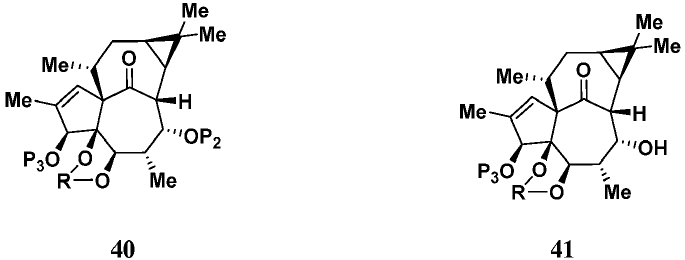

A reaction vessel, such as, for instance, a plastic vial, is charged with **40** (which may be a crude product) and acetonitrile (CH₃CN). A fluoride source, such as 47% aqueous HF is then added and the mixture heated to 50 °C, at ambient atmosphere. Though other fluoride reagents may be employed in this step, if HF is selected it is recommended that a plastic reaction vessel be used due to the hazardous nature of HF. Acidic sources of fluoride may be used in this step instead of basic fluoride sources due to the inherent instability of the present intermediates in aqueous basic conditions under some conditions.

Upon completion, typically several hours, or even as many as about 10 hours or more, the reaction is cooled to r.t. and quenched by the slow addition of neutralizing solution, such as saturated aqueous NaHCO₃ or other suitable quenching agent. Organic solvent EtOAc is added, though other known organic solvents may be employed in the work up. The layers may then be separated, and the aqueous layer washed several times with organic solvent, such as EtOAc. The combined organic fractions are then dried and evaporated to give **41** as a colorless oil.

### Step 17: Activation of 41 to Produce 42

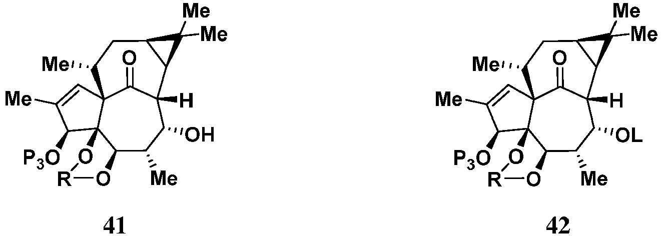

The compound **41** dissolved in pyridine, and/or other similar and compatible co-solvents, is placed under inert gas, such as argon gas, to which is dropwise added an agent for the introduction of a hydroxyl activating group "L", such as Tf₂O, preferably, though other appropriate hydroxyl activating groups may be utilized in this step. The mixture may optionally be heated to as high as 80 °C, but this is not necessary. Generally, higher heat enables the reaction to proceed faster, but the reaction will proceed at r.t. at a slower rate.

Upon completion, the reaction is cooled and quenched by the slow addition of quenching agent, such as saturated aqueous NaHCO₃. An organic solvent, such as, but not limited to, EtOAc is added to extract the product. The organic layer is washed several times with saturated aqueous CuSO₄, for example. The organic layer is then dried and evaporated to yield **42,** which should be used immediately in the next step without further purification due to its inherent instability, especially in the presence of base.

### Step 18: Elimination of Activated 42 to Yield 43

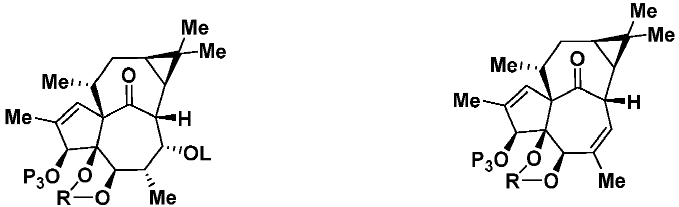

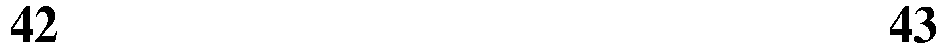

The crude compound **42** is dissolved in organic solvent, such as toluene, under inert gas. To this mixture is added diazabicycloundecene (DBU), though other similar non-nucleophilic bases may also be substituted for this reaction, and the mixture heated to 110 °C. The reaction may proceed somewhat quicker if heated to 110 °C, but this temperature is not necessary. The reaction will also proceed at ambient temperature, as well as temperatures between ambient temperature and 110 °C.

Upon completion, the reaction is optionally cooled and quenched by the addition of a suitable quenching agent such as saturated aqueous CuSO₄, though, as noted above, other quenching agents known in the art may be similarly utilized in such work-ups. The layers are separated and the aqueous layer extracted several times with a suitable organic solvent, such as DCM. The combined organic fractions are dried to produce **43** as a colorless oil, can be utilized immediately in the next step without further purification.

It is further noted that elimination of the alcohol and introduction of the second double bond into compound **43** may be achieved by other synthetic methodologies. For instance, one could also use a Martin Sulfurane (bis[α,α-bis(trifluoromethyl)benzenemethanolato]diphenylsulfur) reaction as an effective reagent for the elimination of the alcohol. This reagent will transform compound **41** directly into compound **43.** (*See,* Martin et al., J. Am. Chem. Soc., 93(17):4327-4329, 1971). The Martin Sulfurane is known in the art as a common reagent for alcohol elimination that functions in the same way (activation, followed by elimination) except that both activation and elimination proceed without any intermediate isolation. Additionally, one may use Mitsunobu conditions to achieve the same result. (*See,* Organic Reactions, Vol. 42, pages 335-656, Eds. Leo A Paquette et al., 1992, John Wiley & Sons, Inc.). Mistunobu conditions commonly employ an azodicabroxylate, such as diethyl azodicarboxylate (DEAD) or diisopropyl azodicarboxylate (DIAD), and triphenylphosphine, which also accomplish activation and elimination of the alcohol group in one step.

### Step 19: Deprotection of 43 to Provide 20

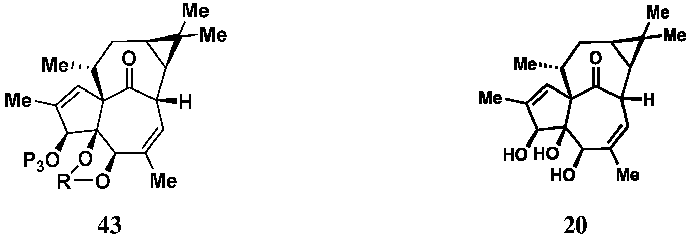

To a solution comprising the compound **43** dissolved in organic solvent, such as MeOH, under ambient atmosphere, is added an aqueous base, such as, but not limited to, K₂CO₃. Alternatively, other aqueous bases such as KOH, NaOH, or DBU may be used.

Saturated aqueous base, such as NaHCO₃ and organic solvent, such as DCM are added upon completion of the reaction which usually only requires a few minutes, though the reaction may be monitored by known methods if needed. The layers are separated and the aqueous layer extracted several times with organic solvent, such as DCM. The combined organic fractions are dried and the crude mixture optionally purified by chromatography, such as preparative TLC, to give 20-deoxyingenol, **20,** as a white solid.

Conversion of compound **41** to **20,** encompassing steps 17 through 20, may be executed in a single pot reaction by successive addition of the reagents indicated separately for these steps in a telescoping manner, as described in the case of other combinable steps, above.

### Step 20: Allylic Oxidation of 20 to Obtain Ingenol (21)

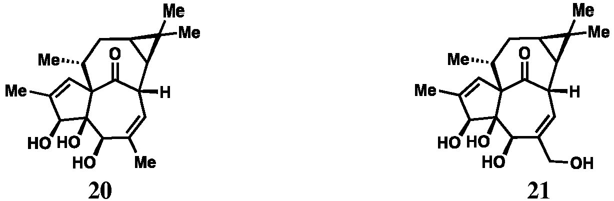

Though this conversion has been reported in the literature, a significantly better yield may be obtained by modifying this procedure as follows. (*See,* Nickel et al., J. Am. Chem. Soc., 126:16300-16301, 2004, reaction "1" in Scheme 5). To a solution of **20** dissolved in a solvent mixture, such as dioxane and formic acid, is added SeO₂. The suspension may be heated to 80 °C for several hours, or until the reaction is complete as judged by monitoring appearance of product. The reaction may then be cooled and quenched by the addition of a suitable neutralizing solution, such as, but not limited to, saturated aqueous NaHCO₃ and Et₂O. The aqueous layers are removed and sodium hydroxide is added. The biphasic mixture is vigorously shaken and the organic layer removed. The aqueous layer is extracted with a suitable solvent, such as Et₂O, and the combined organic layers dried and concentrated. The crude product may optionally be further purified, for instance by column chromatography in a suitable solvent system such as, for example, 1:1 DCM:EtOAc, to yield ingenol **(21)** as a white film.

Finally, ingenol **(21)** may be further converted to ingenol-3-angelate **(29)** by known means. (*See,* for instance, WO2012/010172). Various hydroxyl protecting groups and corresponding reagents may be employed as described above.

Briefly, this process of converting ingenol **(21)** to ingenol-3-angelate **(29)** may comprise the steps of:
(a) reacting one or both hydroxyl groups in positions 5 and 20 of ingenol with suitable hydroxyl protecting agents, same or different, *i.e.* protecting one or both hydroxyl groups in positions 5 and 20 of ingenol with a protective group,
(b) esterifying the compounds corresponding to **21** wherein carbons 5 and 20 are protected, *i.e.* esterifying the hydroxyl group at the 3-position, to obtain either of the compounds depicted below: 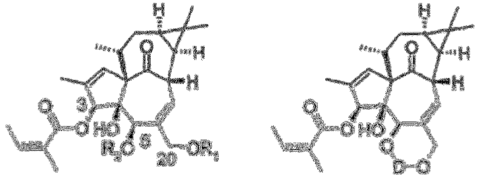 wherein R₁ represents a hydrogen or a hydroxyl protective group and R₂ represents a hydrogen or a hydroxyl protective group, or R₁ represents a hydroxyl protective group and R₂ represents hydrogen or a hydroxyl protective group, or wherein D represents a dihydroxyl protective group, and
(c) removing the hydroxyl protective groups R₁, or R₁ and R₂, or D from the above compounds to obtain ingenol-3-angelate **(29).**

Alternatively, ingenol **(21)** may be esterified to obtain: 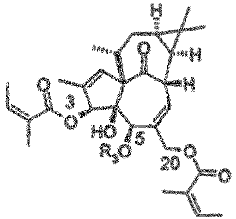 wherein R₃ represents hydrogen or angeloyl, *i.e.* esterifying the 3- and the 20-hydroxyl group and optionally esterifying the 5-hydroxyl group of ingenol **(21)** to obtain the above-depicted compound. Esterification is followed by cleaving the angelate ester(s) in position 20 or in position 5 and 20 to obtain ingenol-3-angelate.

Alternatively, the 3-hydroxy group of ingenol may be esterified to obtain ingenol-3-angelate.

Further alternative synthetic procedures are contemplated. For instance, conversion of **34** to **21** may proceed instead by the route depicted in *Scheme 4* (Figure 4), as briefly described, above. This alternative protocol may provide various efficiencies in reaction scale-up or other factors such as cost, time and/or toxicity of reagents, etc.

### EXAMPLES

### General

It is understood that the examples and embodiments described herein are for illustrative purposes and that various modifications or changes in light thereof will be suggested to persons skilled in the art and are to be included within the spirit and purview of this application and scope of the claims. Accordingly, the following examples are offered to illustrate, but not to limit, the claimed invention.

Disclosed hereinbelow are two general synthetic approaches to achieve efficient total synthesis of ingenol. The first experimental procedure includes several optional intermediate steps, while the second experimental procedure is a shortened and further optimized procedure which takes advantage of execution of various groups of steps in a single pot to minimize possible loss of unstable intermediates due to degradation.

### PROCEDURE 1: Longer Protocol - Scheme 1

### Example 1: Chlorination of 1

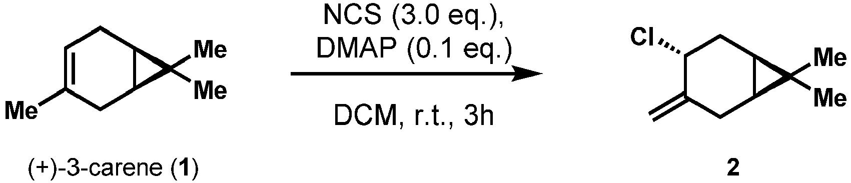

To a solution of (+)-3-carene **(1)** (22.5 g, 165.2 mmol, 1.0 equiv) in CH₂Cl₂ (600 mL) was added *N*-chlorosuccinimide (66.2 g, 495.5 mmol, 3.0 equiv) and DMAP (2.02 g, 16.5 mmol, 0.1 equiv) and the solution was stirred at room temperature for 3 h. Pentane (600 mL) was added and the resulting suspension was stirred for 5 min before being filtered through a pad of SiO₂. The solution was concentrated under reduced pressure to give chloro-carene **2,** which was used in the next step without further purification. A small sample of crude **2** could be further purified by column chromatography (pentane) to give analytically pure **2.**
**2: ¹H NMR (500 MHz, CDCl₃):** δ 4.93 - 4.88 (m, 1 H), 4.81 - 4.76 (m, 1 H), 4.54 (t, *J* = 3.0 Hz, 1 H), 2.87 (ddt, *J* = 16.5, 8.1, 2.8 Hz, 1 H), 2.48 (ddd, *J* = 15.7, 9.3, 2.7 Hz, 1 H), 2.30 (d, *J =* 16.6 Hz, 1 H), 1.76 (dt, *J* = 15.7, 3.7 Hz, 1 H), 1.02 (s, 3H), 0.90 - 0.86 (m, 1 H), 0.85 (s, 3 H), 0.80 (td, *J* = 9.2, 3.9 Hz, 1 H).

### Example 2: Ozonolysis of 2

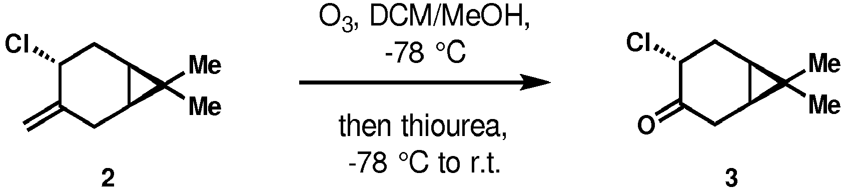

To a solution of the crude chloro-carene **2** in CH₂Cl₂ (400 mL) was added MeOH (125 mL) under argon, and the solution was cooled to -78 °C. The solution was bubbled with O₃ at -78 °C until the solution turned blue. Excess O₃ was expelled by bubbling O₂ through the solution until it became colorless again. Thiourea (21 g, 276 mmol) was added and the reaction mixture was warmed to room temperature and stirring was continued for 2 h. The reaction mixture was washed with water (2 × 400 mL) and brine (400 mL) and the combined organic layers were dried over Na₂SO₄, filtered and carefully concentrated *in vacuo.* Purification of the residue by flash column chromatography (silica gel, column packed in CH₂Cl₂, then pentane/Et₂O = 20:1 → 10:1) yielded the chloro-ketone **3** (13.8 g, 48% over 2 steps) as a colorless liquid.
**3: ¹H NMR (500 MHz, CDCl₃)**: δ 3.93 (t, *J* = 3.0 Hz, 1 H), 3.02 (dd, *J* = 18.6, 9.0 Hz, 1 H), 2.62 (dddd, *J* = 16.5, 9.2, 2.9, 1.1 Hz, 1 H), 2.24 (dt, *J* = 18.6, 1.4 Hz, 1 H), 2.03 (ddd, *J* = 16.4, 5.0, 3.1 Hz, 1 H), 1.26 (td, *J* = 9.1, 1.9 Hz, 1 H), 1.07 (s, 3 H), 0.95 (td, *J* = 9.2, 5.0 Hz, 1 H), 0.85 (s, 3 H).

### Example 3: Reductive Alkylation of 3

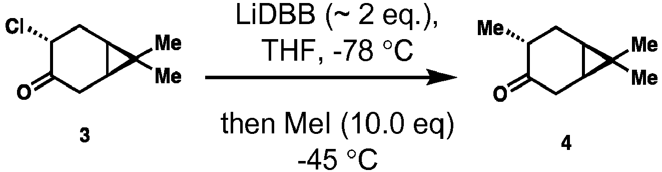

A three neck flask was charged with dry THF (90 mL), di-tert-butyl-biphenyl (DBB) (9.2 g, 34.7 mmol, 6.0 equiv) and freshly cut lithium metal (200 mg, 28.9 mmol, 5.0 equiv). The suspension was stirred at room temperature for 3 h to give a dark-green solution. In a separate flask, chloro-ketone **3** (1.0 g, 5.79 mmol, 1.0 equiv) was dissolved in THF (29 mL) and cooled to -78 °C. The LiDBB solution was added to the solution of **3** via cannula over 30 min until the green color persisted. Methyl iodide (3.6 mL, 57.9 mmol, 10 equiv) was slowly added and the stirring was continued for 5 h at -45 °C. Saturated aqueous NH₄Cl (100 mL) was added and the reaction mixture was warmed to room temperature. The organic layer was separated and the aqueous layer was extracted with Et₂O (3 × 100 mL). The combined organic layers were dried over Na₂SO₄, filtered and carefully concentrated under reduced pressure. The crude product was purified by flash column chromatography (silica gel, pentane, then pentane/Et₂O = 80:1 → 40:1 → 20:1 → 10:1) to afford methyl ketone **4** (353 mg, 40%) as a light yellow oil.
**4: ¹H NMR (600 MHz, CDCl₃)**: δ 2.58 (dd, *J* = 18.3, 8.6 Hz, 1 H), 2.23 (qdd, *J* = 7.3, 5.0, 2.7 Hz, 1 H), 2.09 (dd, *J* = 18.3, 3.4 Hz, 1 H), 2.02 (dddd, *J* = 15.0, 9.0, 2.6, 1.0 Hz, 1 H), 1.72 (ddd, *J* = 15.1, 6.1, 4.8 Hz, 1 H), 1.23 (d, *J* = 7.2 Hz, 3 H), 1.07 (s, 3 H), 0.93 (s, 3 H), 0.88 - 0.78 (m, 2 H).

### Example 4: Aldol Reaction of 4

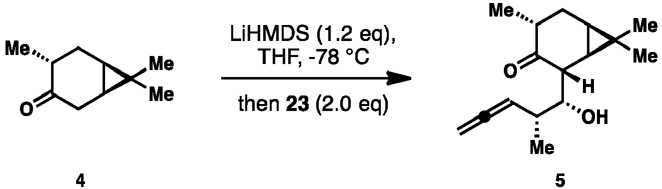

To a solution of **4** (62 mg, 0.41 mmol, 1.0 equiv) in 0.8 mL of THF at -78 °C was added LiHMDS (1 M solution in THF, 101 µL, 0.49 mmol, 1.2 equiv). The mixture was stirred at -78 °C for 1 hour before a solution of **23** (78 mg, 0.811 mmol, 2.0 equiv) in 7 mL of THF was added over 15 min. The mixture was stirred at -78 °C for 3 h then quenched by the addition of saturated aqueous NH₄Cl solution (15 mL) and EtOAc (20 mL). The reaction mixture was separated and the aqueous layer was extracted with EtOAc (3 × 20 mL). The combined organic fractions were dried with sodium sulfate, filtered, evaporated, and purified by column chromatography (hexanes/EtOAc = 20:1 → 15:1) to provide **5** (76 mg, 75%) as a colorless oil.
**5: ¹H NMR (400 MHz, CDCl₃):** δ 5.28 - 5.21 (m, 1 H), 4.73 - 4.62 (m, 2 H), 4.08 (dd, *J* = 2.7, 1.4 Hz, 1 H), 3.76 (dt, *J* = 8.9, 2.4 Hz, 1 H), 2.56 - 2.45 (m, 1 H), 2.26 (qdd, *J* = 7.3, 5.8, 2.8 Hz, 1 H), 2.07 (dd, *J* = 8.9, 7.4 Hz, 1 H), 1.84 (ddd, *J* = 14.9, 7.8, 2.9 Hz, 1 H), 1.69 (ddd, *J* = 14.7, 8.5, 5.9 Hz, 1 H), 1.19 - 1.16 (m, 6 H), 1.15 (s, 3 H), 1.09 (s, 3 H), 0.86 (q, *J* = 8.5 Hz, 1 H), 0.40 (dd, *J* = 9.1, 7.4 Hz, 1 H).

### Example 5: Acetylide Addition to 5

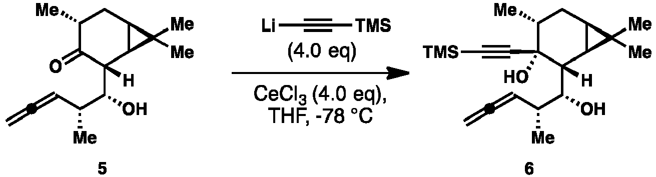

To a solution of trimethylsilylacetylene (1.06 g, 10.8 mmol, 4.0 equiv) in 20 mL of THF under argon at -78 ° C was added 2.1 M *n*-BuLi solution in hexanes (5.1 mL, 10.8 mmol, 4.0 equiv). The solution was stirred for 1 hour at -78 °C then added to a suspension of CeCl₃ (2.66 g, 10.8 mmol, 4.0 equiv) in 50 mL of THF under argon at -78 °C. The resulting suspension was vigorously stirred at -78 °C for 1 hour then a solution of **5** (670 mg, 2.7 mmol, 1.0 equiv) in 10 mL of THF was added dropwise. The suspension was stirred at -78 °C for 3 h then quenched by the addition of water (200 mL) and EtOAc (200 mL). The layers were separated and the aqueous layer was extracted with EtOAc (3 × 150 mL). The combined organic layers were dried over sodium sulfate, filtered and concentrated to give **6** (680 mg) as a 6:1 mixture of diastereomers.
**6: ¹H NMR (600 MHz, CDCl₃):** δ 5.20 (dt, *J* = 8.2, 6.7 Hz, 1H), 4.75 - 4.70 (m, 2H), 3.79 (dt, *J* = 7.2, 4.6 Hz, 1H), 3.44 (d, *J* = 4.8 Hz, 1H), 2.91 (ddq, *J* = 10.6, 6.6, 2.0 Hz, 1H), 2.79 (s, 1H), 1.68 (ddd, *J* = 14.8, 7.0, 1.0 Hz, 1H), 1.61 - 1.49 (m, 2H), 1.18 (d, *J* = 7.0 Hz, 3H), 1.06 (d, *J* = 6.5 Hz, 3H), 1.04 (s, 3H), 0.96 (s, 3H), 0.79 - 0.64 (m, 1H), 0.31 (dd, *J=* 9.5, 4.9 Hz, 1H), 0.17 (s, 9H).

### Example 6: Desilylation of 6

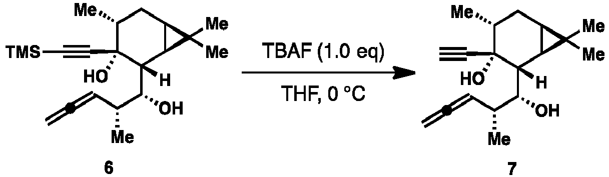

To a solution of **6** (450 mg, 1.30 mmol, 1.0 equiv) in 13 mL of THF under argon at 0 °C was added 1.0 M TBAF (1.3 mL, 1.30 mmol, 1.0 equiv) in THF. After 10 min EtOAc (20 mL) and H₂O (20 mL) were added and the organic layer was washed with H₂O (5 × 20 mL). The organic layer was dried with sodium sulfate, filtered, and concentrated to give 7 (326 mg, 90%) as a colorless oil.
**7: ¹H NMR (400 MHz, CDCl₃):** δ 5.19 (dt, *J* = 8.0, 6.7 Hz, 1 H), 4.73 (d, *J=* 5.9 Hz, 2 H), 3.79 (dt, *J* = 7.0, 4.9 Hz, 1 H), 3.14 (d, *J* = 5.4 Hz, 1 H), 2.94 (s, 1 H), 2.92 - 2.84 (m, 1 H), 2.66 (s, 1 H), 1.73 - 1.64 (m, 1 H), 1.60 - 1.51 (m, 2 H), 1.31 (dq, *J* = 12.2, 6.6 Hz, 1 H), 1.17 (d, *J* = 6.9 Hz, 3 H), 1.08 (d, *J* = 6.6 Hz, 3 H), 1.04 (s, 3 H), 0.95 (s, 3 H), 0.72 (t, *J* = 8.7 Hz, 1 H), 0.30 (dd, *J* = 9.5, 4.8 Hz, 1 H).

### Example 7: TBS Protection of 7

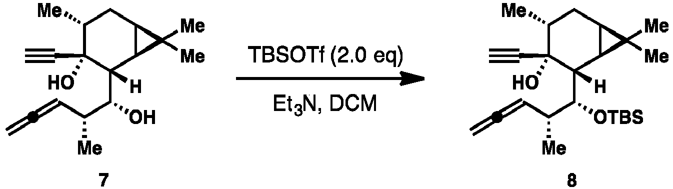

To a solution of 7 (358 mg, 1.3 mmol, 1.0 equiv) and Et₃N (2.7 mL, 19.5 mmol, 15 equiv) in 8 mL of DCM at 0 °C was added TBSOTf (687 mg, 2.6 mmol, 2.0 equiv). After 30 min the reaction was quenched by the addition of saturated aqueous NaHCO₃ (8 mL). DCM (15 mL) was added, the layers were separated, and the aqueous layer was extracted with DCM (2 × 15 mL). The combined organic fractions were dried with sodium sulfate, filtered, and concentrated to give **8** (384 mg, 76%) as a colorless oil.
**8: ¹H NMR (600 MHz, CDCl₃):** δ 5.35 (dt, *J* = 8.2, 6.7 Hz, 1H), 4.66 (ddd, *J* = 6.6, 1.9, 0.8 Hz, 2H), 4.12 (dd, *J* = 5.2, 3.4 Hz, 1H), 3.31 (dddt, *J* = 8.8, 7.0, 5.2, 1.9 Hz, 1H), 3.08 (s, 1H), 2.52 (s, 1H), 1.70 - 1.57 (m, 2H), 1.54 (s, 1H), 1.11 (d, *J* = 6.9 Hz, 3H), 1.05 (d, *J* = 6.6 Hz, 3H), 1.04 (s, 3H), 0.94 (s, 9H), 0.92 (s, 3H), 0.83 (dd, *J* = 9.3, 5.2 Hz, 1H), 0.67 (dd, *J* = 9.2, 7.7 Hz, 1H), 0.13 (s, 3H), 0.10 (s, 3H).

### Example 8:TMS Protection of 8

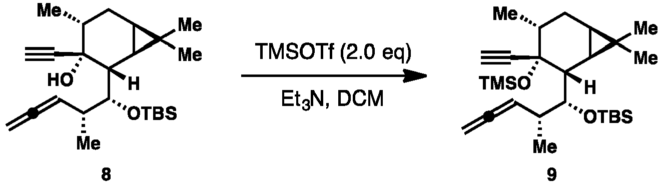

To a solution of **8** (120 mg, 0.25 mmol, 1.0 equiv) and Et₃N (500 µL) in 2 mL of DCM under argon at 0 °C was added TMSOTf (111.13 mg, 0.5 mmol, 2.0 equiv). After 30 min the reaction was quenched by the addition of saturated aqueous NaHCO₃ (3 mL). DCM (3 mL) was added, the layers were separated, and the aqueous layer was extracted with DCM (3 × 3 mL). The combined organic fractions were dried with sodium sulfate, filtered, concentrated, and the crude product was purified by column chromatography (hexanes) to give **9** (120 mg, 85%) as a colorless oil.
**9: ¹H NMR (400 MHz, CDCl₃):** δ 5.52 (dt, *J* = 7.9, 6.8 Hz, 1 H), 4.59 (dd, *J* = 6.8, 2.0 Hz, 2 H), 4.44 (d, *J* = 2.9 Hz, 1 H), 3.01 - 2.85 (m, 1 H), 2.58 (s, 1 H), 1.58 - 1.50 (m, 1 H), 1.50 - 1.39 (m, 1 H), 1.36 (dd, *J* = 5.9, 3.0 Hz, 1 H), 1.24 - 1.14 (m, 2 H), 1.13 (d, *J* = 7.1 Hz, 3 H), 1.01 (d, *J* = 6.8 Hz, 3 H), 1.00 (s, 3 H), 0.92 (s, 9 H), 0.87 (s, 3 H), 0.56 (ddd, *J* = 9.3, 7.8, 1.3 Hz, 1 H), 0.19 (s, 9 H), 0.08 (s, 3 H), 0.04 (s, 3 H).

### Example 9: Pauson-Khand Reaction of 9

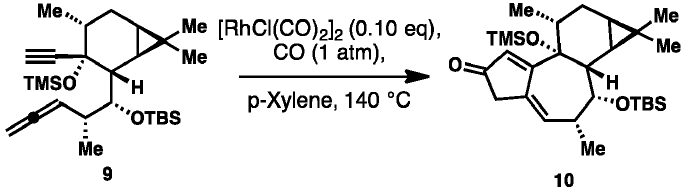

A 1L three-neck flask was charged with a solution of **9** (1.5 g, 3.25 mmol, 1.0 equiv) in anhydrous *p*-xylene (650 mL) and the solution was degassed using carbon monoxide under sonication. [RhCl(CO)₂]₂ (126.3 mg, 0.325 mmol, 0.1 equiv) was added and the reaction mixture was transferred into a preheated oil bath and stirred at 140 °C under 1 atm. of CO for 12 h. The reaction mixture was cooled to rt and concentrated under reduced pressure. Purification of the residue by flash column chromatography (silica gel, hexanes then hexanes/Et2O = 40:1 → 30:1 → 20:1 → 10:1) yielded **10** (1.15 g, 72%) as a light brown foam.
**10: ¹H NMR (400 MHz, CDCl₃):** δ 6.06 (s, 1 H), 5.85 (d, *J* = 8.4 Hz, 1 H), 4.01 (dd, *J* = 7.8, 3.1 Hz, 1 H), 3.19 (d, *J* = 19.3 Hz, 1 H), 2.96 (d, *J* = 19.3 Hz, 1 H), 2.58 - 2.48 (m, 1 H), 1.76 - 1.66 (m, 1 H), 1.63 - 1.51 (m, 3 H), 1.38 (d, *J* = 7.4 Hz, 3 H), 1.05 (s, 3 H), 1.04 - 1.03 (m, 1 H), 1.02 (d, *J* = 6.2 Hz, 3 H), 0.90 (s, 9 H), 0.86 (s, 3 H), 0.66 (t, *J* = 8.4 Hz, 1 H), 0.07 (s, 9 H), -0.01 (s, 3 H), -0.02 (s, 3 H).

### Example 10: Methyl Addition to 10

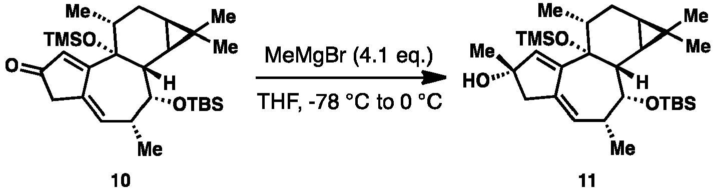

To a solution of **10** (1.32 g, 2.70 mmol, 1.0 equiv) in THF (52 mL) was added methyl magnesium bromide (3.0 M in Et₂O, 2.7 mL, 8.10 mmol, 3.0 equiv) over 5 min at -78 °C. The reaction mixture was stirred at this temperature for 15 min before being warmed to 0 °C. After 15 min, the reaction mixture was cooled back to -78 °C and another portion of MeMgBr (1.0 mL, 3 mmol, 1.11 equiv) was added. The reaction mixture was warmed to -30 °C and carefully quenched with water (100 mL) after 15 min. The mixture was extracted with EtOAc (3 × 100 mL) and the combined organic layers were dried over Na₂SO₄, filtered and concentrated under reduced pressure. Purification of the crude product by flash column chromatography (silica gel, column packed in DCM, then hex/EtOAc = 10:1 → 5:1) afforded **11** (1.08 g, 80%) as a white foam and recovered **10** (240 mg, 18%).
**11: ¹H NMR (400 MHz, CDCl₃):** δ 5.66 (s, 1 H), 5.52 (d, *J* = 8.3 Hz, 1 H), 4.00 (dd, *J* = 7.9, 3.0 Hz, 1 H), 2.68 (d, *J* = 12.8 Hz, 1 H), 2.55 (d, *J* = 12.9 Hz, 1 H), 2.47 - 2.36 (m, 1 H), 1.66 (dd, *J* = 14.2, 7.1 Hz, 1 H), 1.57 - 1.44 (m, 2 H), 1.38 (t, *J* = 7.8 Hz, 1 H), 1.31 - 1.27 (m, 6 H), 1.05 - 1.01 (m, 6 H), 1.00 - 0.95 (m, 1 H), 0.89 (s, 10 H), 0.84 (s, 3 H), 0.63 - 0.56 (m, 1 H), 0.11 (s, 9 H), -0.02 (s, 6 H).

### Example 11: Dihydroxylation of 11

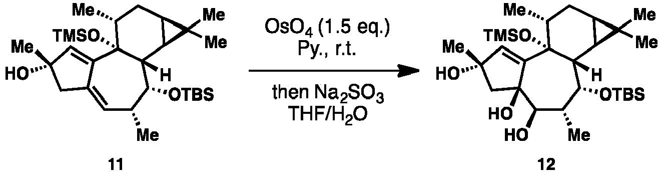

To a solution of **11** (100 mg, 0.198 mmol, 1.0 equiv) in pyridine (4 mL) was added OsO₄ (76 mg, 0.297 mmol, 1.5 equiv) as a freshly prepared solution in pyridine (760 µL of a 100mg/l mL solution). The reaction mixture was stirred for 12 h at room temperature before being quenched by the addition of sat. aq. Na₂SO₃ (20 mL). The reaction mixture was extracted with EtOAc (3 × 10 mL) and the combined organic layers were dried over Na₂SO₄, filtered and concentrated *in vacuo.* The crude osmate ester was dissolved in THF (20 mL) and sat. aq. Na₂SO₃ (20 mL) was added. The resulting reaction mixture was vigorously stirred for 24 h before water (20 mL) was added. The mixture was extracted with EtOAc (3 × 40 mL) and the combined organic layers were dried over Na₂SO₄, filtered and concentrated under reduced pressure to afford the crude diol **12** which was used immediately without further purification.
**12: ¹H NMR (400 MHz, CDCl₃):** δ 5.79 (s, 1H), 4.80 (d, *J* = 4.8 Hz, 1H), 4.29 (dd, *J* = 8.0, 5.3 Hz, 1H), 2.82 (pd, *J* = 7.8, 4.8 Hz, 1H), 2.55 (d, *J* = 14.5 Hz, 1H), 2.40 (d, *J* = 14.5 Hz, 1H), 1.68 (dd, *J* = 14.8, 6.8 Hz, 1H), 1.52 - 1.46 (m, 4H), 1.40 (t, *J* = 5.9 Hz, 1H), 1.20 - 1.11 (m, 6H), 1.05 (s, 3H), 0.97 - 0.93 (m, 12H), 0.64 (d, *J* = 6.7 Hz, 3H), 0.20 (s, 9H), 0.11 (s, 3H), 0.10 (s, 3H).

### Example 12: Carbonate Protection of 12

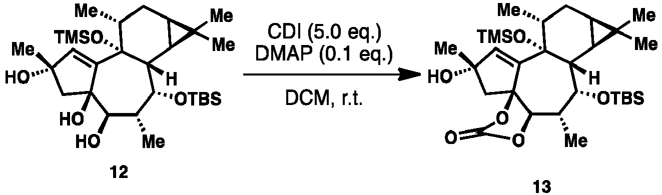

Crude **12** was dissolved in DCM (6 mL) and *N,N*-carbonyldiimidazole (172 mg, 1.06 mmol, 5.0 equiv) and DMAP (2.6 mg, 21.2 µmol, 0.1 equiv) were added. The solution was stirred at room temperature for 8 h before being quenched by the addition of saturated aqueous CuSO₄ (6 mL). The layers were separated, and the aqueous layer was extracted with DCM (3 × 10 mL). The combined organic layers were dried over sodium sulfate, filtered and concentrated in vacuo. Purification of the crude product by flash column chromatography (silica gel, column packed in DCM, then hex/EtOAc = 10:1 → 5:1) afforded **13** (72 mg, 64%) as a white solid.
**13: ¹H NMR (400 MHz, CDCl₃):** δ 5.79 (s, 1H), 4.80 (d, *J* = 4.8 Hz, 1H), 4.29 (dd, *J* = 8.0, 5.3 Hz, 1H), 2.82 (pd, *J* = 7.8, 4.8 Hz, 1H), 2.55 (d, *J* = 14.5 Hz, 1H), 2.40 (d, *J* = 14.5 Hz, 1H), 1.68 (dd, *J* = 14.8, 6.8 Hz, 1H), 1.52 - 1.46 (m, 4H), 1.40 (t, *J* = 5.9 Hz, 1H), 1.20 - 1.11 (m, 6H), 1.05 (s, 3H), 0.97 - 0.93 (m, 12H), 0.64 (d, *J* = 6.7 Hz, 3H), 0.20 (s, 9H), 0.11 (s, 3H), 0.10 (s, 3H).

### Example 33: Dihydroxylation of 11 - catalytic amounts of OsO₄

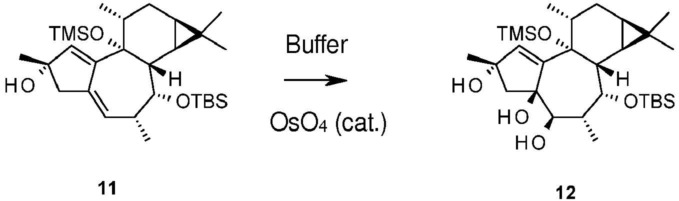

A 25 mL round flask was charged with compound **11** (174 mg, 0.345 mmol, 1.0 equiv), Me₃NO.2H₂O (383 mg, 3.45 mmol, 10 equiv) and DABCO (39 mg, 0.345 mmol, 1.0 equiv) in Acetone/CH₃CN/0.5 M buffer solution (7 mL, 0.05 M, 5:3:2). The buffer solution was prepared by adding citric acid (840 mg) and NaHPO4 (280 mg) to a solution of 10 mL distilled H2O and stirring at room temperature for 1 minute whereby the pH value of the solution was 3 via pH paper test. Then, OsO₄ solution (2.5 wt. % in *tert*-butanol, 0.17 mL, 0.017 mmol, 0.05 equiv) was added to the flask and stirred vigorously at 50° C for 22 h, before being quenched by the addition of saturated aqueous Na₂SO₃ (10 mL) (After 15 h, the reaction was monitored by TLC every 3 h. If starting material stopped to convert the reaction was quenched). The reaction mixture was extracted with ether (3 × 10 mL) and the combined organic layers were dried over Na₂SO₄, filtered and concentrated in vacuo to afford crude **12** (*R_{f}* = 0.33 (Hex/EtOAc = 7:3; anisaldehyde).

### Example 34: Carbonate protection of 12

Crude **11** was dissolved in hexane (7 mL, 0.05 M) and *N,N*-carbonyldiimidazole (280 mg, 1.73 mmol, 5.0 equiv) was added. The solution was stirred at room temperature for 20 hours. Purification of the crude product by loading crude solution to flash column chromatography (silica gel, column packed in Hexane, then hexanes/EtOAc = 20:1 → 5:1) afforded **13** (100 mg, 51%) as a white foam and **11** (19mg, 20% pure).

### Example 13: Pinacol Rearrangement of 13

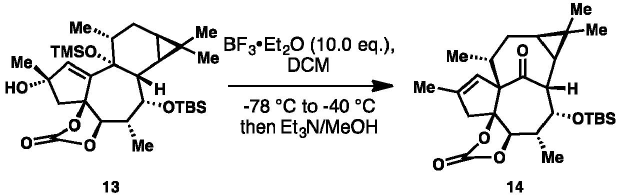

To a solution of **13** (191 mg, 0.338 mmol, 1.0 equiv) in DCM (7 mL) was added BF₃ Et₂O (420 µL, 3.38 mmol, 10 equiv) dropwise at -78 °C. The reaction mixture was stirred at this temperature for 2 min before being warmed to -50 °C. After 30 min, a 1:1 mixture of Et₃N/MeOH (3 mL) was added at -40 °C, the solution was stirred for 2 min and saturated aqueous NaHCO₃ (5 mL) was added. The reaction mixture was warmed to rt and extracted with DCM (3 × 25 mL). The combined organic layers were dried over Na₂SO₄, filtered and concentrated under reduced pressure. Purification of the crude product by flash column chromatography (silica gel, column packed in DCM, then hex/EtOAc = 20:1 → 10:1 → 5:1) afforded **14** (128 mg, 80%) as a white foam.
**14: ¹H NMR (400 MHz, CDCl₃):** δ 5.65 (s, 1H), 4.80 (d, *J* = 4.8 Hz, 1H), 4.19 (dd, *J* = 7.6, 4.1 Hz, 1H), 3.20 (d, *J* = 17.8 Hz, 1H), 2.81 (pd, *J* = 7.9, 4.7 Hz, 1H), 2.52 (d, *J* = 17.7 Hz, 1H), 2.44 - 2.32 (m, 2H), 1.88 - 1.80 (m, 2H), 1.72 (s, 3H), 1.09 (s, 3H), 1.03 (s, 3H), 1.01 - 0.95 (m, 1H), 0.95 (d, *J* = 7.1 Hz, 3H), 0.93 (s, 9H), 0.83 (d, *J* = 8.0 Hz, 3H), 0.68 (q, *J* = 8.1 Hz, 1H), 0.10 (s, 3H), 0.04 (s, 3H).

### Example 14: Allylic Oxidation of 14

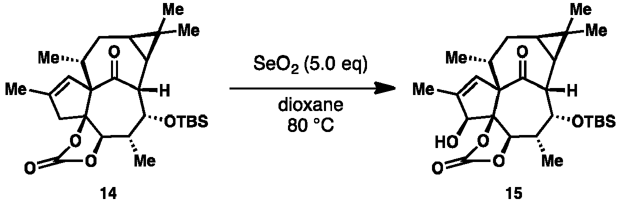

**14** (9.2 mg, 0.019 mmol, 1.0 equiv) was dissolved in 0.6 mL of dioxane. SeO₂ (10 mg, 0.095 mmol, 5.0 equiv) was added and the mixture was heated to 80 °C for 12 h under ambient atmosphere. After cooling, solid NaHCO₃ was added to the reaction mixture and the crude mixture was filtered through a plug of celite to obtain crude 15. The material was typically used without further purification.
**15: ¹H NMR (400 MHz, CDCl₃):** δ 5.90 (s, 1H), 4.80 (d, *J* = 4.6 Hz, 1H), 4.21 (dd, *J* = 7.4, 4.3 Hz, 1H), 4.18 (s, 1H), 2.83 (ddq, *J* = 11.9, 7.6, 3.7 Hz, 1H), 2.53 (d, *J* = 17.4 Hz, 1H), 2.40 (dq, *J* = 8.4, 3.8, 3.3 Hz, 1H), 1.99 - 1.85 (m, 2H), 1.84 (dd, *J* = 1.5, 0.8 Hz, 3H), 1.12 (s, 3H), 1.04 (s, 3H), 1.02 - 0.97 (m, 1H), 0.96 (d, *J* = 7.0 Hz, 3H), 0.94 (s, 9H), 0.83 (d, *J* = 7.9 Hz, 3H), 0.70 (td, *J* = 8.5, 6.2 Hz, 1H), 0.11 (s, 3H), 0.06 (s, 3H).

### Example 15: Acylation of 15

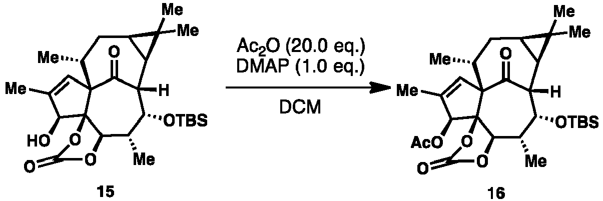

Crude **15** was dissolved in DCM (500 µL). Ac₂O (20 µL, 0.38 mmol, 20.0 equiv) and DMAP (5 mg, 0.019 mmol, 1.0 equiv) were added and the mixture was stirred for 30 min. The reaction was quenched by the addition of saturated aqueous CuSO₄ (500 µL), the layers were separated, and the aqueous layer was washed with DCM (3 × 500 µL). The combined organic fractions were dried with sodium sulfate, filtered, and concentrated to give **16** (9 mg, 87%, crude) as a colorless oil.
**16: ¹H NMR (600 MHz, CDCl₃):** δ 6.14 (d, *J* = 1.9 Hz, 1 H), 5.18 (s, 1 H), 5.14 (d, *J* = 4.5 Hz, 1 H), 4.20 (dd, *J* = 7.5, 4.1 Hz, 1 H), 2.84 (pd, *J* = 8.0, 4.4 Hz, 1 H), 2.33 (qt, *J* = 7.1, 3.5 Hz, 1 H), 2.26 (dd, *J* = 11.3, 4.1 Hz, 1 H), 2.18 (s, 3 H), 1.85 (dd, *J* = 8.3, 3.5 Hz, 2 H), 1.77 (d, *J* = 1.9 Hz, 3 H), 1.07 (s, 3 H), 1.04 (s, 3 H), 0.99 (d, *J* = 7.1 Hz, 3 H), 0.98 - 0.95 (m, 1 H), 0.93 (s, 9 H), 0.79 (d, *J* = 8.1 Hz, 3H), 0.69 (q, *J* = 8.4 Hz, 1H), 0.10 (s, 3H), 0.05 (d, *J* = 3.1 Hz, 3H).

### Example 16: Silyl Deprotection of 16

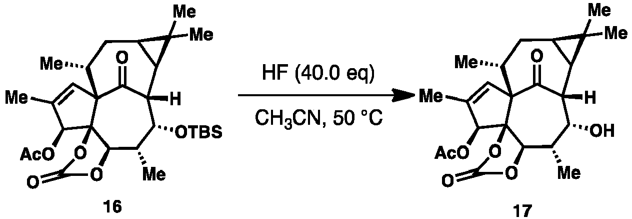

A plastic falcon tube was charged with **16** (82 mg, 0.154 mmol, 1.0 equiv) and CH₃CN (4.5 mL). 47% aqueous HF (336 µL, 9.24 mmol, 60.0 equiv) was added and the mixture was heated to 50 °C. After 10 h the reaction was cooled to rt and quenched by the slow addition of saturated aqueous NaHCO₃ (10 mL). EtOAc (10 mL) was added, the layers were separated, and the aqueous layer was extracted with EtOAc (3 × 10 mL). The combined organic fractions were dried over sodium sulfate, filtered, and concentrated *in vacuo.* Purification of the crude product by flash column chromatography (silica gel, column packed in DCM, then hex/EtOAc = 5:1 → 2:1 → 1:1) afforded **17** (58 mg, 90%) as a colorless oil.
**17: ¹H NMR (400 MHz, CDCl₃):** δ 6.07 (s, 1H), 5.19 - 5.16 (m, 2H), 4.17 (dd, *J* = 8.1, 3.5 Hz, 1H), 3.03 (tt, *J* = 8.2, 4.1 Hz, 1H), 2.72 (d, *J* = 10.9 Hz, 1H), 2.48 - 2.29 (m, 2H), 2.18 (s, 3H), 1.91 - 1.84 (m, 2H), 1.81 - 1.77 (m, 3H), 1.09 (s, 3H), 1.06 (s, 3H), 1.00 (d, *J* = 7.1 Hz, 3H), 0.96 - 0.91 (m, 1H), 0.81 (d, *J* = 8.2 Hz, 3H), 0.72 (q, *J* = 8.3 Hz, 1H).

### Example 17: Triflation of 17

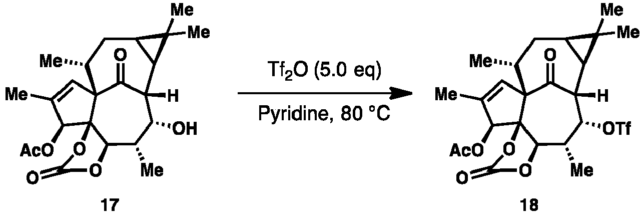

**17** (2 mg, 0.0047 mmol, 1.0 equiv) was dissolved in pyridine (400 µL). Tf₂O (5 µL, 0.0235 mmol, 5.0 equiv) was added dropwise and the mixture was heated to 80 °C. After 1 hour the reaction was cooled and quenched by the slow addition of saturated aqueous NaHCO₃ (1 mL). EtOAc (1 mL) was added, the layers were separated, and the organic layer was washed with saturated aqueous CuSO₄ (3 × 1 mL). The organic layer was dried with sodium sulfate, filtered, and concentrated to give **18,** which was used immediately without further purification.
**18: ¹H NMR (600 MHz, CDCl₃):** δ 6.13 (s, 1H), 5.19 - 5.17 (m, 2H), 5.15 (dd, *J* = 7.8, 4.0 Hz, 1H), 3.26 (td, *J* = 8.0, 4.3 Hz, 1H), 2.53 - 2.48 (m, 1H), 2.43 - 2.38 (m, 1H), 2.35 (t, *J* = 7.5 Hz, 1H), 2.33 - 2.27 (m, 1H), 2.19 (s, 3H), 1.82 - 1.80 (m, 3H), 1.09 (s, 3H), 1.06 (d, *J* = 1.3 Hz, 3H), 1.02 (d, *J* = 7.1 Hz, 3H), 1.00 - 0.96 (m, 1H), 0.95 (d, *J* = 8.0 Hz, 3H), 0.79 (t, *J* = 7.9 Hz, 1H).

### Example 18: Elimination of Triflate 18

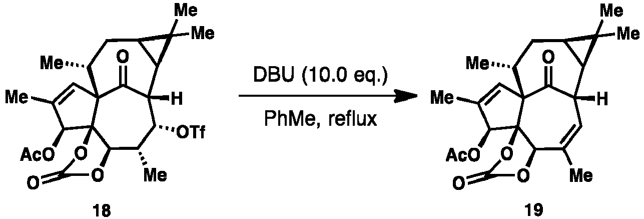

Crude **18** was dissolved in toluene (500 µL). DBU (10 µL, 0.047 mmol, 10.0 equiv) was added and the mixture was heated to 110 °C. After 6 h, the reaction was cooled and quenched by the addition of saturated aqueous CuSO₄ (1 mL). The layers were separated and the aqueous layer was extracted with DCM (3 × 1 mL). The combined organic fractions were dried over sodium sulfate, filtered, and evaporated to give **19,** which was used without further purification.
**19: ¹H NMR (600 MHz, CDCl₃):** δ 6.19 (d, *J* = 1.9 Hz, 1H), 5.97 - 5.93 (m, 1H), 5.44 (s, 1H), 4.55 (s, 1H), 3.53 (d, *J* = 9.2 Hz, 1H), 3.50 (t, *J* = 6.0 Hz, 1H), 2.78 (s, 1H), 2.18 (d, *J* = 2.1 Hz, 3H), 2.07 (p, *J* = 5.9 Hz, 1H), 1.81 (d, *J* = 1.6 Hz, 3H), 1.80 (s, 3H), 1.06 (s, 6H), 1.03 (d, *J* = 7.2 Hz, 3H), 0.98 - 0.93 (m, 1H), 0.71 (dd, *J* = 9.6, 6.6 Hz, 1H).

### Example 19: Deprotection of 19

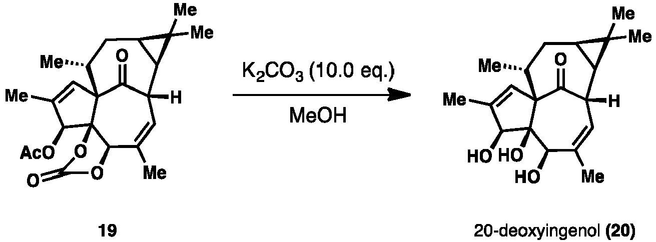

Crude **19** was dissolved in MeOH (500 µL) under ambient atmosphere and K₂CO₃ (1 mg, 0.0047 mmol, 10 equiv) was added. After 15 min saturated aqueous NaHCO₃ (1 mL) and DCM (1 mL) were added. The layers were separated and the aqueous layer was extracted with DCM (3 × 1 mL). The combined organic fractions were dried with sodium sulfate and evaporated. The crude mixture was purified by preparative TLC (3:1 Et₂O:Hex) to give 20-deoxyingenol **(20)** (0.5 mg, 33% over 3 steps) as a white solid (for NMR data, see Uemura et al., "Isolation and structures of 20-deoxyingenol new diterpene, derivatives and ingenol derivative obtained from 'kansui'," Tet. Lett. 29:2527-2528, 1974, incorporated herein by reference in its entirety for all purposes).

### Example 20: Allylic Oxidation of 20

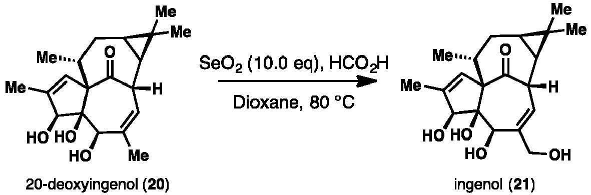

To a solution of **20** (4.9 mg, 0.015 mmol, 1.0 equiv) in dioxane (500 µL) and formic acid (250 µL) was added SeO₂ (17 mg, 0.15 mmol, 10.0 equiv). The suspension was heated to 80 °C for 2 h then cooled and quenched by the addition of saturated aqueous NaHCO₃ (4 mL) and Et₂O (4 mL). The aqueous layer was removed and 10% sodium hydroxide (4 mL) was added. The biphasic mixture was vigorously shaken and the organic layer was removed. The aqueous layer was extracted with Et₂O (2 × 4 mL) and the combined organic layers were dried and evaporated. The crude product was purified by column chromatography (1:1 DCM:EtOAc) to give ingenol (**21**, 3.6 mg, 70%) as a white film (for NMR data describing ingenol **(21),** see Appendio et al., "An Expeditious Procedure for the Isolation of Ingenol from the Seeds of Euphorbia lathyris," J. Nat. Prod., 62:76-79, 1999, incorporated herein by reference in its entirety for all purposes).

### Example 21: IBX Oxidation of 22

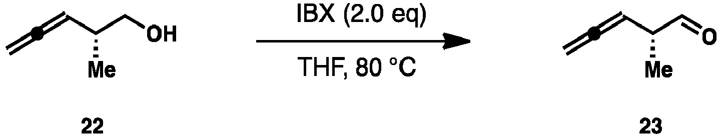

A round-bottom flask was charged with a solution of **22** (100 mg, 1.02 mmol, 1.0 equiv) in THF (4 mL). IBX (571 mg, 2.04 mmol, 2.0 equiv) was added, the flask was tightly sealed with a yellow cap and the resulting suspension was heated to 80 °C for 1 h. The suspension was cooled down and subsequently filtered through a plug of cotton. The residue was washed with THF (2 mL) and the filtrate containing aldehyde **23** (85 mg, 86%) was used in the next step without further purification (Note: the yield of the reaction was determined by NMR using trimethoxybenzene as an internal standard).
**23: ¹H NMR (400 MHz, CDCl₃):** δ 9.58 (dd, *J* = 1.7, 0.5 Hz, 1H), 5.23 (q, *J* = 6.7 Hz, 1H), 4.85 (ddd, *J* = 6.7, 3.1, 0.5 Hz, 2H), 3.06 - 2.96 (m, 1H), 1.22 (dd, *J* = 7.0, 0.5 Hz, 3H).

### PROCEDURE 2: Shorter Protocol - Scheme 2

The procedure for obtaining compound **3** are as described above, in Examples 1 and 2. Conversion of compound **22** to reactant compound **23** proceeds substantially as described above, in Example 21.

### Example 22:

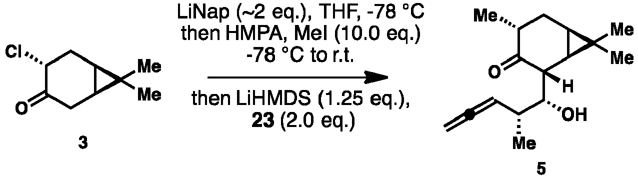

A 1L three-neck flask was charged with naphthalene and freshly distilled THF (550 mL). To this solution, freshly cut lithium (1.54 g, 222 mmol, 6.0 equiv) was added. The mixture was sonicated for 2.5 h. A separate 1L three-neck flask was charged with a solution of **3** (6.39 g, 37 mmol, 1.0 equiv) in freshly distilled THF (160 mL) and cooled to -78 °C. The freshly prepared Li-napthalene solution was slowly added over 40 min until the dark-green color of the reaction mixture persisted for ca. 1 min. A solution of HMPA (38 mL) and methyl iodide (23 mL, 370 mmol, 10 equiv) in THF (50 mL) was added over 15 min and the resulting reaction mixture was stirred at -78 °C for 1 h.

The flask containing the reaction mixture was transferred into a water bath (r.t.) and stirring was continued for 20 min. Then, excess methyl iodide was removed by applying vacuum (ca. 200 torr for 15 min).

The reaction mixture was cooled back to -78 °C, LiHMDS (46.3 mL, 46.3 mmol, 1.25 equiv) was added dropwise over 15 min and stirring was continued for 1 h. The freshly prepared solution of aldehyde **23** (2.0 equiv, see preparation of **23** below) was next added over 30 min and stirring was continued at -78 °C. After 4 h, the reaction was quenched by addition of saturated aqueous NH₄Cl (100 mL). The reaction mixture was extracted with EtOAc (3 × 500 mL) and the combined organic layers were dried over sodium sulfate, filtered and concentrated under reduced pressure. Purification of the residue by flash column chromatography (silica gel, gradient: hexanes, then hexanes/EtOAc = 40:1 → 30:1 → 20:1 → 10:1) yielded **5** (4.08 g, 44%) as a colorless liquid.

### Example 23

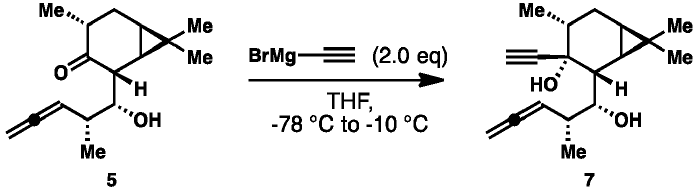

A solution of ketone **5** (3.0 g, 12.1 mmol, 1.0 equiv) in THF (120 mL) was cooled to -78 °C and Ethynylmagnesium bromide (0.5 M solution in THF, 121 mL, 60.4 mmol, 5.0 equiv) was added dropwise. The reaction mixture was warmed to -10 °C and stirring was continued for 2 h. Saturated aqueous NH₄Cl (40 mL) was added and the mixture was extracted with EtOAc (3 × 15 mL). The combined organic layers were dried over Na₂SO₄, filtered and concentrated under reduced pressure. Purification of the crude product by flash column chromatography (silica gel, hexanes/EtOAc = 10:1 → 5:1) afforded alcohol 7 (2.69 g, 81%) as an inseparable 10:1 mixture of diastereomers and a colorless oil.

### Example 24

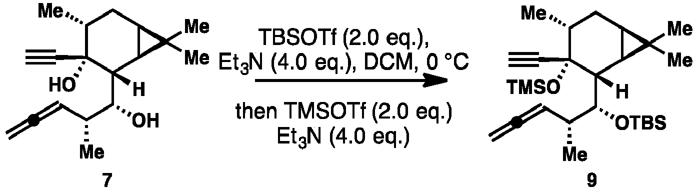

To a solution of **7** (2.69 g, 9.80 mmol, 1.0 equiv) in DCM (49 mL) was added triethylamine (5.46 mL, 39.2 mmol, 4.0 equiv) then TBSOTf (4.49 mL, 19.6 mmol, 2.0 equiv) dropwise at 0 °C. After 30 min, the starting material was judged as consumed by TLC, and triethylamine (5.46 mL, 39.2 mmol, 4.0 equiv) then TMSOTf (3.5 mL, 19.6 mmol, 2.0 equiv) were added dropwise. The reaction mixture was stirred at 0 °C for 1.5 h before being quenched with saturated aqueous NaHCO₃ (50 mL). The mixture was extracted with DCM (3 × 50 mL) and the combined organic layers were dried over Na₂SO₄, filtered and concentrated under reduced pressure. Purification of the crude product by flash column chromatography (silica gel, hexanes) afforded **9** (3.22 g, 71%) as a viscous colorless oil that solidified upon cooling.

Conversion of compound **9** to compound **10** and then to compounds **11, 12, 13,** and **14,** proceed substantially as described above, in Example 9 through Example 13, respectively. Conversion of compound **14** to compound **16** is described in the next Example.

### Example 25

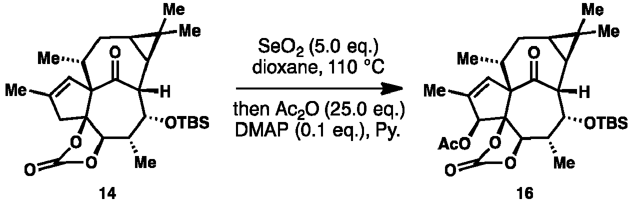

To a solution of **14** (128 mg, 0.26 mmol, 1.0 equiv) in dioxane (7.5 mL) was added SeO₂ (144 mg, 1.3 mmol, 5.0 equiv). The flask was sealed with a yellow cap and the suspension was heated to 80 °C for 14 h. The suspension was cooled to rt and pyridine (1.05 mL, 13 mmol, 50 equiv), Ac₂O (614 µL, 6.5 mmol, 25 equiv) and DMAP (3.2 mg, 0.026 mmol, 0.1 equiv) were added. The mixture was stirred for 45 min, diluted with EtOAc (20 mL) and filtered through celite. The filtrate was washed with saturated aqueous CuSO₄ (20 mL) and the combined organic layers were dried over sodium sulfate, filtered and evaporated. Purification of the crude product by flash column chromatography (silica gel, column packed in DCM, then hex/EtOAc = 20:1 → 10:1 → 5:1) afforded **16** (82 mg, 59%) as an orange oil.

### Example 26

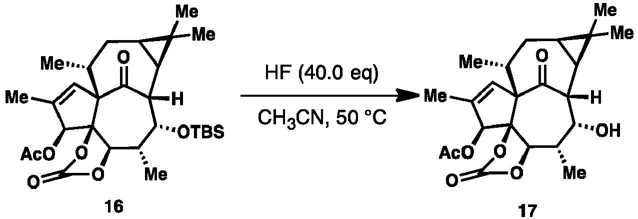

A plastic falcon tube was charged with 16 (82 mg, 0.154 mmol, 1.0 equiv) and CH₃CN (4.5 mL). 47% aqueous HF (336 µL, 9.24 mmol, 60.0 equiv) was added and the mixture was heated to 50 °C. After 10 h the reaction was cooled to rt and quenched by the slow addition of saturated aqueous NaHCO₃ (10 mL). EtOAc (10 mL) was added, the layers were separated, and the aqueous layer was extracted with EtOAc (3 × 10 mL). The combined organic fractions were dried over sodium sulfate, filtered, and concentrated *in vacuo.* Purification of the crude product by flash column chromatography (silica gel, column packed in DCM, then hex/EtOAc = 5:1 → 2:1 → 1:1) afforded **17** (58 mg, 90%) as a colorless oil.

### Example 27

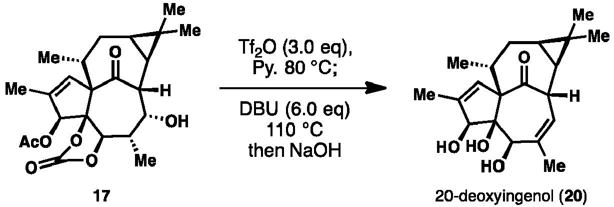

To a solution of **17** (9.0 mg, 0.0215 mmol, 1.0 equiv) and DMAP (0.26 mg, 0.00215 mmol, 0.1 equiv) in of pyridine (1 mL), was added Tf₂O (11 µL, 0.0645 mmol, 3.0 equiv). The solution was warmed to 80 °C for 30 min. To the solution was added DBU (19 µL, 0.129 mmol, 6.0 equiv). The solution was heated to 110 °C for 30 min. The mixture was cooled and 10% aqueous sodium hydroxide (200 µL) was added. The mixture was stirred for 30 min then quenched by the addition of 3 mL of saturated aqueous NaHCO₃ and 3 mL of EtOAc. The aqueous layer was removed and the organic layer was washed with saturated aqueous CuSO₄ (2 × 3 mL). The organic layer was dried and evaporated to give crude **20.** The crude material was purified by column chromatography (50:1 DCM/MeOH) to give 20-deoxyingenol (**20**, 2.0 mg, 28%).

### Example 28

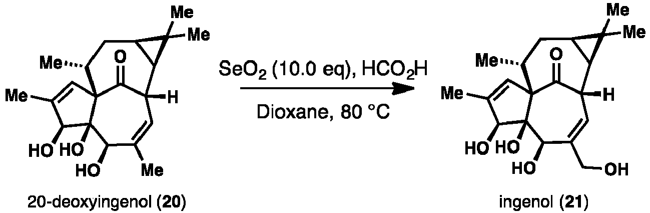

To a solution of **20** (4.9 mg, 0.015 mmol, 1.0 equiv) in dioxane (500 µL) and formic acid (250 µL) was added SeO₂ (17 mg, 0.15 mmol, 10.0 equiv). The suspension was heated to 80 °C for 2 h then cooled and quenched by the addition of saturated aqueous NaHCO₃ (4 mL) and Et₂O (4 mL). The aqueous layer was removed and 10% sodium hydroxide (4 mL) was added. The biphasic mixture was vigorously shaken and the organic layer was removed. The aqueous layer was extracted with Et₂O (2 × 4 mL) and the combined organic layers were dried and evaporated. The crude product was purified by column chromatography (1:1 DCM:EtOAc) to give ingenol (**21**, 3.6 mg, 70%) as a white film.

### ALTERNATIVE PROCEDURE: Conversion of 10 to 26 - Scheme 4

### Example 29

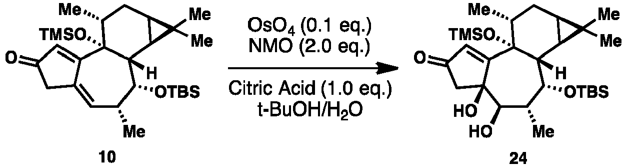

**10** (30 mg, 0.061 mmol) was dissolved in a mixture of water (1.0 ml) and *t*-butanol (1.0 ml). A solution of OsO₄ in *t*-butanol (200 µl, 0.058 M, 0.006 mmol, 0.1 eq) was added. *N-*Methylmorpholine-*N*-oxide (NMO) (14 mg, 0.123 mmol, 2.0 eq) and citric acid (24 mg, 0.123 mmol, 2.0 eq) were added. The mixture was stirred at room temperature for 16 h. Saturated aqueous Na₂SO₃ (5 ml) was added and the mixture was extracted with ethyl acetate (3 × 5 ml). The combined organics were dried (Na₂SO₄), filtered, and the solvent was evaporated. The residue was subjected to flash chromatography (pentane to 3:1 pentane/EtOAc) yielding white crystals of **24** (25 mg, 78%).
**24: ¹H NMR (500 MHz, CDCl₃):** δ 6.19 (s, 1 H), 3.82 (d, *J* = 1.7 Hz, 1 H), 3.34 (d, *J* = 10.0 Hz, 1 H), 3.14 (d, *J* = 17.1 Hz, 1 H), 2.71 (s, 1 H), 2.55 (d, *J* = 17.1 Hz, 1 H), 2.18 (s, 1 H), 1.99 - 1.89 (m, 1 H), 1.86 (m, 1 H), 1.72 - 1.65 (m, 1 H), 1.60 (m, 1 H), 1.15 (d, *J* = 6.9 Hz, 3 H), 1.10 (s, 3 H), 0.97 (s, 3 H), 0.85 (s, 9 H), 0.75 - 0.72 (m, 2 H), 0.71 (d, *J* = 6.9 Hz, 3 H), 0.27 (s, 9 H), 0.16 (s, 3 H), 0.10 (s, 3 H).

### Example 30

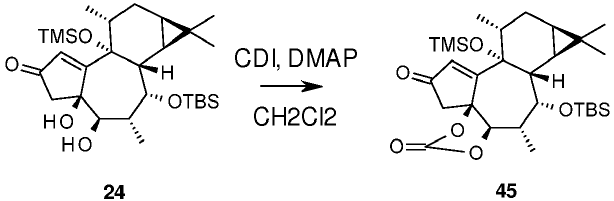

To a solution of **24** (80 mg, 0.15 mmol, 1.0 equiv.) in CH2Cl2 (3 mL) was added CDI (50 mg, 0.30 mmol, 2.0 equiv.) and DMAP (2 mg, 0.015 mmol, 0.1 equiv.). The solution was stirred at room temperature for 3 hours. Additionally CDI (50 mg, 0.30 mmol, 2.0 equiv.) and DMAP (2 mg, 0.015 mmol, 0.1 equiv) were added and stirring was continued for 3 hours. The reaction was quenched by the addition of saturated aqueous CuSO4 (4 mL). The organic layer was removed and the aqueous layer was extracted with CH2Cl2 (3 × 4 mL). The combined organic fractions were dried with Na2SO4, filtered, and concentrated. The crude product was purified by column chromatography (10:1 Hex/EtOAc) to give **45** (67 mg, 80%) as a white foam.
**45: 1H-NMR** (600 MHz, CDCl3) δ 6.31 (s, 1 H), 4.81 (d, *J* = 5.1 Hz, 1 H), 4.35 (dd, *J* = 8.1, 5.1 Hz, 1H), 3.16 (d, *J* = 18.2 Hz, 1 H), 2.90 (pd, *J* = 7.9, 5.1 Hz, 1 H), 2.79 (d, *J* = 18.2 Hz, 1 H), 1.79 -1.72 (m, 1 H), 1.58 - 1.50 (m, 1 H), 1.46 (dd, *J* = 6.4, 5.1 Hz, 1 H), 1.42 - 1.33 (m, 1 H), 1.17 (dd, *J* = 9.7, 6.4 Hz, 1 H), 1.08 (s, 3 H), 0.98 (s, 3 H), 0.97 - 0.93 (m, 12 H), 0.70 (t, *J* = 8.9 Hz, 1 H), 0.65 (d, *J* = 6.5 Hz, 3 H), 0.22 (s, 9 H), 0.13 (s, 3 H), 0.11 (s, 3 H);
Organometallic methylating reagents, such as for example MeMgBr, MeMgCl or MeMgI may react with the ketone of compound **45** or compound **25** to obtain the tertiary alcohol **26.**

### ALTERNATIVE PROCEDURE: Conversion of 14 to 21 - Scheme 5

### Example 31

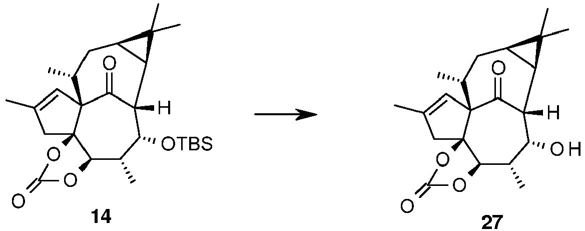

48% aqueous HF (0.33 mL, 9.10 mmol, 140 equiv) was added dropwise to compound **14** (31 mg, 0.065 mmol, 1.0 equiv) in THF (0.66 mL, 0.1 M) in a plastic falcon tube at 0 °C. The reaction was transferred to room temperature and stirred for 12 h, followed by the slow addition of saturated aqueous NaHCO₃ (10 mL) to quench reaction at room temperature. EtOAc (5 mL) was added, the layers were separated, and the aqueous layer was extracted with EtOAc (3 × 5 mL). The combined organic fractions were dried over Na₂SO₄, filtered, and concentrated *in vacuo.* Purification of the crude product by flash column chromatography (silica gel, column packed in Hexane, then hexanes/EtOAc = 9:1 → 8:2 → 1:1) afforded **27** (20 mg, 85%) as a brown oil (*R_{f}* = 0.44 (Hex/EtOAc = 5:5; anisaldehyde)).
¹H NMR (600 MHz, CDCl₃): δ 5.53 (s, 1 H), 4.77 (d, *J* = 4.8 Hz, 1 H), 4.13-4.12 (m, 1 H), 3.13 (d, *J* = 17.8 Hz, 1 H), 2.95-2.88 (m, 1 H), 2.64 (s, 1 H), 2.57 (d, *J* = 17.9 Hz, 1 H), 2.49-2.43 (m, 1 H), 1.87 (t, *J* = 6.0 Hz, 1 H), 1.75 (s, 3 H), 1.12 (s, 3 H), 1.05 (s, 3 H), 0.95 (d, *J* = 6.9 Hz, 3 H), 0.71 (q, *J* = 7.1 Hz, 1 H).
¹³C NMR (151 MHz, CDCl₃): 6 211.0, 152.9, 135.7, 123.5, 93.5, 92.8, 76.4, 67.9, 53.6, 47.1, 38.6, 37.4, 30.9, 29.7, 28.7, 22.9, 22.1, 17.1, 16.6, 14.9, 10.5.

### Example 32

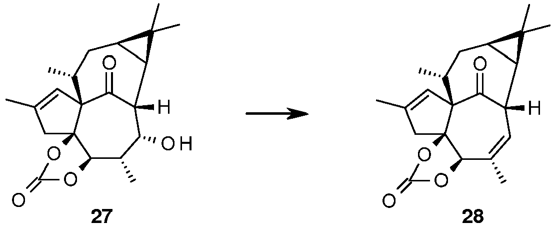

Tf₂O (7.4 µL, 0.0439 mmol, 1.5 equiv) in CH₂Cl₂ (0.10 mL) was added dropwise to compound **27** (10.5 mg, 0.0292 mmol, 1.0 equiv) and DMAP (17.8 mg, 0.146 mmol, 5.0 equiv) in CH₂Cl₂ (0.70 mL) at 0 °C. The reaction was transferred to room temperature and stirred for 20 minutes to afford purple solution, then quenched by addition of ice water (1 mL) and the layers were separated. The aqueous layer was extracted with diethyl ether (3 × 3 mL). The combined organic fractions were dried over Na₂SO₄, filtered, and concentrated *in vacuo.* The resulting residue was purified by short silica gel pad (hexanes/EtOAc = 8:2 as eluent) to afford the required triflate as a yellow oil (*R_{f}* = 0.44 (Hex/EtOAc = 7:3; anisaldehyde)).

To a solution of triflate in toluene (0.7 mL) was added DMAP (17.8 mg, 0.146 mmol, 5.0 equiv). The reaction mixture was stirred at 110 °C for 3 h, then ice water (1 mL) was added at room temperature and the layers were separated. The aqueous layer was extracted with diethyl ether (3 × 3 mL). The combined organic fractions were dried over Na₂SO₄, filtered, and concentrated *in vacuo.* Purification of the crude product by flash column chromatography (silica gel, column packed in Hexane, then hexanes/EtOAc = 9:1) afforded compound **28** (5.2 mg, 52%) as a yellow oil (*R_{f}* = 0.49 (Hex/EtOAc = 7:3; anisaldehyde)).
¹H NMR (600 MHz, CDCl₃): δ 5.95 (dd, *J* = 5.8, 1.6 Hz, 1 H), 5.69 (s, 1 H), 4.57 (s, 1 H), 3.71-3.68 (m, 1 H), 2.99 (d, *J* = 16.4 Hz, 1 H), 2.59 (d, *J* = 16.4 Hz, 1 H), 2.50-2.48 (m, 1 H), 2.10 (qd, *J* = 8.8, 3.1 Hz, 1 H), 1.85-1.81 (m, 1 H), 1.79 (t, *J* = 1.7 Hz, 3 H), 1.77 (s, 3 H), 1.07 (s, 3 H), 1.05 (s, 3 H), 0.98 (d, *J* = 7.0 Hz, 3 H) ,0.72-0.68 (m, 1H).
¹³C NMR (151 MHz, CDCl₃): δ 203.9, 153.7, 135.3, 133.4, 128.1, 124.8, 93.7, 90.3, 72.2, 49.0, 43.5, 36.7, 30.7, 29.7, 28.5, 24.2, 23.0, 22.1, 17.0, 16.8, 15.3.

### CLAUSES

The clauses 1-25, 35-37, 42-58, 60-88 are described herein and the clauses 26-34, 38-41, 59, 89-93 form part of the invention:
1. A method of synthesizing ingenol **(21)** from a compound of formula **5,** which comprises: 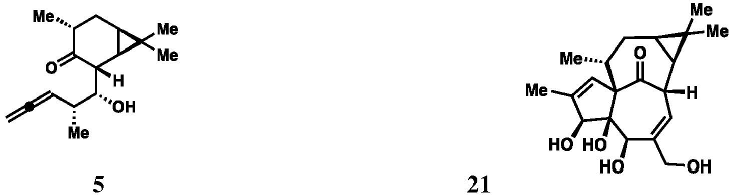 contacting the compound of formula **5** with an alkynylating reagent to form a compound of formula **31** 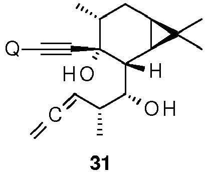 wherein Q is an alkyne protecting group, and
   converting compound **31** to ingenol in one or more steps.
2. The method according to clause 1, which further comprises conversion of ingenol **21** to ingenol mebutate **29** 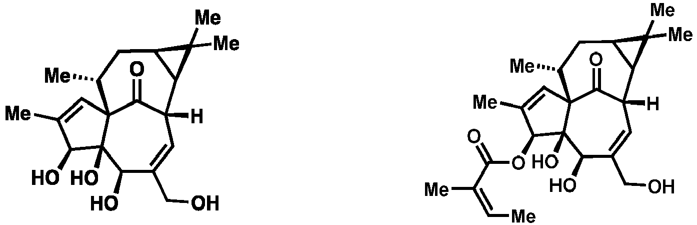 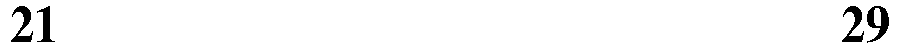
3. The method according to clause 1, wherein the alkynylating reagent is trimethylsilylacetylide.
4. The method according to clause 1, which comprises the preparation of at least one of the intermediates selected from the group consisting of: a compound of formula **4,** a compound of formula **34,** a compound of formula **35,** a compound of formula **37,** and a compound of formula **38** 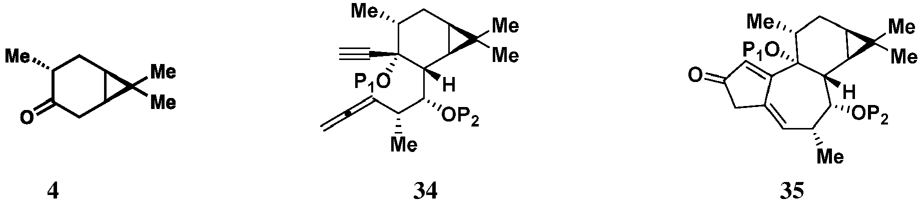 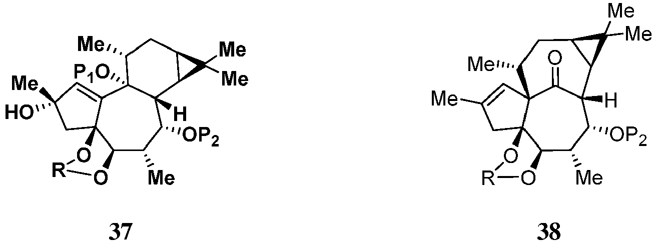 wherein P₁ and P₂ are each individually a hydroxyl protecting group, and
   wherein R is any diol protecting group.
5. The method according to clause 1, which further comprises converting a compound of formula **33** to a compound of formula **34** 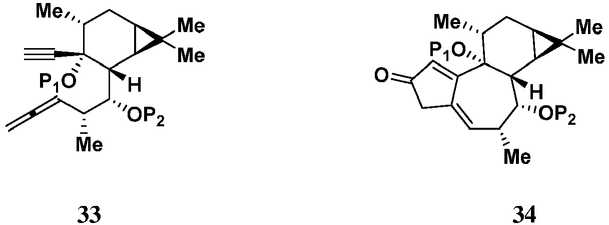 wherein P₁ and P₂ are each individually a hydroxyl protecting group.
6. The method according to clause 5, wherein converting the compound of formula **33** to the compound of formula **34** comprises incubating the compound of formula **33** with a rhodium (I) catalyst.
7. The method according to clause 6, wherein the rhodium (I) catalyst is a chlorodicarbonylrhodium(I) dimer selected from the group consisting of: ([RhCl(CO)₂]₂), [RhCl(COD)]₂, [RhCl(CO)(dppp)]₂, and [Rh(dppp)₂]Cl.
8. The method according to clause 6, wherein incubating of the compound of formula 33 comprises heating the compound of formula **33** to a temperature greater than 140 °C.
9. The method according to clause 8, further comprising dissolving the compound of formula **33** in a high boiling point solvent.
10. The method according to clause 9, wherein the high boiling point solvent is aromatic.
11. The method according to clause 10, wherein the high boiling point aromatic solvent is selected from the group consisting of: xylenes, toluene, mesitylene, and para-dichlorobenzene.
12. The method according to clause 4, which further comprises converting a compound of formula of formula **37** to a compound of formula **38.**
13. The method according to clause 12, wherein converting the compound of formula **37** to the compound of formula **38** occurs by pinacol rearrangement and comprises incubating the compound of formula **37** at a temperature of at least about -50 °C to -78 °C or lower.
14. The method according to clause 12, which further comprises quenching the reaction at least at about -78 °C or lower by addition of bicarbonate.
15. The method according to clause 12, which further comprises dissolving the compound of formula **37** in one or more solvents selected from the group consisting of: dichloromethane, dichloroethane, acetonitrile, and mixtures thereof.
16. The method according to clause 12, which further comprises heating the compound of formula **38** to room temperature in a neutral solution to avoid exposure to acid.
17. The method according to clause 12, which further comprises contacting the compound of formula **37** with a complex of BF₃•Et₂O.
18. The method according to clause 17, wherein BF₃•Et₂O is present in an amount of approximately 10.0 molar equivalents.
19. The method according to clause 1, which further comprises converting (+)-3-carene **(1)** to a compound of formula **4** 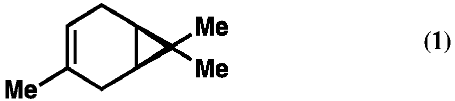
20. The method according to clause 19, wherein conversion of the compound of formula **1** to the compound of formula **4** proceeds through one or more of intermediates of formula **2** and/or **3:** 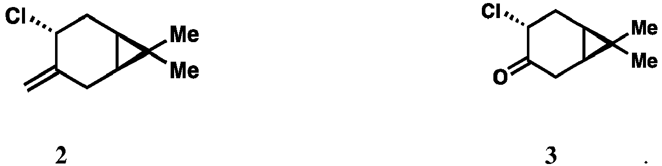
21. The method according to clause 20, which further comprises chlorinating the compound of formula **1.**
22. The method according to clause 20, which further comprises exposing the compound of formula **2** to ozone to yield the compound of formula **3.**
23. The method according to clause 20, which further comprises reductively alkylating the compound of formula **3** to yield the compound of formula **4.**
24. The method according to clause 23, which further comprises reducing the compound of formula **3** at a temperature of -78 °C or lower, followed by alkylating at a temperature of -40 °C or lower.
25. The method according to clause 24, wherein reducing comprises incubating the compound of formula **3** in a lithium-naphthalenide solution to form a reduced compound, and alkylating comprises incubating the reduced compound with methyl iodide.
26. A method of synthesizing ingenol **(21),** which comprises:
   (a) chlorinating the compound of formula 1 to form a compound of formula **2:** 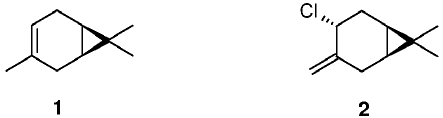
   (b) ozonolysing the compound of formula **2** to form a compound of formula **3:** 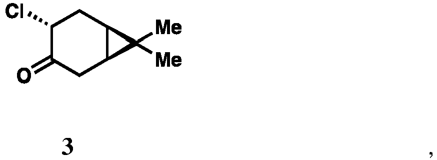
   (c) reductively alkylating **3** to form a compound of formula **4:** 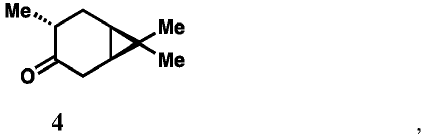
   (d) forming an alcohol of formula **5** from the compound of formula **4:** 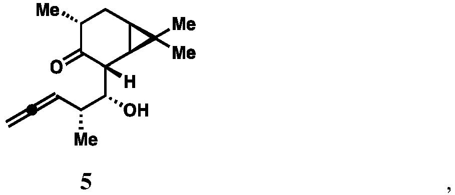
   (e) forming a compound of formula **31** by acetylide addition to the compound of formula 5: 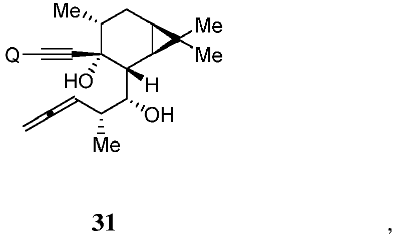
   (f) deprotecting the compound of formula **31** to form a compound of formula **7:** 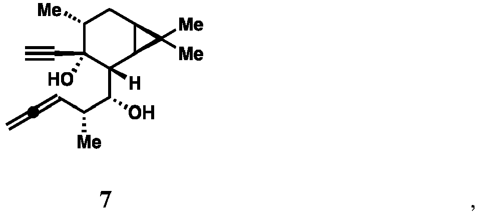
   (g) protecting the compound of formula **7** to form a compound of formula **32:** ,
   (h) protecting the 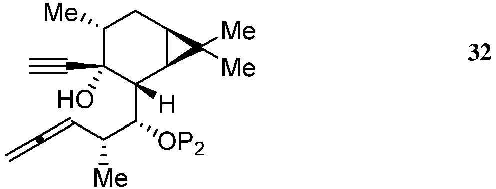 compound of formula **32** to form a compound of formula **33:** 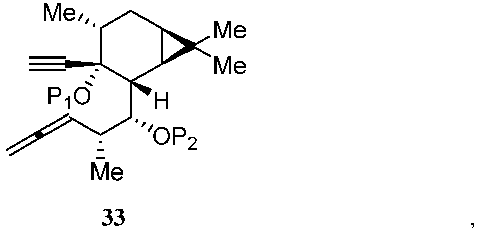
   (i) cyclizing the compound of formula **33** to form a compound of formula **34:** 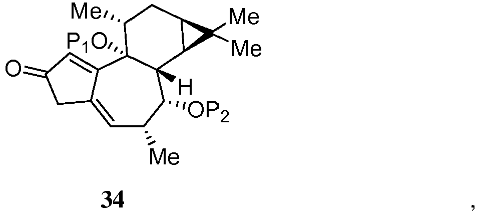
   (j) methylating the compound of formula **34** to form a compound of formula **35:** 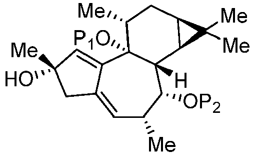 **35** ,
   (k) dihydroxylating the compound of formula **35** to form a compound of formula **36:** 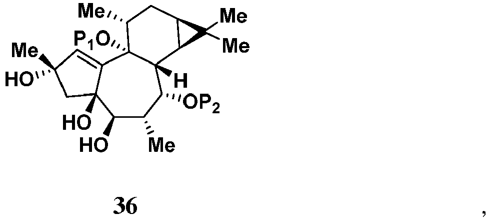
   (l) protecting the compound of formula **36** to form a compound of formula **37:** 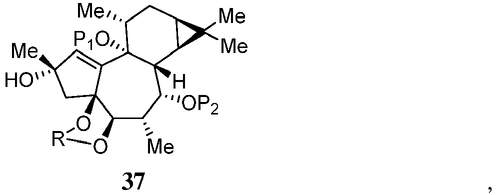
   (m) performing a pinacol rearrangement of the compound of formula **37** to form a compound of formula **38:** 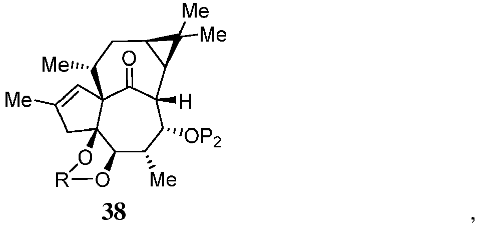
   (n) oxidizing the compound of formula **38** to form a compound of formula **39**: 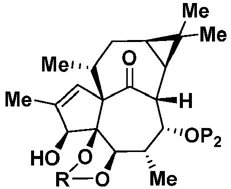 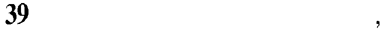
   (o) protecting the compound of formula **39** to form a compound of formula **40:** 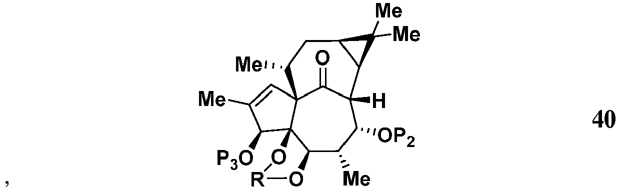
   (p) deprotecting the compound of formula 40 to form a compound of formula **41:** 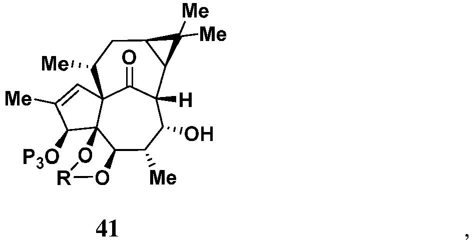
   (q) activating the compound of formula **41** with an hydroxyl activating group to form a compound of formula **42:** 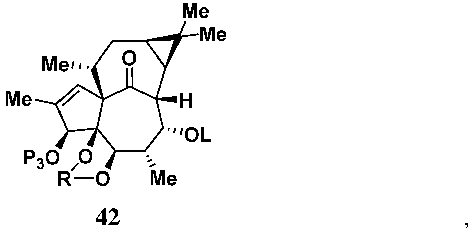
   (r) eliminating the activated hydroxyl group of the compound of formula **42** to form a compound of formula **43**: 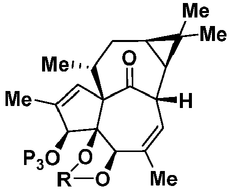 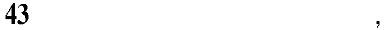
   (s) deprotecting the compound of formula **43** to form a compound of formula **20:** 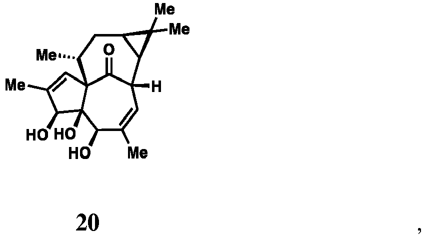 and
   (t) oxidizing the compound of formula **20** to form the compound of formula **21,** 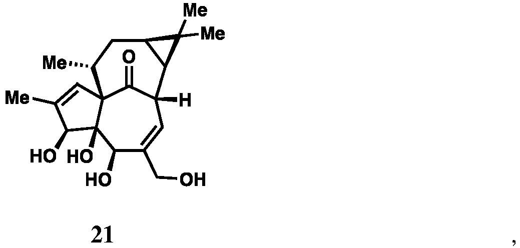 wherein step (d) comprises incubating a reagent of formula **23** with the compound of formula **4:** 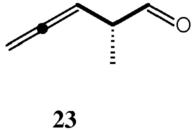 wherein
      P₁, P₂, and P₃ are each individually a hydroxyl protecting group,
      Q is an alkyne protecting group,
      L is an hydroxyl activating group derivative, and
      R is a diol protecting group.
27. The method according to clause 26, wherein steps (e) and (f) are performed in a single reaction vessel by use of telescoping reactions.
28. The method according to clause 26, wherein steps (g) and (h) are performed in a single reaction vessel by use of telescoping reactions.
29. The method according to clause 26, wherein steps (n) and (o) are performed in a single reaction vessel by use of telescoping reactions.
30. The method according to clause 26, wherein one or more of steps (q), (r) and/or (s) are performed in a single reaction vessel by use of telescoping reactions.
31. The method according to clause 26, wherein steps (k) and (l) are performed in a single reaction vessel by use of telescoping reactions.
32. The method according to clause 26, wherein steps (a) and (b) are performed in a single reaction vessel by use of telescoping reactions.
33. The method according to clause 26, wherein in one or more of steps (a), (b), (e), (f), (g), (h), (k), (l), (n), (o), (q), (r) and/or (s) are performed in a single reaction vessel by use of telescoping reactions.
34. The method according to clause 26, which further comprises:
   (u) converting the compound of formula 21 to a compound of formula 29 to form ingenol-3-angelate: 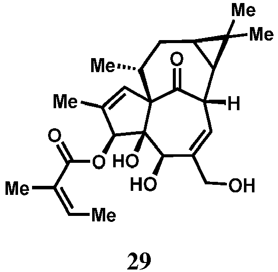
35. The method according to clause 26, wherein the diol protecting group is selected from the group consisting of derivatives of: ketal, acetal, orther ester, bisacetal, silyl, cyclic carbonates and cyclic boronates.
36. A method of synthesizing ingenol (**21**), which comprises:
   removing protecting groups from compound **43** to yield 20-deoxyingenol (**20**): 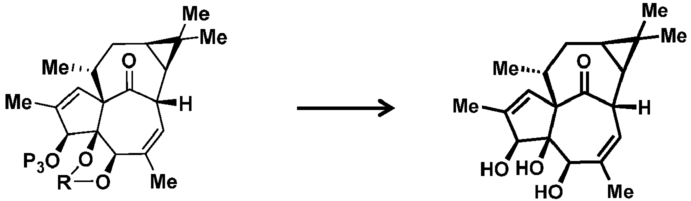 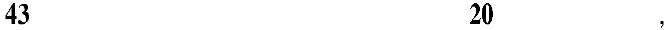 and oxidizing 20-deoxyingenol (**20**) to give ingenol (**21**): 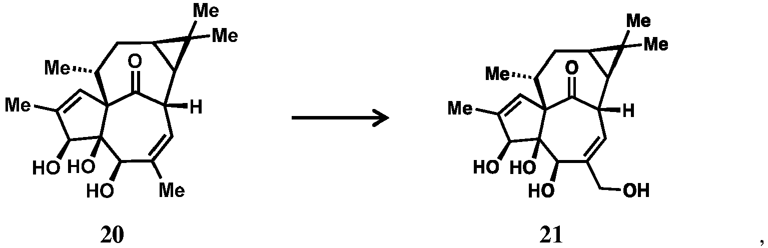 wherein R is a diol protecting group, and P₃ is a hydroxyl protecting group.
37. The method according to clause 36, which further comprises conversion of compound **42** by elimination of -OL to form compound **43:** 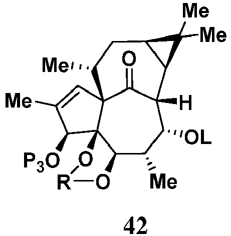 wherein L is a hydroxyl activating group, and R is a diol protecting group.
38. A method of synthesizing compound **34** from compound **7,** which comprises:
   protecting compound **7** hydroxyl moieties to yield compound **33** 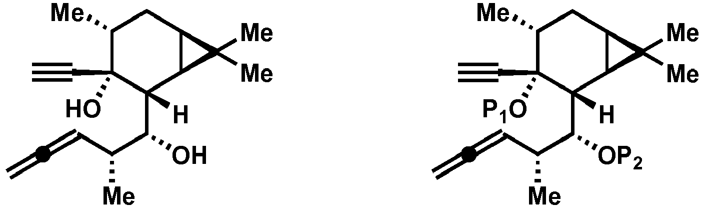 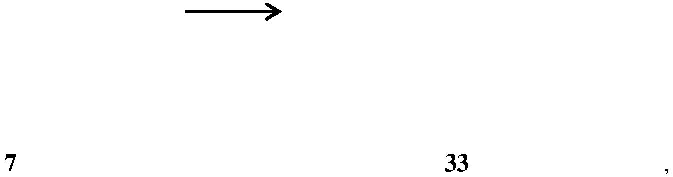 and
   cyclizing compound **33** by incubation of compound **33** with a chlorodicarbonylrhodium(I) dimer selected from the group consisting of: ([RhCl(CO)₂]₂), [RhCl(COD)]₂, [RhCl(CO)(dppp)]₂, and [Rh(dppp)₂]Cl, to yield compound **34:** 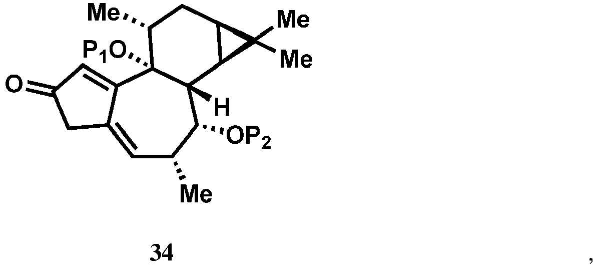 wherein P₁ and P₂ are each individually a hydroxyl protecting group.
39. A method of synthesizing compound **44** from compound **34,** which comprises:
   incubating compound **34** with Grignard reagent XMgBr to produce compound **44** 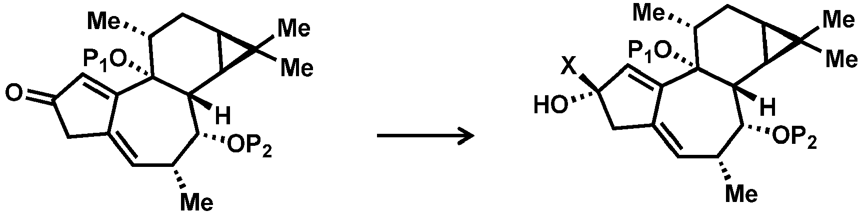 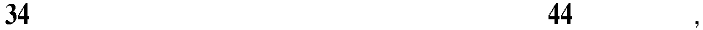 wherein P₁ and P₂ are each individually a hydroxyl protecting group and X is an alkyl group.
40. A method of synthesizing compound **38** from compound **37,** which comprises:
   incubating compound **37** with a Lewis acid under reducing conditions, to yield compound 38 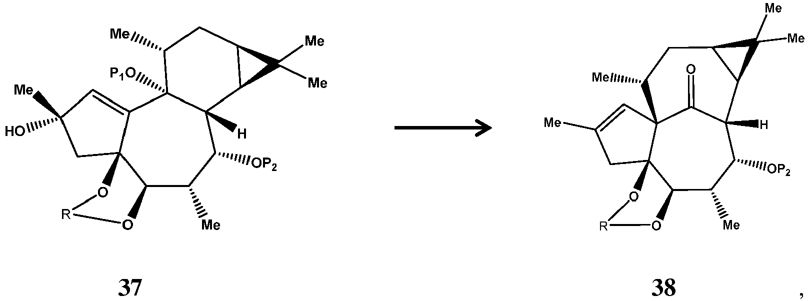 wherein P₁ and P₂ are each individually a hydroxyl protecting group and R is a diol protecting group.
41. The method according to clause 40, wherein the Lewis acid is BF₃•Et₂O.
42. A method of synthesizing ingenol, which comprises the methods according to clause 37, 38 and 40, and which further comprises:
   oxidizing and protecting compound **35** to yield compound **37,** 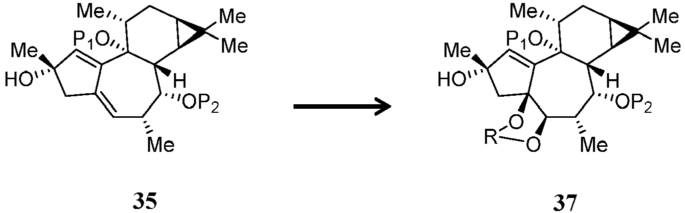 wherein
      P₁ and P₂ are each individually a hydroxyl protecting group, and
      R is a diol protecting group.
43. A compound selected from the group consisting of: 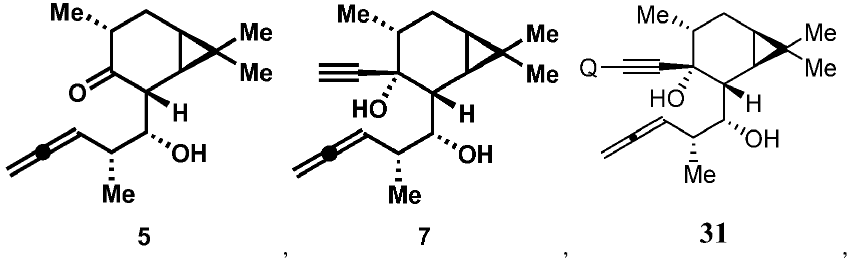 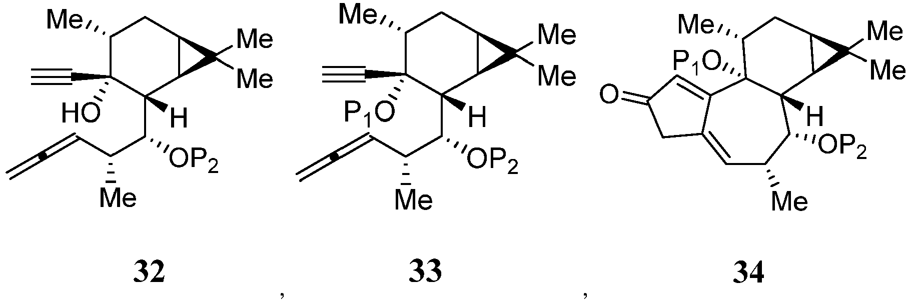 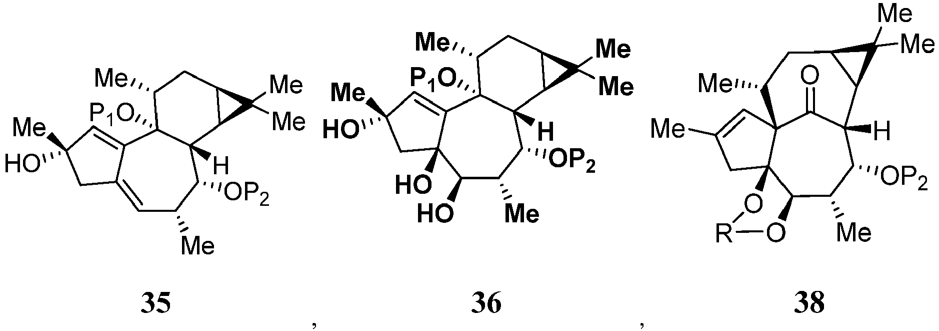 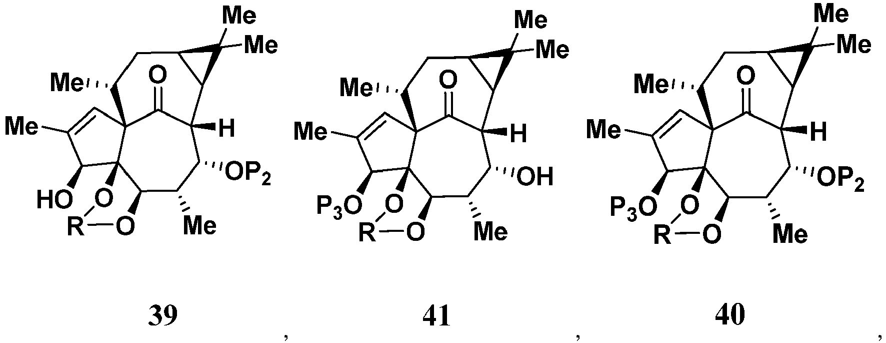 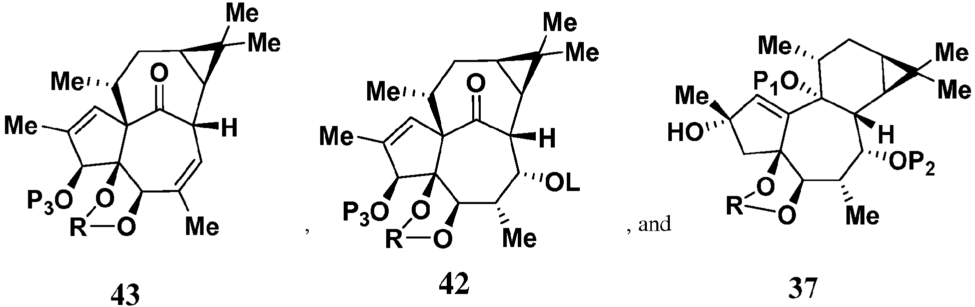
   wherein P₁, P₂, and P₃ are each individually a hydroxyl protecting group,
   Q is an alkyne protecting group,
   L is an hydroxyl activating group, and
   R is a diol protecting group.
44. The compound according to clause 43, which is compound 5.
45. The compound according to clause 43, which is compound 7.
46. The compound according to clause 43, which is compound 31.
47. The compound according to clause 43, which is compound 32.
48. The compound according to clause 43, which is compound 33.
49. The compound according to clause 43, which is compound 34.
50. The compound according to clause 43, which is compound 35.
51. The compound according to clause 43, which is compound 36.
52. The compound according to clause 43, which is compound 37.
53. The compound according to clause 43, which is compound 38.
54. The compound according to clause 43, which is compound 39.
55. The compound according to clause 43, which is compound 40.
56. The compound according to clause 43, which is compound 41.
57. The compound according to clause 43, which is compound 42.
58. The compound according to clause 43, which is compound 43.
59. A compound selected from the group consisting of: 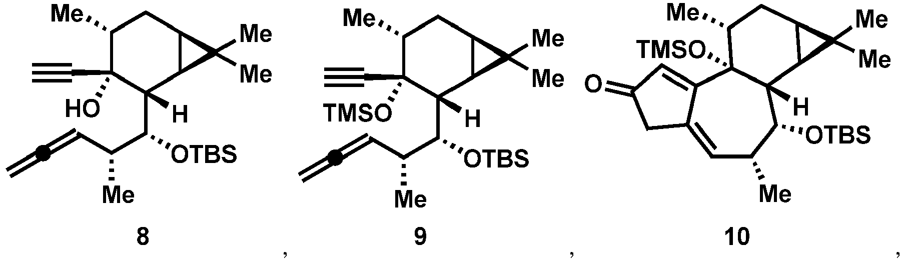 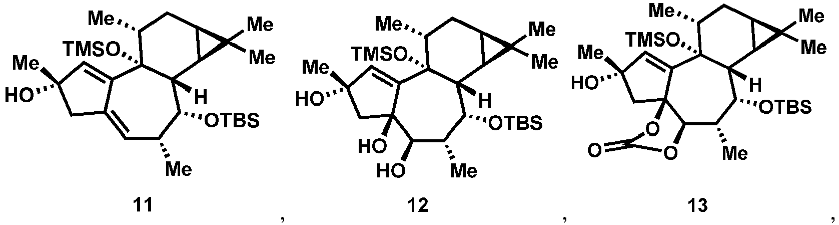 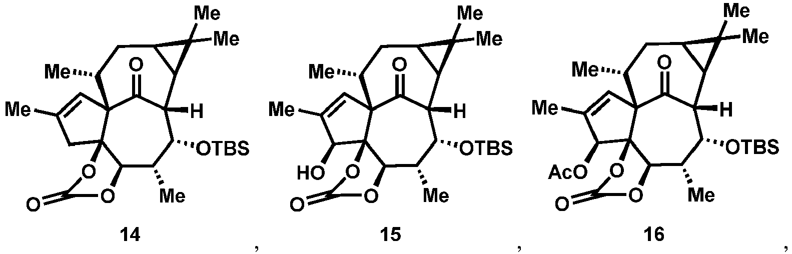 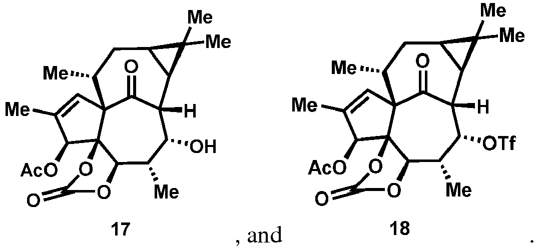
60. The compound according to clause 59, which is compound **8.**
61. The compound according to clause 59, which is compound **9.**
62. The compound according to clause 59, which is compound **10.**
63. The compound according to clause 59, which is compound **11.**
64. The compound according to clause 59, which is compound **12.**
65. The compound according to clause 59, which is compound **13.**
66. The compound according to clause 59, which is compound **14.**
67. The compound according to clause 59, which is compound **15.**
68. The compound according to clause 59, which is compound **16.**
69. The compound according to clause 59, which is compound **17.**
70. The compound according to clause 59, which is compound **18.**
71. A method of synthesizing ingenol (**21**) from a compound of formula **5,** which comprises: 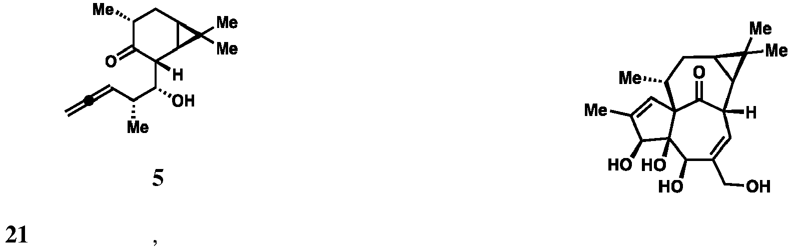 contacting the compound of formula **5** with an alkynylating reagent to form a compound of formula **31** 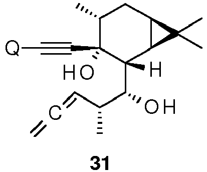 wherein Q is an alkyne protecting group or hydrogen, and
   converting compound **31** to ingenol in one or more steps.
72. The method according to clause 71, which further comprises conversion of ingenol **21** to ingenol mebutate **29** 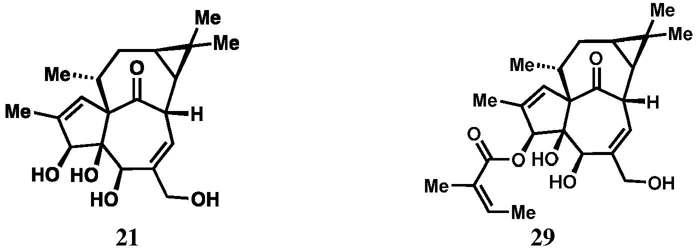
73. The method according to any one of clauses 71-72, wherein the alkynylating reagent is trimethylsilylacetylide.
74. The method according to any one of clauses 71-22, wherein Q is hydrogen.
75. The method according to any one of clauses 71-72 or 74, wherein the alkynylating reagent is ethynyl magnesium bromide.
76. The method according to any one of clauses 71-75, which comprises the preparation of at least one of the intermediates selected from the group consisting of: a compound of formula **4,** a compound of formula 33, a compound of formula **34,** a compound of formula **37,** and a compound of formula **38** 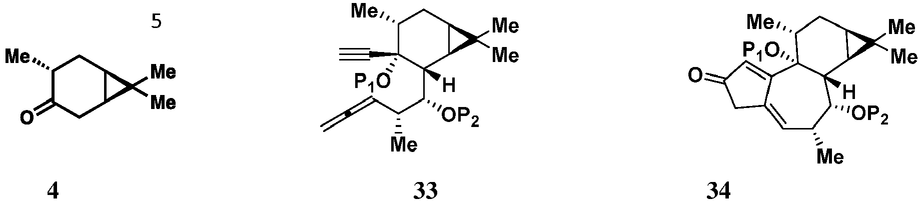 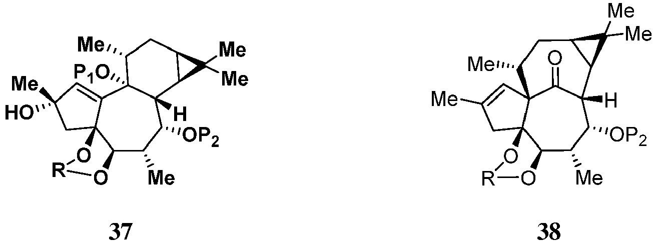 wherein P₁ and P₂ are each individually a hydroxyl protecting group, and
   wherein R is any diol protecting group.
77. The method according to clause 76, which further comprises converting a compound of formula **33** to a compound of formula **34** 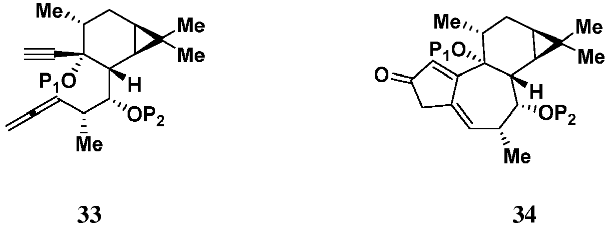 wherein P₁ and P₂ are each individually a hydroxyl protecting group.
78. The method according to clause 77, wherein converting the compound of formula **33** to the compound of formula **34** comprises incubating the compound of formula **33** with a rhodium (**I**) catalyst.
79. The method according to clause 78, wherein the rhodium (**I**) catalyst is a chlorodicarbonylrhodium(**I**) dimer selected from the group consisting of: ([RhCl(CO)₂]₂), [RhCl(COD)]₂, [RhCl(CO)(dppp)]₂, and [Rh(dppp)₂]Cl.
80. The method according to any one of clauses 78-79, wherein incubating of the compound of formula 33 comprises heating the compound of formula **33** to a temperature greater than 140 °C.
81. The method according to clause 76, which further comprises converting a compound of formula of formula **37** to a compound of formula **38.**
82. The method according to claim 81, wherein converting the compound of formula **37** to the compound of formula **38** occurs by pinacol rearrangement and comprises incubating the compound of formula **37** at a temperature of at least about -50 °C to -78 °C or lower.
83. The method according to any one of clauses 81-82, which further comprises contacting the compound of formula **37** with a complex of BF₃•Et₂O.
84. The method according to any one of claims 71-83, which further comprises converting (+)-3-carene (**1**) to a compound of formula **4** 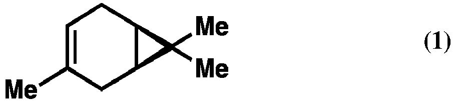
85. The method according to claim 84, wherein conversion of the compound of formula **1** to the compound of formula **4** proceeds through one or more of intermediates of formula **2** and/or **3:** 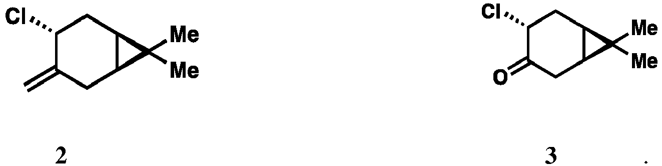
86. A method of synthesizing ingenol (**21**), which comprises:
   (a) chlorinating the compound of formula **1** to form a compound of formula **2:** 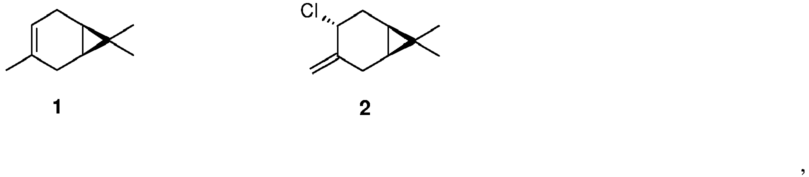
   (b) ozonolysing the compound of formula **2** to form a compound of formula **3:** 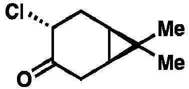 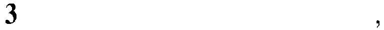
   (c) reductively alkylating **3** to form a compound of formula **4:** 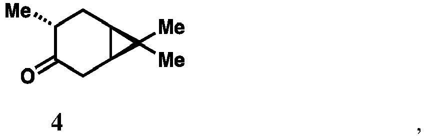
   (d) forming an alcohol of formula **5** from the compound of formula **4:** 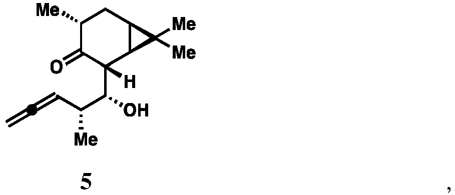
   (e) forming a compound of formula **31** by acetylide addition to the compound of formula **5:** 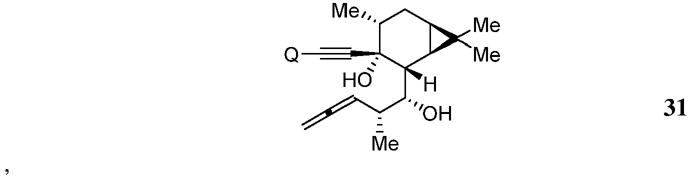
   (f) deprotecting the compound of formula **31** when Q is not hydrogen, to form a compound of formula **7:** 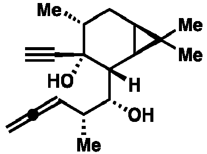 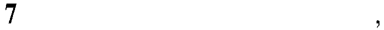
   (g) protecting the compound of formula **7** to form a compound of formula **32:** 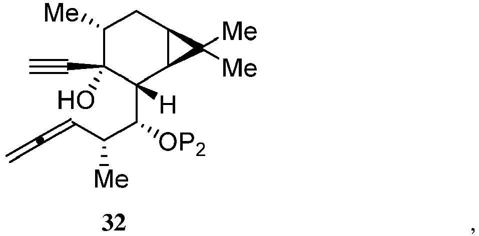
   (h) protecting the compound of formula **32** to form a compound of formula **33:** 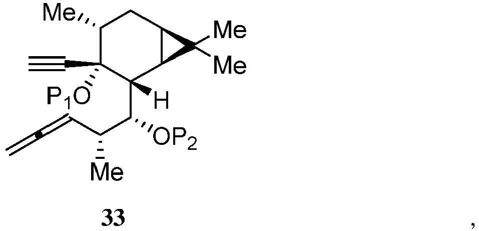
   (i) cyclizing the compound of formula **33** to form a compound of formula **34:** 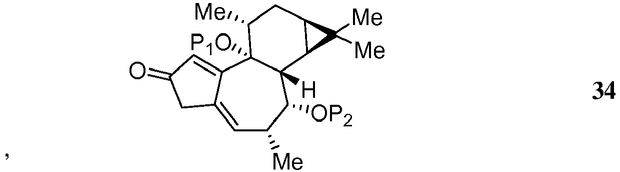
   (j) methylating the compound of formula **34** to form a compound of formula **35:** 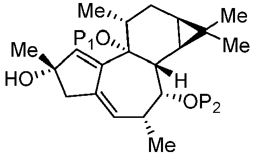 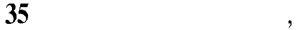
   (k) dihydroxylating the compound of formula **35** to form a compound of formula **36:** 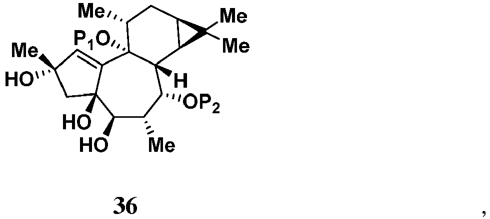
   (l) protecting the compound of formula **36** to form a compound of formula **37:** 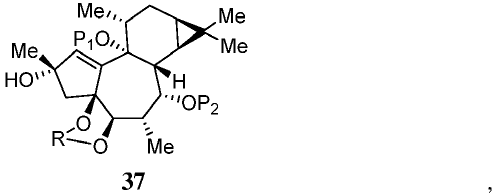
   (m) performing a pinacol rearrangement of the compound of formula **37** to form a compound of formula **38:** 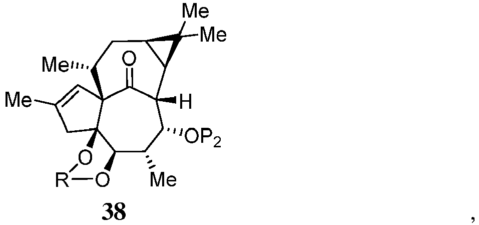
   (n) oxidizing the compound of 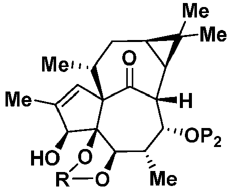 formula **38** to form a compound of formula **39**: 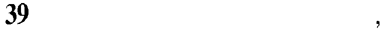
   (o) protecting the compound of formula **39** to form a compound of formula **40:** 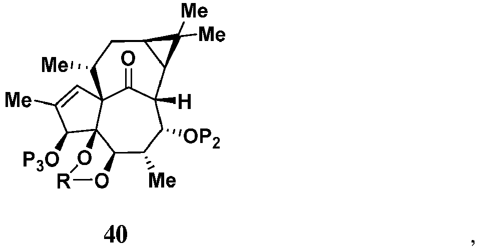
   (p) deprotecting the compound of formula **40** to form a compound of formula **41:** 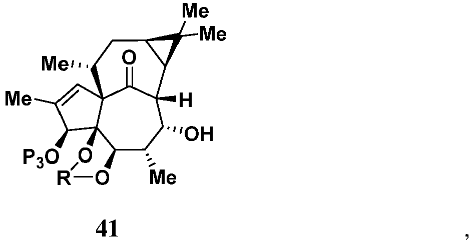
   (q) activating the compound of formula **41** with an hydroxyl activating group to form a compound of formula **42:** ,
   (r) eliminating the activated 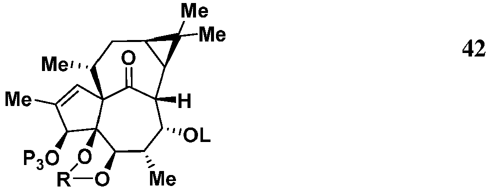 hydroxyl group of the compound of formula **42** to form a compound of formula **43**: 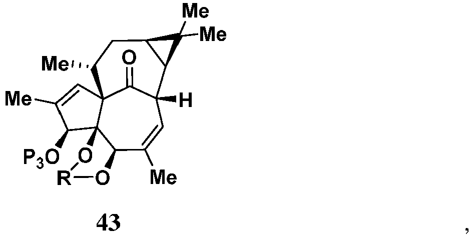
   (s) deprotecting the compound of formula **43** to form a compound of formula **20:** 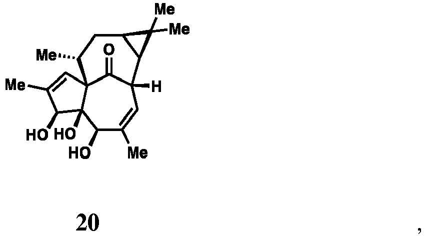 and
   (t) oxidizing the compound of formula **20** to form the compound of formula **21,** 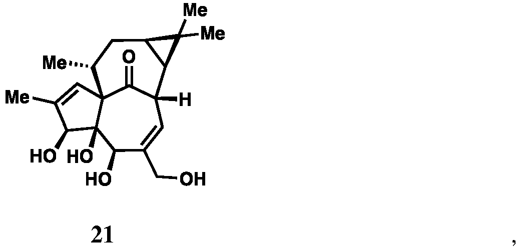 wherein step (d) comprises incubating a reagent of formula **23** with the compound of formula **4:** 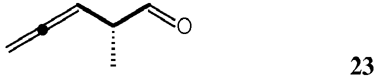 wherein
      P₁, P₂, and P₃ are each individually a hydroxyl protecting group,
      Q is an alkyne protecting group or hydrogen,
      L is an hydroxyl activating group derivative, and
      R is a diol protecting group.
87. The method according to clause 86, wherein in one or more of steps (a), (b), (e), (f), (g), (h), (k), (l), (n), (o), (q), (r) and/or (s) are performed in a single reaction vessel by use of telescoping reactions.
88. The method according to any one of clauses 86-87, which further comprises:
   (u) converting the compound of formula **21** to a compound of formula **29** to form ingenol-3-angelate: 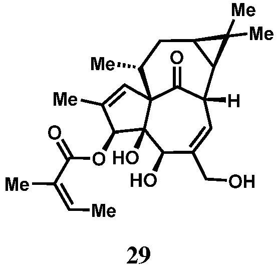
89. A method of synthesizing compound **36** from compound **35,** which comprises: incubating compound **35** with catalytic amounts of OsO₄ to produce compound **36.** 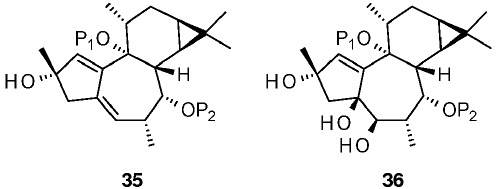 wherein P₁ and P₂ are each individually a hydroxyl protecting group.
90. The method according to clause 89 comprising: incubating compound **35** with catalytic amounts of OsO₄ in the presence of an oxidant and in the presence of a buffer to produce compound **36.**
91. The method according to any one of claims 89-90 wherein the oxidant is selected from the group consisting of trimethylamine-*N*-oxide, *N*-methylmorpholine-N-oxide and tert-butyl hydroperoxide.
92. The method according to any one of claims 90-91 wherein pH of the buffer is within pH 1 - pH 6.
93. The method according to any one of clauses 90-92 wherein the buffer comprise an acid, or salts of an acid, selected from the group consisting of citric acid, phosphoric acid and acetic acid, or mixtures thereof.

## Claims

1. A method of synthesizing ingenol (**21**), which comprises:
(a) chlorinating the compound of formula **1** to form a compound of formula **2:** 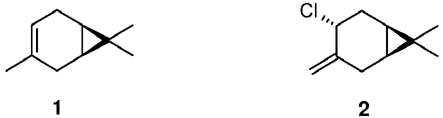
(b) ozonolysing the compound of formula **2** to form a compound of formula **3:** 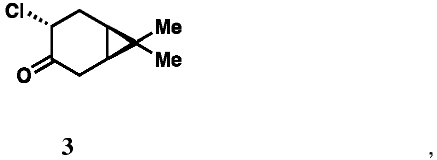
(c) reductively alkylating **3** to form a compound of formula **4:** 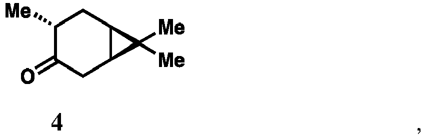
(d) forming an alcohol of formula **5** from the compound of formula **4:** 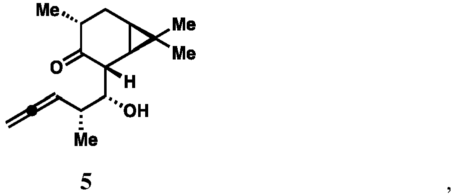
(e) forming a compound of formula **31** by acetylide addition to the compound of formula **5:** 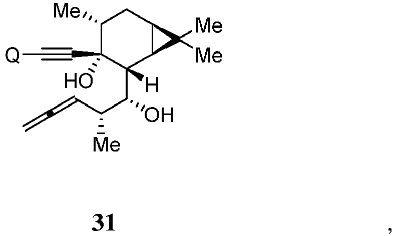
(f) deprotecting the compound of formula **31** when Q is not hydrogen, to form a compound of formula **7:** 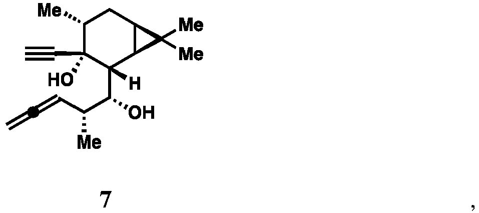
(g) protecting the compound of formula **7** to form a compound of formula **32:** 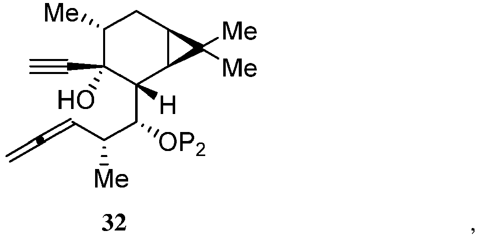
(h) protecting the compound of formula **32** to form a compound of formula **33:** 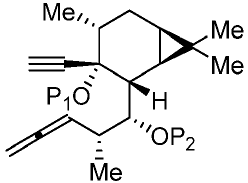 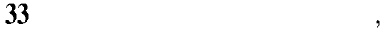
(i) cyclizing the compound of formula **33** to form a compound of formula **34:** 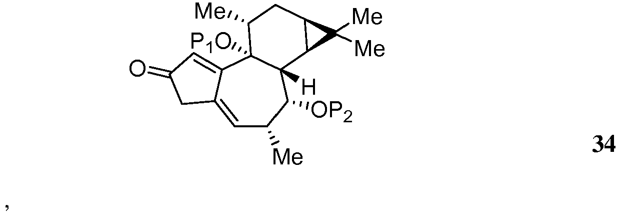
(j) methylating the compound of formula **34** to form a compound of formula **35:** 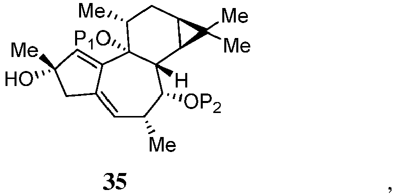
(k) dihydroxylating the compound of formula **35** to form a compound of formula **36:** 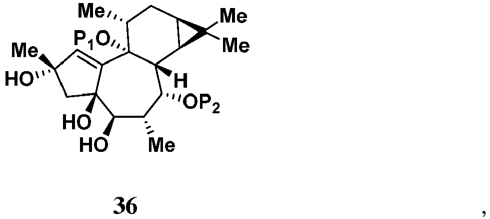
(l) protecting the compound of formula **36** to form a compound of formula **37:** 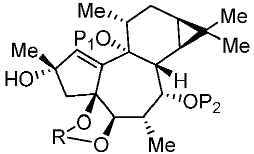 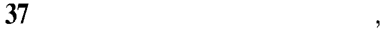
(m) performing a pinacol rearrangement of the compound of formula **37** to form a compound of formula **38:** 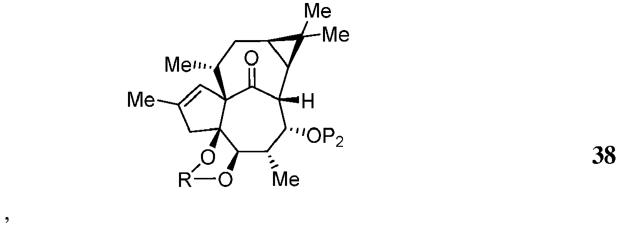
(n) oxidizing the compound of formula **38** to form a compound of formula **39:** 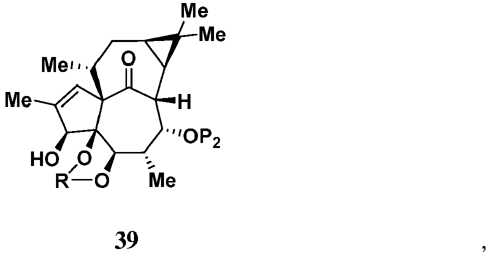
(o) protecting the compound of formula **39** to form a compound of formula **40:** 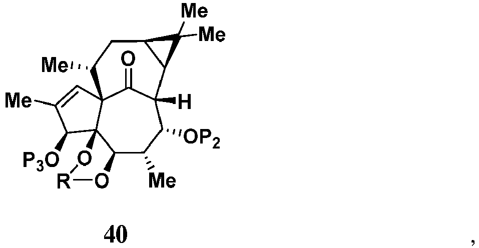
(p) deprotecting the compound of formula **40** to form a compound of formula **41:** 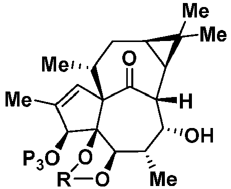 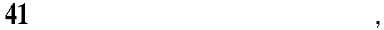
(q) activating the compound of formula **41** with an hydroxyl activating group to form a compound of formula **42:** 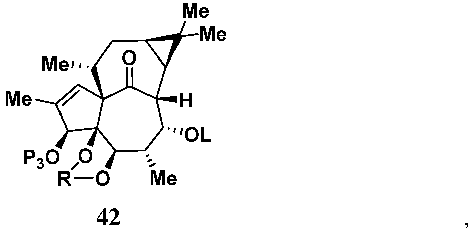
(r) eliminating the activated hydroxyl group of the compound of formula **42** to form a compound of formula **43**: 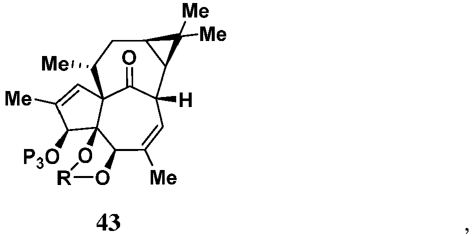
(s) deprotecting the compound of formula **43** to form a compound of formula **20:** 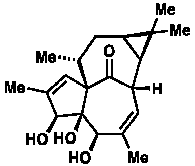 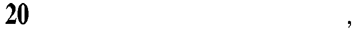 and
(t) oxidizing the compound of formula **20** to form the compound of formula **21,** 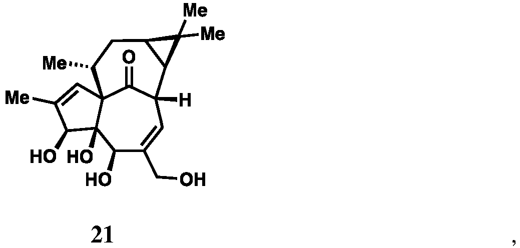 wherein step (d) comprises incubating a reagent of formula **23** with the compound of formula **4:** 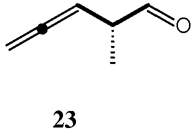 wherein
P₁, P₂, and P₃ are each individually a hydroxyl protecting group,
Q is an alkyne protecting group or hydrogen,
L is an hydroxyl activating group derivative, and
R is a diol protecting group.

2. The method according to claim 1, wherein in one or more of steps (a), (b), (e), (f), (g), (h), (k), (l), (n), (o), (q), (r) and/or (s) are performed in a single reaction vessel by use of telescoping reactions.

3. The method according to any one of claims 1-2, which further comprises:
(u) converting the compound of formula **21** to a compound of formula **29** to form ingenol-3-angelate:

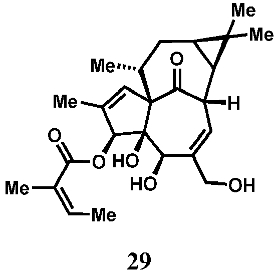

4. A method of synthesizing compound **34** from compound **7,** which comprises:
protecting compound **7** hydroxyl moieties to yield compound 33 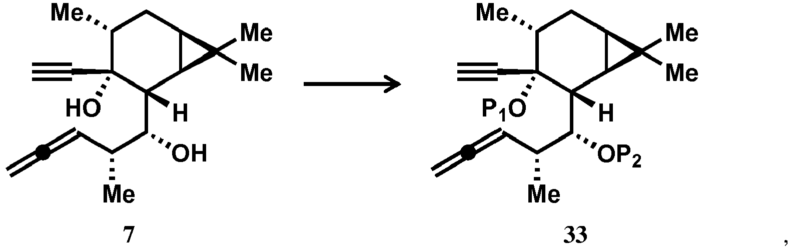 and cyclizing compound **33** by incubation of compound **33** with a chlorodicarbonylrhodium(I) dimer selected from the group consisting of: ([RhCl(CO)₂]₂), [RhCl(COD)]₂, [RhCl(CO)(dppp)]₂, and [Rh(dppp)₂]Cl, to yield compound **34:** 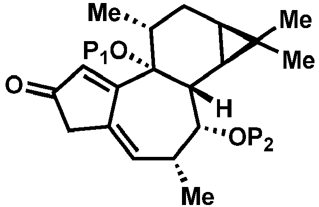 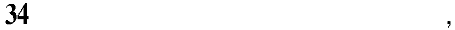 wherein P₁ and P₂ are each individually a hydroxyl protecting group.

5. A method of synthesizing compound **44** from compound **34,** which comprises:
incubating compound **34** with Grignard reagent XMgBr to produce compound **44** 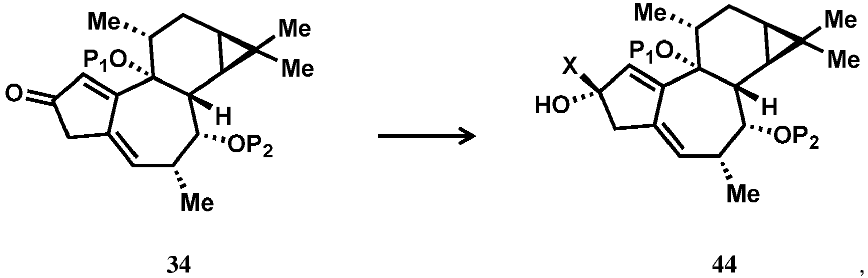 wherein P₁ and P₂ are each individually a hydroxyl protecting group and X is an alkyl group.

6. A method of synthesizing compound **36** from compound **35,** which comprises: incubating compound **35** with catalytic amounts of OsO₄ to produce compound **36.** 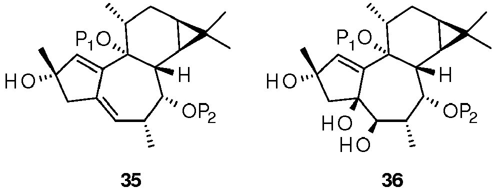 wherein P₁ and P₂ are each individually a hydroxyl protecting group.

7. The method according to claim 6 comprising: incubating compound **35** with catalytic amounts of OsO₄ in the presence of an oxidant and in the presence of a buffer to produce compound **36.**

8. The method according to any one of claims 6-7 wherein the oxidant is selected from the group consisting of trimethylamine-*N*-oxide, *N*-methylmorpholine-N-oxide and tert-butyl hydroperoxide.

9. The method according to any one of claims 7-8 wherein pH of the buffer is within pH 1 - pH6.

10. The method according to any one of claims 7-8 wherein the buffer comprise an acid, or salts of an acid, selected from the group consisting of citric acid, phosphoric acid and acetic acid, or mixtures thereof.

11. A method of synthesizing compound **38** from compound **37,** which comprises:
incubating compound **37** with a Lewis acid under reducing conditions, to yield compound 38 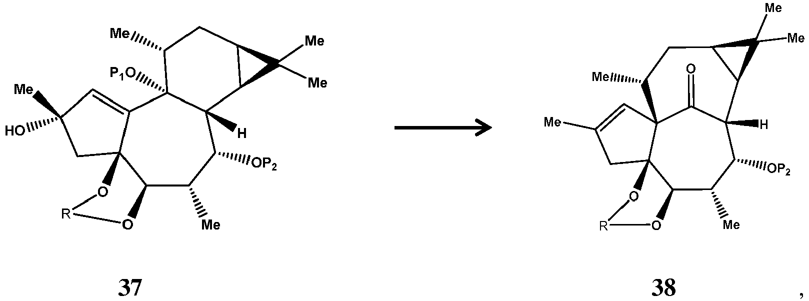 wherein P₁ and P₂ are each individually a hydroxyl protecting group and R is a diol protecting group.

12. The method according to claim 11, wherein the Lewis acid is BF₃•Et₂O.

13. A compound selected from the group consisting of:

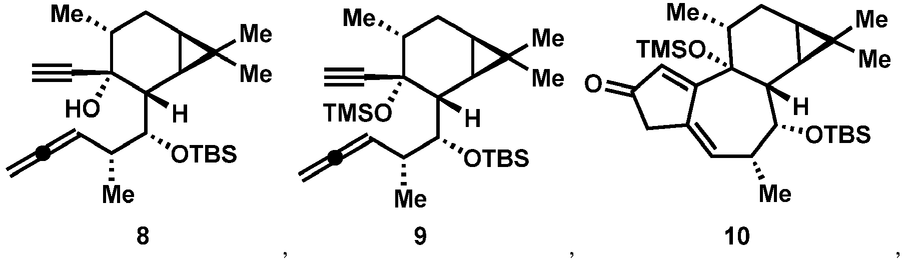

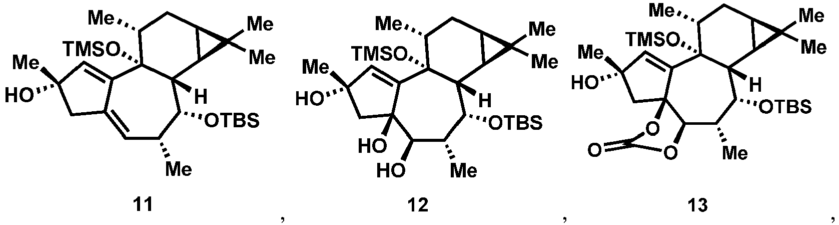

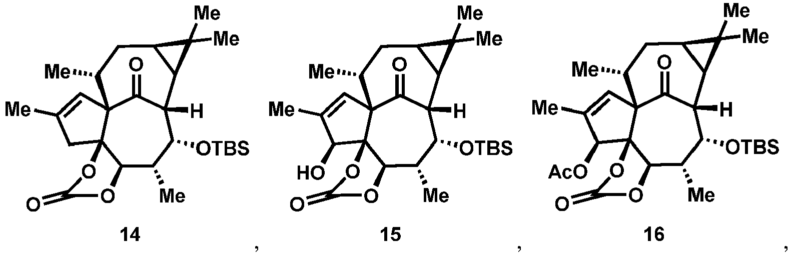

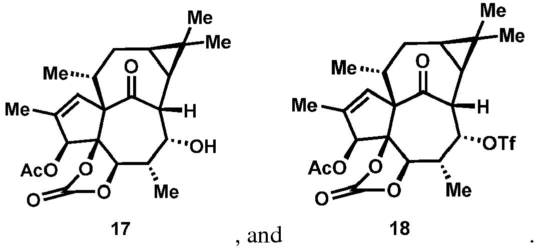

## Patentansprüche

1. Verfahren zum Synthetisieren von Ingenol (21), das Folgendes umfasst:
(a) Chlorieren der Verbindung der Formel 1, um eine Verbindung der Formel 2 zu bilden: 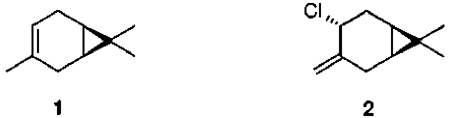
(b) Ozonolysieren der Verbindung der Formel 2, um eine Verbindung der Formel 3 zu bilden: 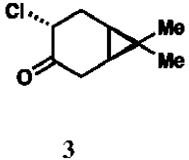
(c) reduktives Alkylieren von 3, um eine Verbindung der Formel 4 zu bilden: 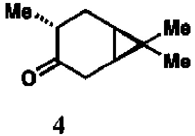
(d) Bilden eines Alkohols der Formel 5 aus der Verbindung der Formel 4: 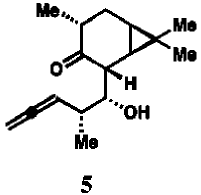
(e) Bilden einer Verbindung der Formel 31 durch Addieren von Acetylid an die Verbindung der Formel 5: 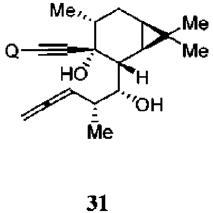
(f) Entschützen der Verbindung der Formel 31, wenn Q kein Wasserstoff ist, um eine Verbindung der Formel 7 zu bilden: 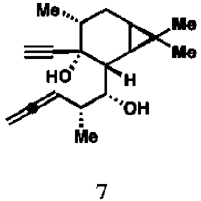
(g) Schützen der Verbindung der Formel 7, um eine Verbindung der Formel 32 zu bilden: 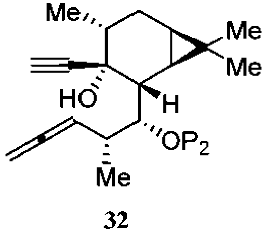
(h) Schützen der Verbindung der Formel 32, um eine Verbindung der Formel 33 zu bilden: 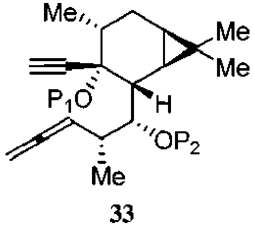
(i) Zyklisieren der Verbindung der Formel 33, um eine Verbindung der Formel 34 zu bilden: 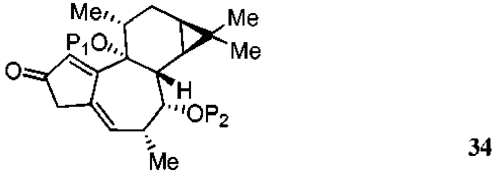
(j) Methylieren der Verbindung der Formel 34, um eine Verbindung der Formel 35 zu bilden: 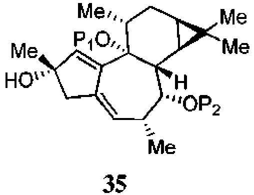
(k) Dihydroxylieren der Verbindung der Formel 35, um eine Verbindung der Formel 36 zu bilden: 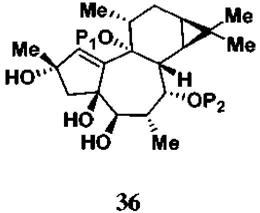
(l) Schützen der Verbindung der Formel 36, um eine Verbindung der Formel 37 zu bilden: 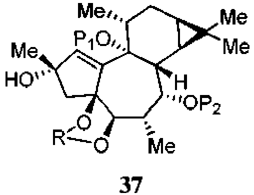
(m) Durchführen einer Pinacol-Umlagerung der Verbindung der Formel 37, um eine Verbindung der Formel 38 zu bilden: 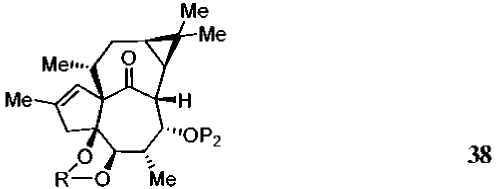
(n) Oxidieren der Verbindung der Formel 38, um eine Verbindung der Formel 39 zu bilden: 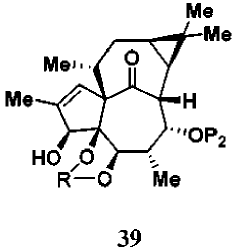
(o) Schützen der Verbindung der Formel 39, um eine Verbindung der Formel 40 zu bilden: 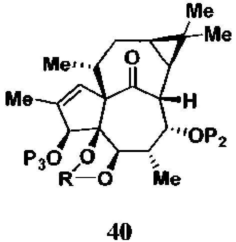
(p) Entschützen der Verbindung der Formel 40, um eine Verbindung der Formel 41 zu bilden: 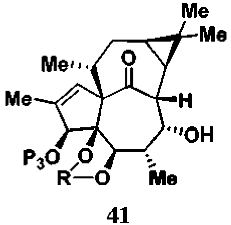
(q) Aktivieren der Verbindung der Formel 41 mit einer Hydroxylaktivierungsgruppe, um eine Verbindung der Formel 42 zu bilden: 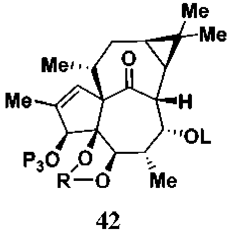
(r) Eliminieren der aktivierten Hydroxylgruppe der Verbindung der Formel 42, um eine Verbindung der Formel 43 zu bilden: 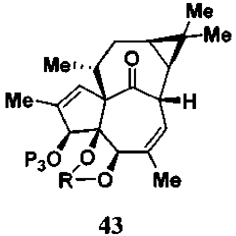
(s) Entschützen der Verbindung der Formel 43, um eine Verbindung der Formel 20 zu bilden: 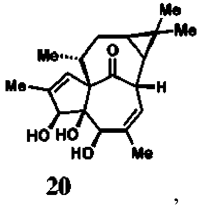 und
(t) Oxidieren der Verbindung der Formel 20, um die Verbindung der Formel 21 zu bilden, 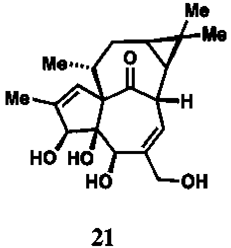 wobei Schritt (d) Inkubieren eines Reagenzes der Formel 23 mit der Verbindung der Formel 4 beinhaltet: 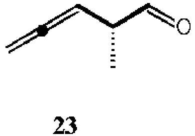 wobei
P₁, P₂ und P₃ jeweils einzeln eine Hydroxylschutzgruppe sind,
Q eine Alkinschutzgruppe oder Wasserstoff ist,
L ein Hydroxylaktivierungsgruppenderivat ist und
R eine Diolschutzgruppe ist.

2. Verfahren nach Anspruch 1, wobei ein oder mehrere der Schritte (a), (b), (e), (f), (g), (h), (k), (l), (n), (o), (q), (r) und/oder (s) in einem Reaktionsgefäß durch Verwendung von teleskopischen Reaktionen durchgeführt wird/werden.

3. Verfahren nach einem der Ansprüche 1 - 2, das ferner Folgendes umfasst:
(u) Umwandeln der Verbindung der Formel 21 zu einer Verbindung der Formel 29, um ein Ingenol-3-angelat zu bilden:

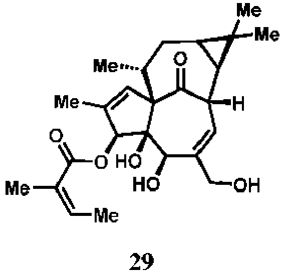

4. Verfahren zum Synthetisieren der Verbindung 34 aus der Verbindung 7, das Folgendes umfasst: Schützen der Hydroxylgruppen der Verbindung 7, um die Verbindung 33 zu erhalten 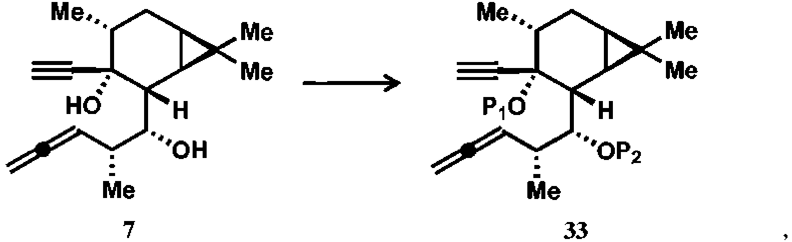 und Zyklisieren der Verbindung 33 durch Inkubieren der Verbindung 33 mit einem Chlordicarbonylrhodium(I)-Dimer, das aus der Gruppe ausgewählt ist, die aus Folgendem besteht: ([RhCl(CO)₂]₂), [RhCl(COD)]₂, [RhCl(CO)(dppp)]₂ und [Rh(dppp)₂]Cl, um die Verbindung 34 zu erhalten: 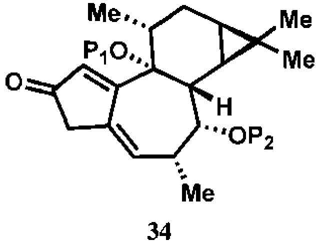 wobei P₁ und P₂ jeweils einzeln eine Hydroxylschutzgruppe ist.

5. Verfahren zum Synthetisieren der Verbindung 44 aus der Verbindung 34, das Folgendes umfasst: Inkubieren der Verbindung 34 mit einem Grignard-Reagenz XMgBr, um die Verbindung 44 zu erhalten 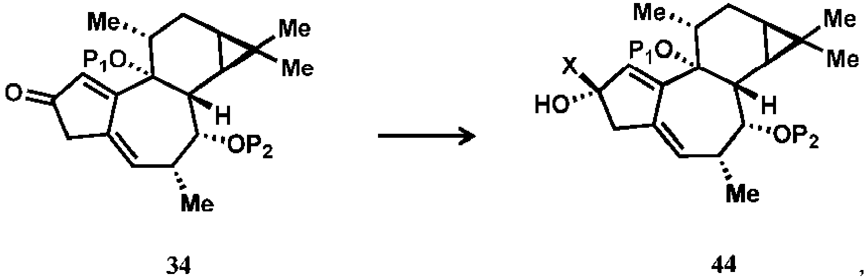 wobei P₁ und P₂ jeweils unabhängig voneinander eine Hydroxylschutzgruppe sind und X eine Alkylgroup ist.

6. Verfahren zum Synthetisieren der Verbindung 36 aus der Verbindung 35, das Folgendes umfasst: Inkubieren der Verbindung 35 mit katalytischen Mengen von OsO₄, um die Verbindung 36 herzustellen. 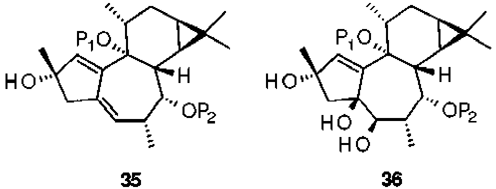 wobei P₁ und P₂ jeweils unabhängig voneinander eine Hydroxylschutzgruppe sind.

7. Verfahren nach Anspruch 6, das Folgendes umfasst: Inkubieren der Verbindung 35 mit katalytischen Mengen von OsO₄ in Gegenwart eines Oxidierungsmittels und in Gegenwart eines Puffers, um die Verbindung 36 herzustellen.

8. Verfahren nach einem der Ansprüche 6 - 7, wobei das Oxidationsmittel aus der Gruppe ausgewählt ist, die aus Trimethylamin-*N*-oxid, *N*-methylmorpholin-N-oxid und tert-Butylhydroperoxid besteht.

9. Verfahren nach einem der Ansprüche 7 - 8, wobei der pH-Wert des Puffers innerhalb von pH1 - pH6 liegt.

10. Verfahren nach einem der Ansprüche 7 - 8, wobei der Puffer eine Säure oder Salze einer Säure umfasst, die aus der Gruppe ausgewählt sind, die aus Zitronensäure, Phosphorsäure und Essigsäure oder Mischungen davon beseht.

11. Verfahren zum Synthetisieren der Verbindung 38 aus der Verbindung 37, das Folgendes umfasst:
Inkubieren der Verbindung 37 mit einer Lewissäure unter Reduktionsbedingungen, um die Verbindung 38 zu erhalten 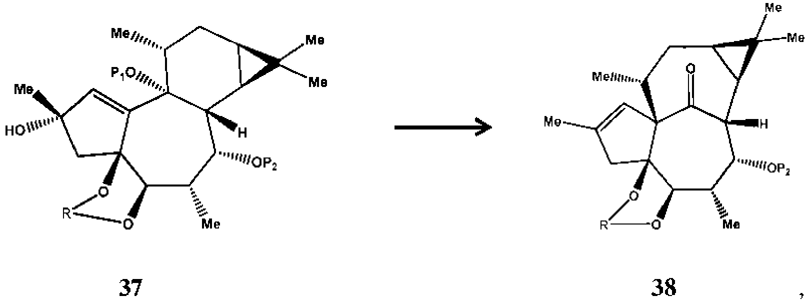 wobei P₁ und P₂ jeweils unabhängig voneinander eine Hydroxylschutzgruppe sind und R eine Diolschutzgruppe ist.

12. Verfahren nach Anspruch 11, wobei die Lewissäure BF₃•Et₂O ist.

13. Verbindung, ausgewählt aus der Gruppe bestehend aus:

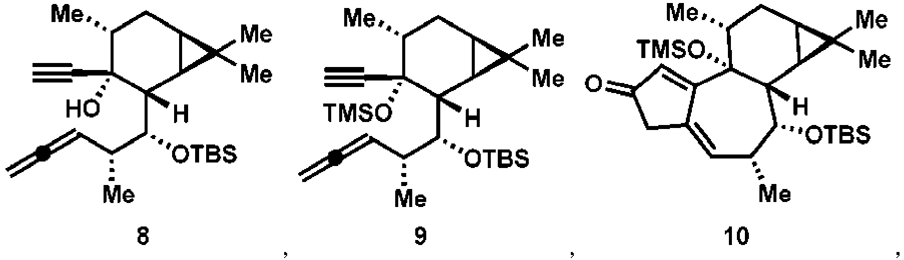

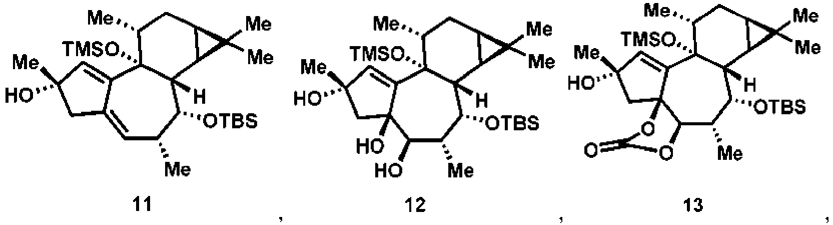

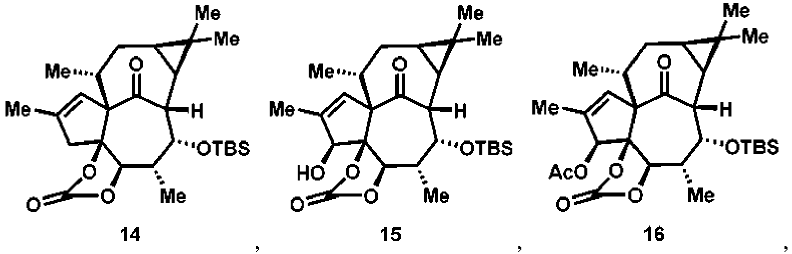

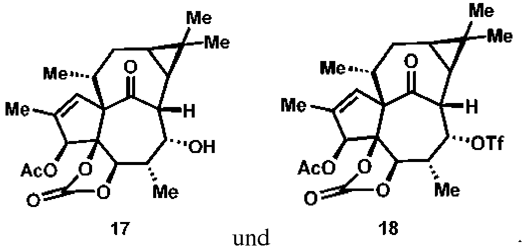

## Revendications

1. Procédé de synthèse d'ingénol (**21**), qui comprend :
(a) la chloration du composé de formule **1** pour former un composé de formule **2 :** 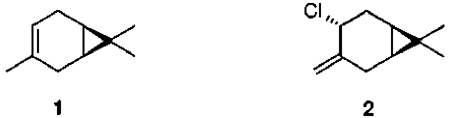
(b) l'ozonolyse du composé de formule **2** pour former un composé de formule **3 :** 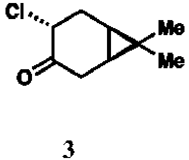
(c) l'alkylation réductrice de **3** pour former un composé de formule **4 :** 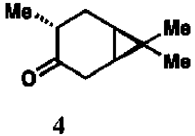
(d) la formation d'un alcool de formule **5** à partir du composé de formule **4 :** 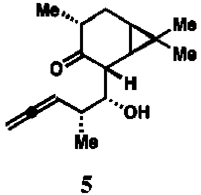
(e) la formation d'un composé de formule **31** par addition d'acétylure au composé de formule **5:** 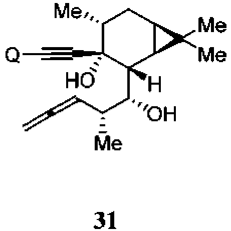
(f) la déprotection du composé de formule **31** lorsque Q ne représente pas un atome d'hydrogène, pour former un composé de formule **7 :** 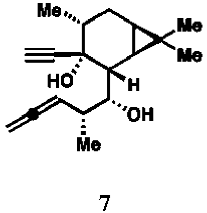
(g) la protection du composé de formule **7** pour former un composé de formule **32 :** 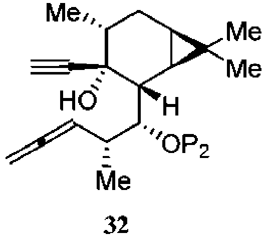
(h) la protection du composé de formule **32** pour former un composé de formule **33 :** 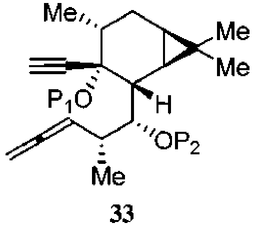
(i) la cyclisation du composé de formule **33** pour former un composé de formule **34 :** 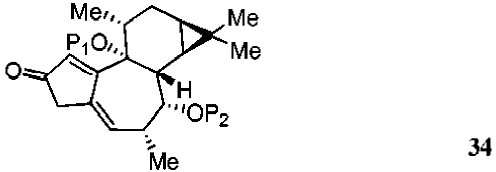
(j) la méthylation du composé de formule **34** pour former un composé de formule **35 :** 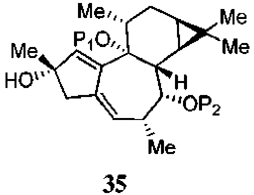
(k) la dihydroxylation du composé de formule **35** pour former un composé de formule **36 :** 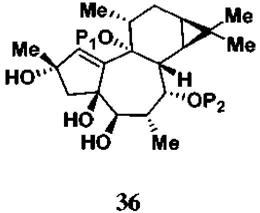
(l) la protection du composé de formule **36** pour former un composé de formule **37 :** 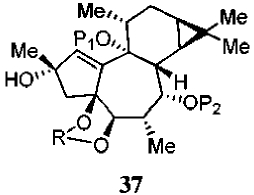
(m) l'exécution d'un réarrangement pinacolique du composé de formule **37** pour former un composé de formule **38 :** 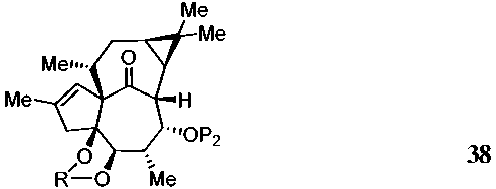
(n) l'oxydation du composé de formule **38** pour former un composé de formule **39 :** 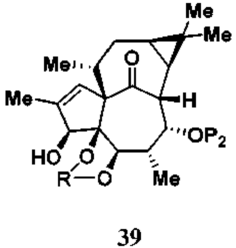
(o) la protection du composé de formule **39** pour former un composé de formule **40 :** 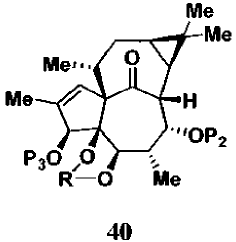
(p) la déprotection du composé de formule **40** pour former un composé de formule **41 :** 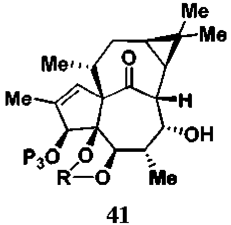
(q) l'activation du composé de formule **41** avec un groupe activateur de groupe hydroxy pour former un composé de formule **42 :** 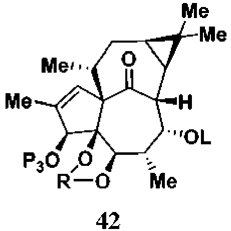
(r) l'élimination du groupe hydroxy activé du composé de formule **42** pour former un composé de formule **43 :** 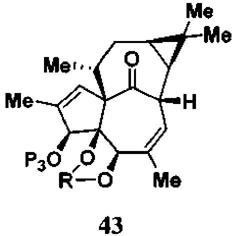
(s) la déprotection du composé de formule **43** pour former un composé de formule **20 :** 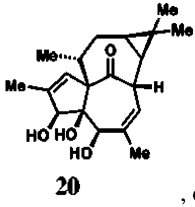 et
(t) l'oxydation du composé de formule **20** pour former le composé de formule **21 :** 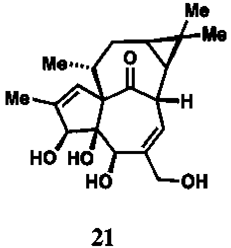 ladite étape (d) comprenant l'incubation d'un réactif de formule **23** avec le composé de formule **4 :** 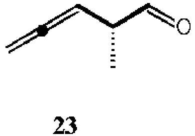 dans laquelle
P₁, P₂ et P₃ représentent chacun individuellement un groupe protecteur de groupe hydroxy,
Q représente un groupe protecteur de groupe alcyne ou un atome d'hydrogène,
L représente un dérivé de groupe activateur de groupe hydroxy, et
R représente un groupe protecteur de diol.

2. Procédé selon la revendication 1, une ou plusieurs des étapes (a), (b), (e), (f), (g), (h), (k), (1), (n), (o), (q), (r) et/ou (s) étant effectuées dans une seule cuve de réaction à l'aide de réactions se téléscopant.

3. Procédé selon l'une quelconque des revendications 1 à 2, qui comprend en outre :
(u) la conversion du composé de formule **21** en composé de formule **29** pour former l'ingénol-3- angélate :

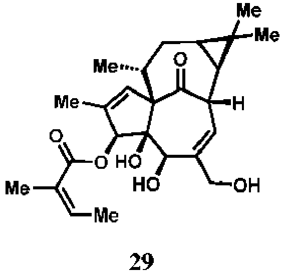

4. Procédé de synthèse d'un composé **34** à partir du composé **7,** qui comprend : la protection des groupements hydroxy du composé **7** pour produire le composé **33** 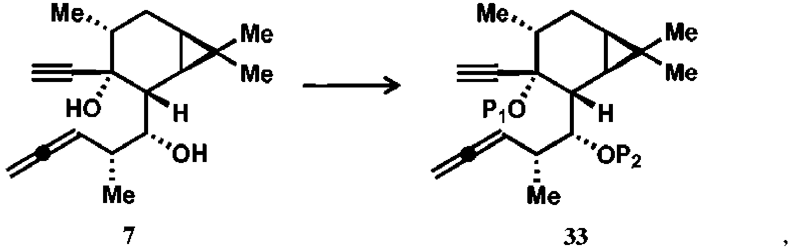 et la cyclisation du composé **33** par incubation du composé **33** avec un dimère de chlorodicarbonylrhodium(I) choisi dans le groupe constitué par : ([RhCl(CO)₂]₂), [RhCl(COD)]₂, [RhCl(CO)(dppp)]₂ et [Rh(dppp)₂]Cl, pour produire le composé 34 : 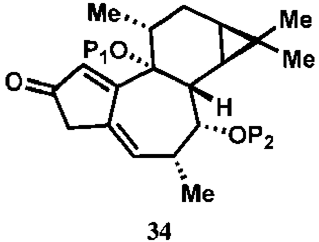 dans lequel P₁ et P₂ représentent chacun individuellement un groupe protecteur de groupe hydroxy.

5. Procédé de synthèse du composé **44** à partir du composé **34,** qui comprend : l'incubation du composé **34** avec un réactif de Grignard XMgBr pour produire un composé **44** 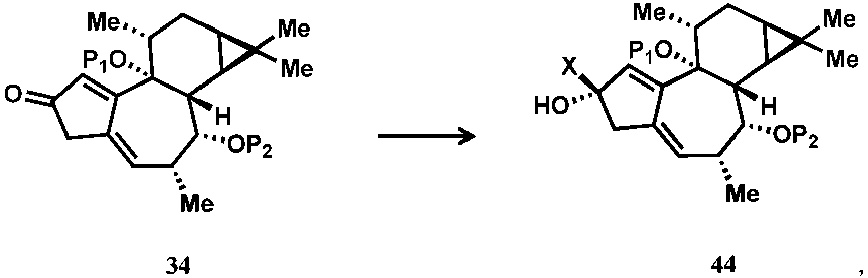 dans lequel P₁ et P₂ représentent chacun individuellement un groupe protecteur de groupe hydroxy et X représente un groupe alkyle.

6. Procédé de synthèse du composé **36** à partir du composé **35,** qui comprend : l'incubation du composé **35** avec des quantités catalytiques d'OsO₄ pour produire le composé **36** 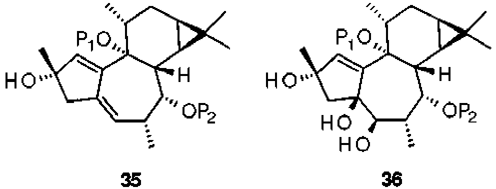 dans lequel P₁ et P₂ représentent chacun individuellement un groupe protecteur de groupe hydroxy.

7. Procédé selon la revendication 6 comprenant : l'incubation du composé **35** avec des quantités catalytiques d'OsO₄ en présence d'un oxydant et en présence d'un tampon pour produire le composé **36.**

8. Procédé selon l'une quelconque des revendications 6 à 7, ledit oxydant étant choisi dans le groupe constitué par le triméthylamine-*N*-oxyde, le *N*-méthylmorpholine-N-oxyde et l'hydroperoxyde de tert-butyle.

9. Procédé selon l'une quelconque des revendications 7 à 8, le pH du tampon se trouvant entre pH 1 à pH 6.

10. Procédé selon l'une quelconque des revendications 7 à 8, ledit tampon comprenant un acide, ou des sels d'acide, choisi dans le groupe constitué par l'acide citrique, l'acide phosphorique et l'acide acétique, ou des mélanges de ceux-ci.

11. Procédé de synthèse du composé **38** à partir du composé **37,** qui comprend :
l'incubation du composé **37** avec un acide de Lewis dans des conditions réductrices, pour produire le composé **38** 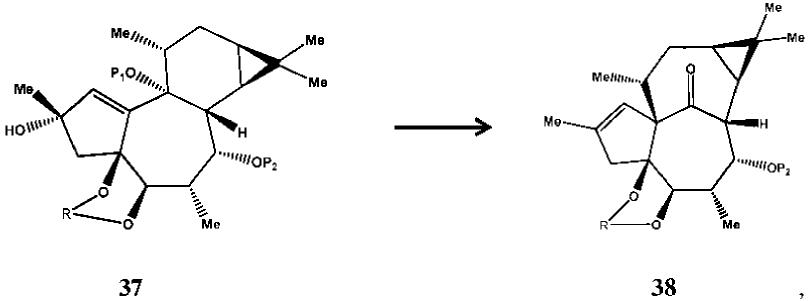 dans lequel P₁ et P₂ représentent chacun individuellement un groupe protecteur de groupe hydroxy et R représente un groupe protecteur de diol.

12. Procédé selon la revendication 11, ledit acide de Lewis étant BF₃•Et₂O.

13. Composé choisi dans le groupe constitué par :
